# EUROPEAN PATENT SPECIFICATION

(11) **EP 1 960 371 B1**
(45) Date of publication and mention of the grant of the patent: **16.09.2009**
(21) Application number: 06820361.1
(22) Date of filing: 29.11.2006
(51) Int. Cl.: C07D 239/94, A61K 31/505, A61P 35/00

(54) **QUINAZOLEINE DERIVATIVES USED AS INHIBITORS OF ERBB TYROSINE KINASE**
ALS INHIBITOREN VON ERBB-TYROSINKINASE VERWENDETE CHINAZOLEINDERIVATE
DERIVES DE QUINAZOLINE UTILISES EN TANT QU INHIBITEURS DES TYROSINE KINASES ERBB ASSOCIEES AU RECEPTEUR

(30) Priority: 02.12.2005 EP 05292551
(43) Date of publication of application: 27.08.2008
(73) Proprietor: AstraZeneca AB, 151 85 Södertälje (SE)
(72) Inventor: BRADBURY, Robert Hugh, Cheshire SK10 4TG (GB); BARLAAM, Bernard Christophe, F-51689 Reims (FR); DUCRAY, Richard, F-51689 Reims (FR)
(86) International application number: PCT/GB2006/004451
(87) International publication number: WO 2007/063293

(56) References cited:
- WO-A-03/040108
- WO-A-20/04093880
- WO-A-20/05051923
- WO-A-20/05118572
- WO-A1-01/94341
- WO-A2-03/040109

## Description

The invention concerns certain novel quinazoline derivatives, or pharmaceutically acceptable salts thereof, which possess anti-tumour activity and are accordingly useful in methods of treatment of the human or animal body. The invention also concerns processes for the manufacture of said quinazoline derivatives, to pharmaceutical compositions containing them and to their use in therapeutic methods, for example in the manufacture of medicaments for use in the prevention or treatment of solid tumour disease in a warm-blooded animal such as man.

Many of the current treatment regimes for diseases resulting from the abnormal regulation of cellular proliferation such as psoriasis and cancer, utilise compounds that inhibit DNA synthesis and cellular proliferation. To date, compounds used in such treatments are generally toxic to cells however their enhanced effects on rapidly dividing cells such as tumour cells can be beneficial. Alternative approaches to these cytotoxic anti-tumour agents are currently being developed, for example as selective inhibitors of cell signalling pathways. These types of inhibitors are likely to have the potential to display an enhanced selectivity of action against tumour cells and so are likely to reduce the probability of the therapy possessing unwanted side effects.

Eukaryotic cells are continually responding to many diverse extracellular signals that enable communication between cells within an organism. These signals regulate a wide variety of physical responses in the cell including proliferation, differentiation, apoptosis and motility. The extracellular signals take the form of a diverse variety of soluble factors including growth factors and other autocrine, paracrine and endocrine factors. By binding to specific transmembrane receptors, these ligands integrate the extracellular signal to the intracellular signalling pathways, therefore transducing the signal across the plasma membrane and allowing the individual cell to respond to its extracellular signals. Many of these signal transduction processes utilise the reversible process of the phosphorylation of proteins that are involved in the promotion of these diverse cellular responses. The phosphorylation status of target proteins is regulated by specific kinases and phosphatases that are responsible for the regulation of about one third of all proteins encoded by the mammalian genome. As phosphorylation is such an important regulatory mechanism in the signal transduction process, it is therefore not surprising that aberrations in these intracellular pathways result in abnormal cell growth and differentiation and so promote cellular transformation (reviewed in Cohen et al, Curr Opin Chem Biol, 1999, 3, 459-465).

It has been widely shown that a number of these tyrosine kinases are mutated to constitutively active forms and/or when over-expressed result in the transformation of a variety of human cells. These mutated and over-expressed forms of the kinase are present in a large proportion of human tumours (reviewed in Kolibaba et al, Biochimica et Biophysica Acta, 1997, 133, F217-F248). As tyrosine kinases play fundamental roles in the proliferation and differentiation of a variety of tissues, much focus has centred on these enzymes in the development of novel anti-cancer therapies. This family of enzymes is divided into two groups - receptor and non-receptor tyrosine kinases, for example EGF Receptors and the SRC family respectively. From the results of a large number of studies including the Human Genome Project, about 90 tyrosine kinase have been identified in the human genome, of this 58 are of the receptor type and 32 are of the non-receptor type. These can be compartmentalised into 20 receptor tyrosine kinase and 10 non-receptor tyrosine kinase sub-families (Robinson et al, Oncogene, 2000, 19, 5548-5557).

The receptor tyrosine kinases are of particular importance in the transmission of mitogenic signals that initiate cellular replication. These large glycoproteins, which span the plasma membrane of the cell possess an extracellular binding domain for their specific ligands (such as Epidermal Growth Factor (EGF) for the EGF Receptor). Binding of ligand results in the activation of the receptor's kinase enzymatic activity that resides in the intracellular portion of the receptor. This activity phosphorylates key tyrosine amino acids in target proteins, resulting in the transduction of proliferative signals across the plasma membrane of the cell.

It is known that the erbB family of receptor tyrosine kinases, which include EGFR, erbB2, erbB3 and erbB4, are frequently involved in driving the proliferation and survival of tumour cells (reviewed in Olayioye et al., EMBO J., 2000, 19, 3159). One mechanism in which this can be accomplished is by overexpression of the receptor at the protein level, generally as a result of gene amplification. This has been observed in many common human cancers (reviewed in Klapper et al., Adv. Cancer Res., 2000, 77, 25) such as breast cancer (Sainsbury et al., Brit. J. Cancer, 1988, 58, 458; Guerin et al., Oncogene Res., 1988, 3, 21; Slamon et al., Science, 1989, 244, 707; Klijn et al., Breast Cancer Res. Treat., 1994, 29, 73 and reviewed in Salomon et al., Crit. Rev. Oncol. Hematol., 1995, 19, 183), non-small cell lung cancers (NSCLCs) including adenocarcinomas (Cemy et al., Brit. J. Cancer, 1986, 54, 265; Reubi et al., Int. J. Cancer, 1990, 45, 269; Rusch et al., Cancer Research, 1993, 53, 2379; Brabender et al, Clin. Cancer Res., 2001, 7, 1850) as well as other cancers of the lung (Hendler et al., Cancer Cells, 1989, 7, 347; Ohsaki et al., Oncol. Rep., 2000, 7, 603), bladder cancer (Neal et al., Lancet, 1985, 366; Chow et al., Clin. Cancer Res., 2001, 7, 1957, Zhau et al., Mol Carcinog., 3, 254), oesophageal cancer (Mukaida et al., Cancer, 1991, 68, 142), gastrointestinal cancer such as colon, rectal or stomach cancer (Bolen et al., Oncogene Res., 1987, 1, 149; Kapitanovic et al., Gastroenterology, 2000, 112, 1103; Ross et al., Cancer Invest., 2001, 19, 554), cancer of the prostate (Visakorpi et al., Histochem. J., 1992, 24, 481; Kumar et al., 2000, 32, 73; Scher et al., J. Natl. Cancer Inst., 2000, 92, 1866), leukaemia (Konaka et al., Cell, 1984, 37, 1035, Martin-Subero et al., Cancer Genet Cytogenet., 2001, 127, 174), ovarian (Hellstrom et al., Cancer Res., 2001, 61, 2420), head and neck (Shiga et al., Head Neck, 2000, 22, 599) or pancreatic cancer (Ovotny et al., Neoplasma 2001, 48, 188). As more human tumour tissues are tested for expression of the erbB family of receptor tyrosine kinases it is expected that their widespread prevalence and importance will be further enhanced in the future.

As a consequence of the mis-regulation of one or more of these receptors (in particular erbB2), it is widely believed that many tumours become clinically more aggressive and so correlate with a poorer prognosis for the patient (Brabender et al, Clin. Cancer Res., 2001, 7, 1850; Ross et al, Cancer Investigation, 2001,19, 554, Yu et al., Bioessays, 2000, 22.7, 673).

In addition to these clinical findings, a wealth of pre-clinical information suggests that the erbB family of receptor tyrosine kinases are involved in cellular transformation. This includes the observations that many tumour cell lines overexpress one or more of the erbB receptors and that EGFR or erbB2 when transfected into non-tumour cells have the ability to transform these cells. This tumourigenic potential has been further verified as transgenic mice that overexpress erbB2 spontaneously develop tumours in the mammary gland. In addition to this, a number of pre-clinical studies have demonstrated that anti-proliferative effects can be induced by knocking out one or more erbB activities by small molecule inhibitors, dominant negatives or inhibitory antibodies (reviewed in Mendelsohn et al., Oncogene, 2000, 19, 6550). Thus it has been recognised that inhibitors of these receptor tyrosine kinases should be of value as a selective inhibitor of the proliferation of mammalian cancer cells (Yaish et al. Science, 1988, 242, 933, Kolibaba et al, Biochimica et Biophysica Acta, 1997, 133, F217-F248; Al-Obeidi et al, 2000, Oncogene, 19, 5690-5701; Mendelsohn et al, 2000, Oncogene, 19, 6550-6565).

In addition to this pre-clinical data, the small molecule EGFR tyrosine kinase inhibitors Iressa (also known as gefitinib and ZD1839) and Tarceva (also known as erlotinib and CP-358,774) have been approved for use in the treatment of advanced non-small cell lung cancer. Furthermore, inhibitory antibodies against EGFR and erbB2 (erbitux (c-225 / cetuximab) and herceptin (trastuzumab) respectively) have proven to be beneficial in the clinic for the treatment of selected solid tumours (reviewed in Mendelsohn et al, 2000, Oncogene, 19, 6550-6565).

Recently mutations in the ATP binding pocket of the intracellular catalytic domain of the EGF receptor have been discovered in certain sub-sets of non-small cell lung cancers (NSCLCs). The presence of mutations in the receptor appear to correlate with response to EGFR tyrosine kinase inhibitors such as gefitinib (Lynch et al, N Engl J Med 2004; 350: 2129-2139; Paez et al, Science 2004; 304: 1497-1500), although it is becoming evident that the clinical benefits of compounds such as gefitinib and erlotinib are not likely to be mediated by EGFR mutations alone. It has been demonstrated that ligand stimulation results in a different phosphorylation pattern in mutated receptors compared with that seen in wild-type receptors and it is thought that mutant EGF receptors selectively transduce survival signals on which NSCLCs become dependent. Inhibition of those signals by compounds such as gefitinib may contribute to the efficacy of such drugs (Sordella et al. Science 2004; 305: 1163-1167). Similarly, mutations within the erbB2 kinase domain have recently been discovered in certain primary tumours, such as NSCLC, glioblastoma and gastric and ovarian tumours (Stephens et al., Nature 2004; 431; 525-526). Accordingly the inhibition of the EGF and/or erbB2 receptor tyrosine kinase in both wild-type and mutated receptors is an important target that would be expected to provide an anti-cancer effect.

Amplification and/or activity of members of the erbB type receptor tyrosine kinases have been detected and so have been implicated to play a role in a number of non-malignant proliferative disorders such as psoriasis (Ben-Bassat, Curr. Pharm. Des., 2000, 6, 933; Elder et al., Science, 1989, 243, 811), benign prostatic hyperplasia (BPH) (Kumar et al., Int. Urol. Nephrol., 2000, 32, 73), atherosclerosis and restenosis (Bokemeyer et al., Kidney Int., 2000, 58, 549). It is therefore expected that inhibitors of erbB type receptor tyrosine kinases will be useful in the treatment of these and other non-malignant disorders of excessive cellular proliferation.

International Patent Applications WO 96/09294, WO 96/15118, WO 96/16960, WO 96/30347, WO 96/33977, WO 96/33978, WO 96/33979, WO 96/33980, WO 96/33981, WO 97/03069, WO 97/13771, WO 97/30034, WO 97/30035, WO 97/38983, WO 98/02437, WO 98/02434, WO 98/02438, WO 98/13354, WO 99/35132, WO 99/35146, WO 01/21596, WO 01/55141 and WO 02/18372 disclose that certain quinazoline derivatives which bear an anilino substituent at the 4-position possess receptor tyrosine kinase inhibitory activity.

International Patent Application WO 01/94341 discloses that certain quinazoline derivatives which carry a 5-position substituent are inhibitors of the Src family of non-receptor tyrosine kinases, such as c-Src, c-Yes and c-Fyn.

WO 03/040108 and WO 03/040109 disclose that certain quinazoline derivatives which carry a 5-position substituent are inhibitors of the erbB family of tyrosine kinase inhibitors, particularly EGF and erbB2 receptor tyrosine kinases. WO 03/040108 and WO 03/040109 each disclose certain 4-anilinoquinazoline derivatives. None of the quinazoline derivatives disclosed contain a methoxy linked amide group at the 5-position on the quinazoline ring.

WO 2004/093880 discloses that certain 4-anilinoquinazoline derivatives which carry a 5-position substituent are inhibitors of the erbB family of tyrosine kinase inhibitors, particularly EGF and erbB2 receptor tyrosine kinases. The compounds disclosed do not carry a cyclic amide substituent on the phenyl ring of the anilino group nor are they substituted at the 5-position on the quinazoline ring by a methoxy linked amide group.

WO 2005/051923 also discloses that certain quinazoline derivatives which carry a 5-position substituent are inhibitors of the erbB family of tyrosine kinase inhibitors, particularly EGF and erbB2 receptor tyrosine kinases. This PCT patent application discloses certain 4-anilinoquinazoline derivatives which carry an acylaminoethoxy substituent at the 5-position on the quinazoline ring. The compounds disclosed do not carry a cyclic amide substituent on the phenyl ring of the anilino group nor are they substituted at the 5-position on the quinazoline ring by a methoxy linked amide group.

WO 2005/118572 (i.e. co-pending PCT patent application number PCT/GB2005/002215) also discloses that certain quinazoline derivatives which carry a 5-position substituent are inhibitors of the erbB family of tyrosine kinase inhibitors, particularly EGF and erbB2 receptor tyrosine kinases. This PCT patent application discloses certain 4-anilinoquinazoline derivatives which carry a methoxy linked amide substituent at the 5-position on the quinazoline ring. There is no disclosure in this application of a 4-anilinoquinazoline derivative that is substituted on the phenyl ring of the anilino group by a cyclic amide.

None of the prior art discloses quinazoline derivatives that are substituted at the 4-position on the quinazoline ring by an anilino group that carries a cyclic amide substituent at the 4-position on the phenyl ring and that are substituted at the 5-position on the quinazoline ring by a methoxy linked amide group.

There remains a need to find further compounds with good *in vivo* activity together with improved pharmacological characteristics compared with known erbB tyrosine kinase inhibitors, particularly compounds that are selective erbB2 tyrosine kinase inhibitors. For example, there is a need for novel compounds with advantageous and/or improved characteristics in, but not limited to, for example, (i) physical properties; (ii) favourable DMPK properties, such as high bioavailability and/or advantageous half life and/or advantageous volume of distribution and/or high absorption; (iii) factors that decrease the liability for clinical drug-drug interactions (for example cytochrome P450 enzyme inhibition or induction); and (iv) compounds with a reduced liability for QT interval prolongation in patients, for example compounds which are inactive or weakly active in a hERG assay.

Surprisingly, we have now found that a select group of 4-anilinoquinazoline derivatives that carry a cyclic amide substituent at the 4-position on the phenyl ring of the anilino group and that are substituted at the 5-position on the quinazoline ring with a substituent containing certain methoxy-linked amide groups possess potent anti-tumour activity. Without wishing to imply that the quinazoline derivatives disclosed in the present invention possess pharmacological activity only by virtue of an effect on a single biological process, it is believed that the quinazoline derivatives provide an anti-tumour effect by way of inhibition of one or more of the erbB family of receptor tyrosine kinases that are involved in the signal transduction steps which lead to the proliferation of tumour cells. In particular, it is believed that the quinazoline derivatives of the present invention provide an anti-tumour effect by way of inhibition of EGF and/or erbB2 receptor tyrosine kinases. More particularly, it is believed that the quinazoline derivatives of the present invention provide an anti-tumour effect by way of the selective inhibition of erbB2 receptor tyrosine kinase, compared to EGF receptor tyrosine kinase. It is also believed that the quinazoline derivatives of the present invention exhibit a combination of favourable properties, such as those described hereinbefore.

References to erbB receptors, particularly erbB2, used herein are intended to include both wild-type and mutated receptors unless specifically stated otherwise. The term "mutation" includes, but is not limited to, gene amplification, nucleotide in-frame deletions or substitutions in one or more of the exons that encode receptors such as erbB2.

Generally the quinazoline derivatives of the present invention possess potent inhibitory activity against the erbB receptor tyrosine kinase family, for example by inhibition of EGF and/or erbB2 and/or erbB4 receptor tyrosine kinases, whilst possessing less potent inhibitory activity against other kinases. Furthermore, generally the quinazoline derivatives of the present invention possess substantially better potency against the erbB2 receptor tyrosine kinase over that of the EGFR tyrosine kinase, thus potentially providing effective treatment for erbB2 driven tumours. Accordingly, it may be possible to administer a quinazoline derivative according to the present invention at a dose that is sufficient to inhibit erbB2 tyrosine kinase whilst having no significant effect upon EGFR or other tyrosine kinases. The selective inhibition provided by the quinazoline derivatives according to the present invention may provide treatments for conditions mediated by erbB2 tyrosine kinase, whilst reducing undesirable side effects that may be associated with the inhibition of other tyrosine kinases.

According to a first aspect of the invention there is provided a quinazoline derivative of the Formula **I**: 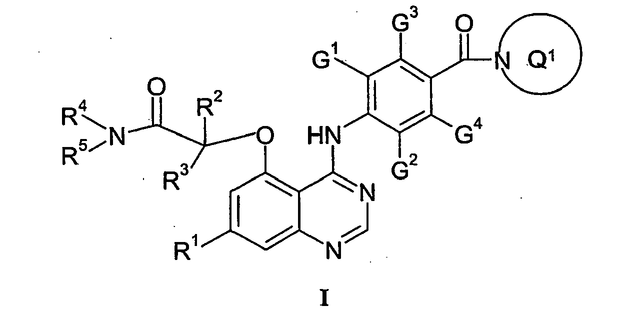 wherein:
**R¹** is selected from hydrogen, hydroxy, (1-4C)alkoxy and (1-4C)alkoxy(1-4C)alkoxy;
**R² and R³,** which may be the same or different, are selected from hydrogen, (1-4C)alkyl, (2-4C)alkenyl and (2-4C)alkynyl, which (1-4C)alkyl optionally bears one or more hydroxy substituents;
**R⁴ and R⁵,** which may be the same or different, are selected from hydrogen, (1-4C)alkyl, (3-4C)alkenyl and (3-4C)alkynyl, which (1-4C)alkyl optionally bears one or more substituents independently selected from halogeno, cyano, hydroxy, amino, (1-4C)alkylamino, di-[(1-4C)alkyl]amino and (1-4C)alkoxy, or
R⁴ and R⁵ together with the nitrogen atom to which they are attached form a saturated 4, 5, 6 or 7 membered heterocyclic ring containing one nitrogen heteroatom and optionally containing one or more additional heteroatoms independently selected from oxygen, nitrogen and sulfur,
and wherein any heterocyclic ring formed by R⁴, R⁵ and the nitrogen atom to which they are attached optionally bears one or more substituents independently selected from halogeno, cyano, hydroxy, (1-4C)alkyl and (1-4C)alkoxy;
**G¹ and G²,** which may be the same or different, are selected from hydrogen and halogeno;
**G³ and G⁴,** which may be the same or different, are selected from hydrogen, halogeno, cyano, (1-4C)akyl, (1-4C)alkoxy, (2-4C)alkenyl and (2-4C)alkynyl;
**the ring -NQ¹** is a nitrogen-linked, saturated or partially unsaturated, 4, 5, 6, 7 or 8 membered heterocyclic ring containing one nitrogen heteroatom and optionally containing one or more additional heteroatoms independently selected from oxygen, nitrogen and sulfur, and which heterocyclic ring -NQ¹ optionally bears one or more substituents independently selected from halogeno, cyano, hydroxy, (1-4C)alkyl, (1-4C)alkoxy and hydroxy-(1-4C)alkyl;
and wherein any heterocyclic ring formed by R⁴, R⁵ and the nitrogen atom to which they are attached and/or any heterocyclic ring -NQ¹ optionally bears 1 or 2 oxo or thioxo substituents;
or a pharmaceutically acceptable salt thereof.

According to a second aspect of the invention there is provided a quinazoline derivative of the Formula **I,** wherein:
R¹ is selected from hydrogen, hydroxy, (1-4C)alkoxy and (1-4C)alkoxy(1-4C)alkoxy;
R² and R³, which may be the same or different, are selected from hydrogen, (1-4C)alkyl, (2-4C)alkenyl and (2-4C)alkynyl, which (1-4C)alkyl optionally bears one or more hydroxy substituents;
R⁴ and R⁵, which may be the same or different, are selected from hydrogen, (1-4C)alkyl, (3-4C)alkenyl and (3-4C)alkynyl, which (1-4C)alkyl optionally bears one or more substituents independently selected from halogeno, cyano, hydroxy, amino, (1-4C)alkylamino, di-[(1-4C)alkyl]amino and (1-4C)alkoxy;
G¹ and G², which may be the same or different, are selected from hydrogen and halogeno;
G³ and G⁴, which may be the same or different, are selected from hydrogen, halogeno, cyano, (1-4C)alkyl, (1-4C)alkoxy, (2-4C)alkenyl and (2-4C)alkynyl;
the ring -NQ¹ is a nitrogen-linked, saturated or partially unsaturated, 4, 5, 6, 7 or 8 membered heterocyclic ring containing one nitrogen heteroatom and optionally containing one or more additional heteroatoms independently selected from oxygen, nitrogen and sulfur, and which heterocyclic ring -NQ¹ optionally bears one or more substituents independently selected from halogeno, cyano, hydroxy, (1-4C)alkyl, (1-4C)alkoxy and hydroxy-(1-4C)alkyl;
and wherein any heterocyclic ring -NQ¹ optionally bears 1 or 2 oxo or thioxo substituents;
or a pharmaceutically acceptable salt thereof.

It is to be understood that, insofar as certain of the quinazoline derivatives of the Formula **I** defined above may exist in optically active or racemic forms by virtue of one or more asymmetric carbon atoms, the invention includes in its definition any such optically active or racemic form which possesses the above-mentioned activity. In particular, the quinazoline derivatives of the Formula **I** may have a chiral centre on the carbon atom attached to the groups R² and R³, if the groups R² and R³ are not identical. The present invention encompasses all such stereoisomers having activity as herein defined, for example the (2R) and (2S) isomers (particularly the (2R) isomers). It is further to be understood that in the names of chiral compounds (*R,S*) denotes any scalemic or racemic mixture while (*R*) and (*S*) denote the enantiomers. In the absence of (*R,S*), (*R*) or (*S*) in the name it is to be understood that the name refers to any scalemic or racemic mixture, wherein a scalemic mixture contains R and S enantiomers in any relative proportions and a racemic mixture contains *R* and *S* enantiomers in the ratio 50:50. The synthesis of optically active forms may be carried out by standard techniques of organic chemistry well known in the art, for example by synthesis from optically active starting materials or by resolution of a racemic form. Similarly, the above-mentioned activity may be evaluated using the standard laboratory techniques referred to hereinafter.

In this specification the generic term "alkyl" includes both straight-chain and branched-chain alkyl groups such as propyl, isopropyl and tert-butyl. However references to individual alkyl groups such as "propyl" are specific for the straight-chain version only, references to individual branched-chain alkyl groups such as "isopropyl" are specific for the branched-chain version only. An analogous convention applies to other generic terms, for example (1-4C)alkoxy includes methoxy and ethoxy, (1-4C)alkylamino includes methylamino, ethylamino and isopropylamino and di-[(1-4C)alkyl]amino includes dimethylamino, diethylamino and N-isopropyl-N-methylamino.

Suitable values for the generic radicals referred to above include those set out below.

Where reference is made herein to R⁴ and R⁵ together with the nitrogen atom to which they are attached forming a saturated (i.e. ring systems with the maximum degree of saturation) 4, 5, 6 or 7 membered heterocyclic ring containing one nitrogen heteroatom and optionally containing one or more additional heteroatoms independently selected from oxygen, nitrogen and sulfur, the ring so formed suitably contains one or two additional heteroatoms and, more suitably contains one additional heteroatom. For example, the ring so formed may be selected from azetidin-1-yl, pyrrolidin-1-yl, pyrazolidin-1-yl, piperidin-1-yl, morpholin-4-yl, piperazin-1-yl, thiomorpholin-4-yl, 1,3-oxazolidin-1-yl, azepan-1-yl, diazepan-1-yl and 1,4-oxazepan-1-yl, particularly azetidin-1-yl, pyrrolidin-1-yl, piperidin-1-yl, morpholin-4-yl, piperazin-1-yl, thiomorpholin-4-yl and azepan-1-yl.

A suitable value for the ring -NQ¹ which is a nitrogen-linked, saturated (i.e. ring systems with the maximum degree of saturation) or partially unsaturated (i.e. ring systems retaining some, but not the full, degree of unsaturation) 4, 5, 6, 7 or 8 membered heterocyclic ring containing one nitrogen heteroatom (i.e. the nitrogen heteroatom that links the ring -NQ¹ to the carbonyl group in the Formula **I**) and optionally containing one or more additional heteroatoms independently selected from oxygen, nitrogen and sulfur is a monocyclic ring with up to a total of three heteroatoms. More suitable heterocyclic rings -NQ¹ may include, for example, saturated 5, 6 or 7 membered monocyclic heterocyclic rings containing one nitrogen heteroatom and optionally containing one or two additional heteroatoms independently selected from nitrogen, oxygen and sulfur (particularly selected from nitrogen and oxygen). Examples of such rings include azetidinyl, pyrrolidinyl, morpholinyl, pyrazolidinyl, thiomorpholinyl, piperidinyl, piperazinyl, oxazolidinyl, azepanyl, diazepanyl and oxazepanyl, particularly pyrrolidinyl, piperidinyl and azepanyl.

Yet more suitable heterocyclic rings -NQ¹ may include, for example, saturated 5, 6 or 7 membered monocyclic heterocyclic rings containing only one nitrogen heteroatom (i.e. the nitrogen heteroatom that links the ring -NQ¹ to the carbonyl group in the Formula **I**). Examples of such rings include pyrrolidinyl, piperidinyl and azepanyl.

Any of the heterocyclic rings formed by R⁴ and R⁵ together with the nitrogen atom to which they are attached and/or any of the heterocyclic rings -NQ¹ optionally bear one or more substituents, which may be the same or different, as defined herein.

Additionally, any of the heterocyclic rings formed by R⁴ and R⁵ together with the nitrogen atom to which they are attached and/or any of the heterocyclic rings -NQ¹ optionally bear 1 or 2 oxo or thioxo substituents. Examples of heterocyclic rings which bear 1 or 2 oxo or thioxo substituents include, for example, 3-oxomorpholinyl, 2-oxo-oxazolidinyl, 2-oxopyrrolidinyl, 2-thioxopyrrolidinyl, 2-oxoimidazolidinyl, 2-thioxoimidazolidinyl, 2-oxopiperidinyl, 2,5-dioxopyrrolidinyl, 2,5-dioxoimidazolidinyl, 2,6-dioxopiperidinyl, 2,4-dioxoimidazolidinyl and 2-oxopiperazinyl.

Additionally, any nitrogen or sulfur atom within a heterocyclic ring may be oxidized to give the corresponding N or S oxide, for example 1,1-dioxo-thiomorpholinyl.

It is to be understood that the quinazoline group in the Formula **I** is unsubstituted at each of the 2-, 6- and 8-positions on the quinazoline ring.

Suitable values for any of the 'R' groups (R¹ to R⁵), for any of the 'G' groups (G¹ to G⁴) or for various groups within a ring -NQ¹ include:-

| | |
|---|---|
| for halogeno | fluoro, chloro, bromo and iodo; |
| for (1-4C)alkyl: | methyl, ethyl, propyl, isopropyl and tert-butyl; |
| for (1-4C)alkoxy: | methoxy, ethoxy, propoxy, isopropoxy and butoxy; |
| for (1-4C)akoxy(1-4C)alkoxy | ethoxymethoxy, propoxymethoxy, methoxyethoxy, ethoxyethoxy, methoxypropoxy, ethoxypropoxy, methoxyisopropoxy and methoxybutoxy; |
| for (1-4C)alkylamino: | methylamino, ethylamino, propylamino, isopropylamino and butylamino; |
| for di-[(1-4C)alkyl]amino: | dimethylamino, diethylamino, N-ethyl-N-methylamino and diisopropylamino; |
| for (2-4C)alkenyl: | vinyl, isopropenyl, allyl and but-2-enyl; |
| for (2-4C)alkynyl: | ethynyl, 2-propynyl and but-2-ynyl; and |
| for hydroxy-(1-4C)alkyl: | hydroxymethyl, 2-hydroxyethyl, 1-hydroxyethyl and 3-hydroxypropyl. |

When in this specification reference is made to a (1-4C)alkyl group it is to be understood that such groups refer to alkyl groups containing up to 4 carbon atoms. Similarly, reference to a (1-2C)alkyl group refers to alkyl groups containing up to 2 carbon atoms such as methyl and ethyl. A similar convention is adopted for the other groups listed above.

It is to be understood that certain quinazoline derivatives of the Formula **I** may exist in solvated as well as unsolvated forms such as, for example, hydrated forms. It is to be understood that the invention encompasses all such solvated forms which exhibit an inhibitory effect on an erbB receptor tyrosine kinase, such as anti-proliferative activity.

It is also to be understood that certain quinazoline derivatives of the Formula **I** may exhibit polymorphism, and that the invention encompasses all such forms which exhibit an inhibitory effect on an erbB receptor tyrosine kinase, such as anti-proliferative activity.

It is also to be understood that the invention relates to all tautomeric forms of the quinazoline derivatives of the Formula **I** which exhibit an inhibitory effect on an erbB receptor tyrosine kinase, such as anti-proliferative activity.

A suitable pharmaceutically acceptable salt of a quinazoline derivative of the Formula **I** is, for example, an acid-addition salt of a quinazoline derivative of the Formula **I**, for example an acid-addition salt with an inorganic or organic acid. Suitable inorganic acids include, for example, hydrochloric, hydrobromic or sulfuric acid. Suitable organic acids include, for example, trifluoroacetic, citric or maleic acid. Another suitable pharmaceutically acceptable salt of a quinazoline derivative of the Formula **I** is for example, a salt of a quinazoline derivative of the Formula **I** which is sufficiently acidic, for example an alkali or alkaline earth metal salt such as a calcium or magnesium salt, or an ammonium salt, or a salt with an organic base such as methylamine, dimethylamine, trimethylamine, piperidine, morpholine or tris-(2-hydroxyethyl)amine.

Particular novel quinazoline derivatives of the invention include, for example, quinazoline derivatives of the Formula **I**, or pharmaceutically acceptable salts thereof, wherein, unless otherwise stated, each of R¹, R², R³, R⁴, R⁵, G¹, G², G³, G⁴ and the ring -NQ¹ has any of the meanings defined hereinbefore or in paragraphs (a) to (ff) hereinafter:-
(a) R¹ is selected from hydrogen, hydroxy, methoxy, ethoxy and methoxyethoxy;
(b) R¹ is selected from hydrogen and methoxy;
(c) R¹ is hydrogen;
(d) G¹ and G² are both hydrogen;
(e) G³ and G⁴, which are the same or different, are selected from hydrogen, chloro and fluoro (particularly hydrogen and chloro);
(f) One of G³ or G⁴ is halogeno (for example chloro) and the other of G³ and G⁴ is hydrogen;
(g) G³ is halogeno (for example chloro) and G¹, G² and G⁴ are all hydrogen;
(h) G⁴ is halogeno (for example chloro) and G¹, G² and G³ are all hydrogen;
(i) R² and R³, which are the same or different, are selected from hydrogen and (1-2C)alkyl (such as methyl);
(j) R² and R³, which are the same or different, are selected from hydrogen and (1-2C)alkyl, wherein at least one of R² and R³ is (1-2C)alkyl (such as methyl);
(k) R² is hydrogen and R³ is (1-2C)alkyl (such as methyl);
(l) R⁴ and R⁵, which may be the same or different, are selected from hydrogen and (1-4C)alkyl, which (1-4C)alkyl optionally bears one or more hydroxy substituents, or
   R⁴ and R⁵ together with the nitrogen atom to which they are attached form a saturated 4, 5, 6 or 7 membered heterocyclic ring which optionally contains one or more additional heteroatoms independently selected from oxygen, nitrogen and sulfur,
   and wherein any heterocyclic ring formed by R⁴, R⁵ and the nitrogen atom to which they are attached optionally bears one or more substituents independently selected from halogeno, cyano, hydroxy, (1-4C)alkyl and (1-4C)alkoxy,
   and wherein any heterocyclic ring formed by R⁴, R⁵ and the nitrogen atom to which they are attached optionally bears 1 or 2 oxo or thioxo substituents;
(m) R⁴ and R⁵, which may be the same or different, are selected from hydrogen and (1-4C)alkyl, which (1-4C)alkyl optionally bears one or more hydroxy substituents, or
   R⁴ and R⁵ together with the nitrogen atom to which they are attached form a heterocyclic ring selected from azetidin-1-yl, pyrrolidin-1-yl, pyrazolidin-1-yl, piperidin-1-yl, morpholin-4-yl and piperazin-1-yl, wherein any heterocyclic ring formed by R⁴, R⁵ and the nitrogen atom to which they are attached optionally bears one or more substituents independently selected from halogeno, cyano, hydroxy, (1-4C)alkyl and (1-4C)alkoxy,
   and wherein any heterocyclic ring formed by R⁴, R⁵ and the nitrogen atom to which they are attached optionally bears 1 or 2 oxo or thioxo substituents;
(n) R⁴ and R⁵, which may be the same or different, are selected from hydrogen and (1-4C)akyl, which (1-4C)alkyl optionally bears one or more hydroxy substituents, or
   R⁴ and R⁵ together with the nitrogen atom to which they are attached form a heterocyclic ring selected from pyrrolidin-1-yl and morpholin-4-yl, wherein any heterocyclic ring formed by R⁴, R⁵ and the nitrogen atom to which they are attached optionally bears one or more substituents independently selected from halogeno, cyano, hydroxy, (1-4C)alkyl and (1-4C)alkoxy,
   and wherein any heterocyclic ring formed by R⁴, R⁵ and the nitrogen atom to which they are attached optionally bears 1 or 2 oxo or thioxo substituents;
(o) R⁴ is hydrogen and R⁵ is (1-4C)alkyl, which (1-4C)alkyl optionally bears one or more hydroxy substituents;
(p) R⁴ and R⁵ are independently selected from hydrogen, methyl, ethyl and 2-hydroxyethyl;
(q) R⁴ and R⁵ are both (1-4C)alkyl, which (1-4C)alkyl optionally bears one or more hydroxy substituents;
(r) R⁴ is methyl and R⁵ is (1-4C)alkyl, which (1-4C)alkyl optionally bears one or more hydroxy substituents;
(s) R⁴ is methyl and R⁵ is selected from methyl, ethyl and 2-hydroxyethyl (particularly methyl and 2-hydroxyethyl);
(t) R⁴ and R⁵ are both methyl;
(u) R⁴ is methyl and R⁵ is 2-hydroxyethyl;
(v) R⁴ and R⁵ together with the nitrogen atom to which they are attached form a heterocyclic ring selected from pyrrolidin-1-yl and morpholin-4-yl, which heterocyclic ring optionally bears one or more substituents independently selected from halogeno, cyano, hydroxy, (1-4C)alkyl and (1-4C)alkoxy, and which heterocyclic ring optionally bears 1 or 2 oxo or thioxo substituents;
(w) R⁴ and R⁵ together with the nitrogen atom to which they are attached form a heterocyclic ring selected from pyrrolidin-1-yl and morpholin-4-yl;
(x) the ring -NQ¹ is a nitrogen-linked, saturated or partially unsaturated, 5, 6 or 7 membered heterocyclic ring containing one nitrogen heteroatom and optionally containing one or two additional heteroatoms independently selected from oxygen, nitrogen and sulfur, and which heterocyclic ring NQ¹ optionally bears one or more substituents independently selected from halogeno, cyano, hydroxy, (1-4C)alkyl, (1-4C)alkoxy and hydroxy-(1-4C)alkyl, and which heterocyclic ring NQ¹ optionally bears 1 or 2 oxo or thioxo substituents;
(y) the ring -NQ¹ is a nitrogen-linked, saturated or partially unsaturated, 5, 6 or 7 membered heterocyclic ring containing one nitrogen heteroatom, and which heterocyclic ring -NQ optionally bears one or more substituents independently selected from halogeno, cyano, hydroxy, (1-4C)alkyl, (1-4C)alkoxy and hydroxy-(1-4C)alkyl, and which heterocyclic ring -NQ¹ optionally bears 1 or 2 oxo or thioxo substituents;
(z) the ring -NQ¹ is selected from azepan-1-yl, piperidin-1-yl and pyrrolidin-1-yl, and which heterocyclic ring -NQ¹ optionally bears one or more substituents independently selected from halogeno, cyano, hydroxy, (1-4C)alkyl, (1-4C)alkoxy and hydroxy-(1-4C)alkyl, and which heterocyclic ring -NQ¹ optionally bears 1 or 2 oxo or thioxo substituents;

(aa) the ring -NQ¹ is azepan-1-yl;
(bb) the ring -NQ¹ is piperidin-1-yl;
(cc) the ring -NQ¹ is pyrrolidin-1-yl;
(dd) the ring -NQ¹ is selected from azepan-1-yl, piperidin-1-yl and pyrrolidin-1-yl;
(ee) R⁴ and R⁵, which may be the same or different, are selected from hydrogen, (1-4C)alkyl, (3-4C)alkenyl and (3-4C)alkynyl, which (1-4C)alkyl optionally bears one or more substituents independently selected from halogeno, cyano, hydroxy, amino, (1-4C)alkylamino, di-[(1-4C)alkyl]amino and (14C)alkoxy; and
(ff) R⁴ and R⁵, which may be the same or different, are selected from hydrogen, (1-4C)alkyl, (3-4C)alkenyl and (3-4C)alkynyl, which (1-4C)alkyl optionally bears one or more hydroxy substituents.

A particular group of quinazoline derivatives of the Formula **I** have the Formula **IA**: 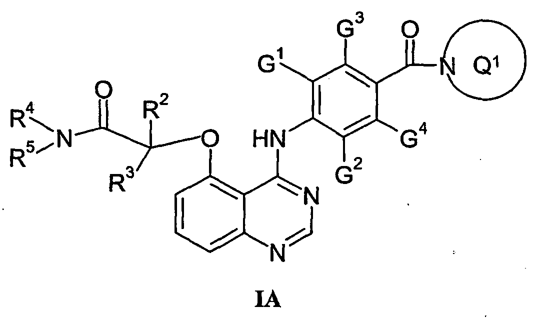 wherein:
R² and R³, which may be the same or different, are selected from hydrogen and (1-4C)akyl, which (1-4C)alkyl optionally bears one or more hydroxy substituents;
R⁴ and R⁵, which may be the same or different, are selected from hydrogen and (1-4C)alkyl, which (1-4C)alkyl optionally bears one or more substituents independently selected from halogeno, cyano, hydroxy, amino, (1-4C)alkylamino, di-[(1-4C)alkyl]amino and (1-4C)alkoxy;
G¹ and G², which may be the same or different, are selected from hydrogen and halogeno;
G³ and G⁴, which may be the same or different, are selected from hydrogen, halogeno, cyano, (1-4C)alkyl, (1-4C)alkoxy, (2-4C)alkenyl and (2-4C)alkynyl;
the ring -NQ¹ is a nitrogen-linked, saturated or partially unsaturated, 4, 5, 6, 7 or 8 membered heterocyclic ring containing one nitrogen heteroatom and optionally containing one or more additional heteroatoms independently selected from oxygen, nitrogen and sulfur, and which heterocyclic ring -NQ¹ optionally bears one or more substituents independently selected from halogeno, cyano, hydroxy, (1-4C)alkyl, (1-4C)alkoxy and hydroxy-(1-4C)alkyl, and which heterocyclic ring -NQ¹ optionally bears 1 or 2 oxo or thioxo substituents;
or a pharmaceutically acceptable salt thereof.

In a particular aspect of the quinazoline derivatives of the Formula **IA,** R² is hydrogen and R³ is (1-2C)alkyl (especially methyl).

In a particular aspect of the quinazoline derivatives of the Formula **IA,** R⁴ and R⁵, which may be the same or different, are selected from (1-4C)alkyl, which (1-4C)alkyl optionally bears one or more hydroxy substituents. For example, R⁴ may be methyl and R⁵ may be selected from methyl and 2-hydroxyethyl.

In a particular aspect of the quinazoline derivatives of the Formula **IA,** G¹ and G² are both hydrogen.

In a particular aspect of the quinazoline derivatives of the Formula **IA,** G³ and G⁴, which may be the same or different, are selected from hydrogen and halogeno. For example, G³ may be hydrogen and G⁴ may be halogeno (such as chloro).

In a particular aspect of the quinazoline derivatives of the Formula **IA,** the ring -NQ¹ is a nitrogen-linked, saturated 5, 6 or 7 membered heterocyclic ring containing one nitrogen heteroatom and optionally containing one or more additional heteroatoms independently selected from oxygen, nitrogen and sulfur, and which heterocyclic ring -NQ¹ optionally bears one or more substituents independently selected from halogeno, cyano, hydroxy, (1-4C)alkyl, (1-4C)alkoxy and hydroxy-(1-4C)alkyl, and which heterocyclic ring -NQ¹ optionally bears 1 or 2 oxo or thioxo substituents. In particular, the ring -NQ¹ may be a nitrogen-linked, saturated 5, 6 or 7 membered heterocyclic ring containing one nitrogen heteroatom, and which heterocyclic ring -NQ¹ optionally bears one or more substituents independently selected from halogeno, cyano, hydroxy, (1-4C)alkyl, (1-4C)alkoxy and hydroxy-(1-4C)alkyl, and which heterocyclic ring -NQ¹ optionally bears 1 or 2 oxo or thioxo substituents. More particularly, the ring -NQ¹ may be selected from azepan-1-yl, piperidin-1-yl and pyrrolidin-1-yl.

It is to be understood that in the quinazoline derivatives of the Formula **IA**, the group at the 7-position on the quinazoline ring is hydrogen (i.e. R¹ in the compounds of the Formula **I**).

Another particular group of quinazoline derivatives of the Formula **I** have the Formula **IB:** 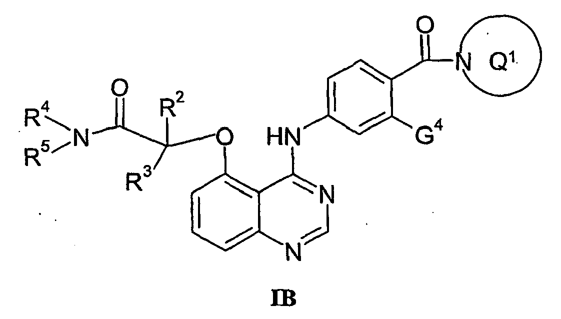 wherein:
R² and R³, which may be the same or different, are selected from hydrogen and (1-4C)alkyl, which (1-4C)alkyl optionally bears one or more hydroxy substituents;
R⁴ and R⁵, which may be the same or different, are selected from hydrogen and (1-4C)alkyl, which (1-4C)alkyl optionally bears one or more substituents independently selected from halogeno, cyano, hydroxy, amino, (1-4C)alkylamino, di-[(1-4C)alkyl]amino and (1-4C)alkoxy;
G⁴ is selected from halogeno, cyano, (1-4C)alkyl, (1-4C)alkoxy, (2-4C)alkenyl and (2-4C)alkynyl;
the ring -NQ¹ is a nitrogen-linked, saturated or partially unsaturated, 4, 5, 6, 7 or 8 membered heterocyclic ring containing one nitrogen heteroatom and optionally containing one or more additional heteroatoms independently selected from oxygen, nitrogen and sulfur, and which heterocyclic ring -NQ¹ optionally bears one or more substituents independently selected from halogeno, cyano, hydroxy, (1-4C)alkyl, (1-4C)alkoxy and hydroxy-(1-4C)alkyl, and which heterocyclic ring -NQ¹ optionally bears 1 or 2 oxo or thioxo substituents;
or a pharmaceutically acceptable salt thereof.

In a particular aspect of the quinazoline derivatives of the Formula **IB,** R² is hydrogen and R³ is (1-2C)alkyl (especially methyl).

In a particular aspect of the quinazoline derivatives of the Formula **IB,** R⁴ and R⁵, which may be the same or different, are selected from (1-4C)alkyl, which (1-4C)alkyl optionally bears one or more hydroxy substituents. For example, R⁴ may be methyl and R⁵ may be selected from methyl and 2-hydroxyethyl.

In a particular aspect of the quinazoline derivatives of the Formula **IB**, G⁴ is halogeno (especially chloro).

In a particular aspect of the quinazoline derivatives of the Formula **IB**, the ring -NQ¹ is a nitrogen-linked, saturated 5, 6 or 7 membered heterocyclic ring containing one nitrogen heteroatom and optionally containing one or more additional heteroatoms independently selected from oxygen, nitrogen and sulfur, and which heterocyclic ring -NQ¹ optionally bears one or more substituents independently selected from halogeno, cyano, hydroxy, (1-4C)alkyl, (1-4C)alkoxy and hydroxy-(1-4C)alkyl, and which heterocyclic ring -NQ¹ optionally bears 1 or 2 oxo or thioxo substituents. In particular, the ring -NQ¹ may be a nitrogen-linked, saturated 5, 6 or 7 membered heterocyclic ring containing one nitrogen heteroatom, and which heterocyclic ring -NQ¹ optionally bears one or more substituents independently selected from halogeno, cyano, hydroxy, (1-4C)alkyl, (1-4C)alkoxy and hydroxy-(1-4C)alkyl, and which heterocyclic ring -NQ¹ optionally bears 1 or 2 oxo or thioxo substituents. More particularly, the ring -NQ¹ may be selected from azepan-1-yl, piperidin-1-yl and pyrrolidin-1-yl.

It is to be understood that in the quinazoline derivatives of the Formula **IB**, the group at the 7-position on the quinazoline ring and the groups at the 2-, 5- and 6-positions on the phenyl ring of the anilino group are hydrogen (i.e. R¹, G¹, G² and G³ in the compounds of the Formula **I**).

Another particular group of quinazoline derivatives of the Formula **I** have the Formula **IC**: 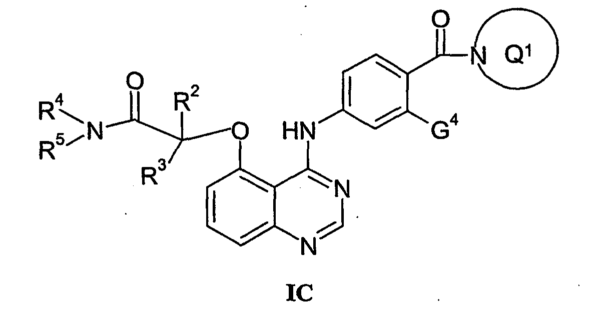 wherein:
R² is hydrogen;
R³ is (1-2C)alkyl (especially methyl);
R⁴ is (1-2C)alkyl (especially methyl);
R⁵ is (1-2C)alkyl, which (1-2C)alkyl optionally bears one or more hydroxy substituents;
G⁴ is halogeno (especially chloro);
the ring -NQ¹ is a nitrogen-linked, saturated 5, 6 or 7 membered heterocyclic ring containing one nitrogen heteroatom, and which heterocyclic ring -NQ¹ optionally bears one or more substituents independently selected from halogeno, hydroxyl and (1-4C)alkyl, and which heterocyclic ring -NQ¹ optionally bears 1 or 2 oxo or thioxo substituents;
or a pharmaceutically acceptable salt thereof.

In a particular aspect of the quinazoline derivatives of the Formula **IC,** R⁵ is selected from methyl and 2-hydroxyethyl.

In a particular aspect of the quinazoline derivatives of the Formula **IC,** the ring -NQ¹ is selected from azepan-1-yl, piperidin-1-yl and pyrrolidin-1-yl.

It is to be understood that in the quinazoline derivatives of the Formula **IC,** the group at the 7-position on the quinazoline ring and the groups at the 2-, 5- and 6-positions on the phenyl ring of the anilino group are hydrogen (i.e. R¹, G¹, G² and G³ in the compounds of the Formula **I**).

Particular quinazoline derivatives of the invention are, for example, one or more quinazoline derivatives of the Formula **I** selected from:
(2*R*)-2-[(4-{[4-(azepan-1-ylcarbonyl)-3-chlorophenyl]amino}quinazolin-5-yl)oxy]-*N*-(2-hydroxyethyl)-*N-*methylpropanamide;
(2*R*)-2-[(4-{[3-chloro-4-(piperidin-1-ylcarbonyl)phenyl]amino}quinazolin-5-yl)oxy]-*N-*(2-hydroxyethyl)-*N-*methylpropanamide;
(2*R*)-2-[(4-{[3-chloro-4-(pyrrolidin-1-ylcarbonyl)phenyl]amino}quinazolin-5-yl)oxy]-*N-*(2-hydroxyethyl)-*N-*methylpropanamide;
(2*R*)-2-[(4-{[4-(azepan-1-ylcarbonyl)-3-chlorophenyl]amino}quinazolin-5-yl)oxy]-*N,N-*dimethylpropanamide;
(2*R*)-2-[(4-{[3-chloro-4-(piperidin-1-ylcarbonyl)phenyl]amino}quinazolin-5-yl)oxy]-*N,N-*dimethylpropanamide; and
(2*R*)-2-[(4-{[3-chloro-4-(pyrrolidin-1-ylcarbonyl)phenyl]amino}quinazolin-5-yl)oxy]-*N,N-*dimethylpropanamide;
or a pharmaceutically acceptable salt thereof.

A quinazoline derivative of the Formula **I**, or a pharmaceutically acceptable salt thereof, may be prepared by any process known to be applicable to the preparation of chemically-related compounds. Suitable processes include, for example, those illustrated in WO 96/15118, WO 01/94341, WO 03/040108 and WO 03/040109. Such processes, when used to prepare a quinazoline derivative of the Formula **I** are provided as a further feature of the invention and are illustrated by the following representative process variants in which, unless otherwise stated, R¹, R², R³, R⁴, R⁵, G¹ G², G³, G⁴ and the ring -NQ¹ have any of the meanings defined hereinbefore. Necessary starting materials may be obtained by standard procedures of organic chemistry. The preparation of such starting materials is described in conjunction with the following representative process variants and within the accompanying Examples. Alternatively, necessary starting materials are obtainable by analogous procedures to those illustrated, which are within the ordinary skill of an organic chemist.

### Process (a) The reaction of a quinazoline of the Formula II:

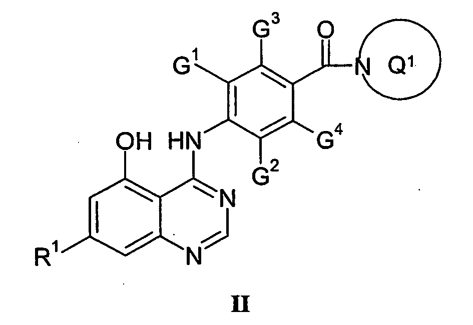

wherein R¹, G¹, G², G³, G⁴ and the ring -NQ¹ have any of the meanings defined hereinbefore except that any functional group is protected if necessary, with an amide of the Formula **III**: 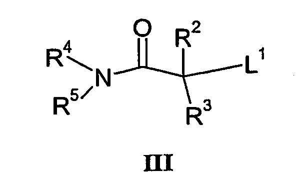 wherein R², R³, R⁴ and R⁵ have any of the meanings defined hereinbefore except that any functional group is protected if necessary and L¹ is a suitable displaceable group, such as halogeno (for example chloro or bromo), a sulfonyloxy group (for example a methylsulfonyloxy or a toluene-4-sulfonyloxy group) or a hydroxy group;
or

### Process (b) The coupling, conveniently in the presence of a suitable base, of a quinazoline of the Formula IV (or a suitable salt thereof, for example an alkali earth metal salt or an alkali metal salt, such as a sodium or a potassium salt, thereof):

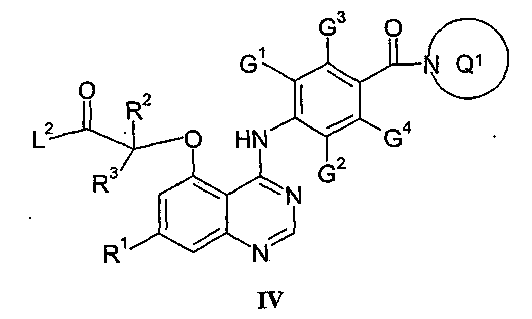

wherein R¹, R², R³, G¹, G², G³, G⁴ and the ring -NQ¹ have any of the meanings defined hereinbefore except that any functional group is protected if necessary, and L² is a suitable displaceable group, for example (1-3C)alkoxy (such as methoxy or ethoxy) or L² is hydroxy, which hydroxy group is conveniently combined with a suitable coupling agent to produce a displaceable group, with an amine of the Formula **V:** 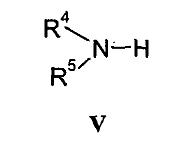 wherein R⁴ and R⁵ have any of the meanings defined hereinbefore except that any functional group is protected if necessary;
or

### Process (c) For quinazoline derivatives of the Formula I wherein R² is 2-hydroxyethyl, the reaction of a quinazoline of the Formula VI:

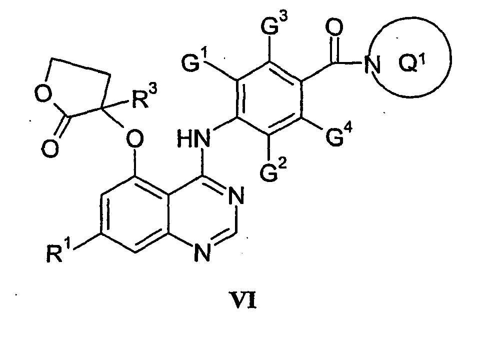

wherein R¹, R³, G¹, G², G³, G⁴ and the ring -NQ¹ have any of the meanings defined hereinbefore except that any functional group is protected if necessary, with an amine of the Formula **V** as defined above;
or

### Process (d) The reaction of a quinazoline of the Formula VII:

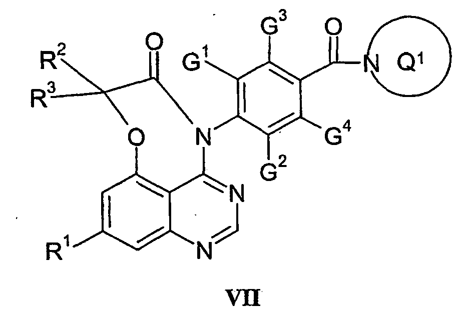

wherein R¹, R², R³, G¹, G², G³, G⁴ and the ring -NQ¹ have any of the meanings defined hereinbefore except that any functional group is protected if necessary, with an amine of the Formula **V** as defined above;
or

### Process (e) The reaction of a quinazolin-4(3H)-one of the Formula VIII:

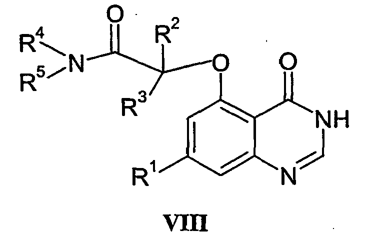

wherein R¹, R², R³, R⁴ and R⁵ have any of the meanings defined hereinbefore except that any functional group is protected if necessary, with a suitable activating group and an amine of the Formula **IX:** 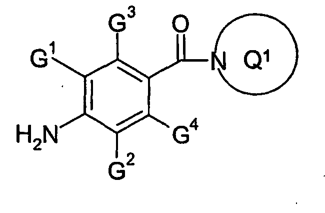 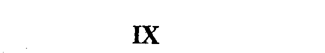 wherein G¹, G², G³, G⁴ and the ring -NQ¹ have any of the meanings defined hereinbefore except that any functional group is protected if necessary;
or

### Process (f) The reaction of a quinazoline of the Formula X:

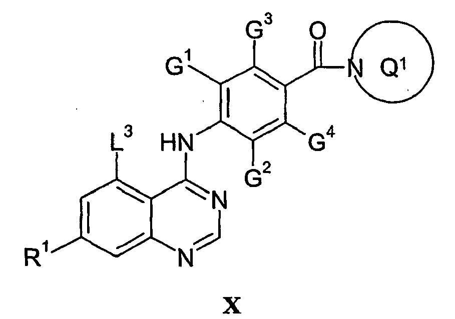

wherein R¹, G¹, G², G³, G⁴ and the ring -NQ¹ have any of the meanings defined hereinbefore except that any functional group is protected if necessary and L³ is a suitable displaceable group such as halogeno (for example fluoro) with a compound of the Formula **XI:** 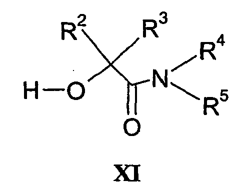 wherein R², R³, R⁴ and R⁵ have any of the meanings defined hereinbefore except that any functional group is protected if necessary;
or

### Process (g) The coupling of a quinazoline of the Formula XII:

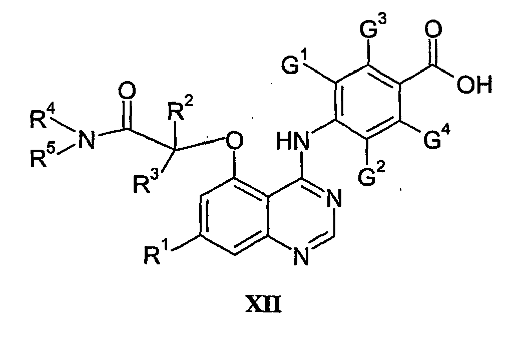

wherein R¹, R², R³, R⁴, R⁵, G¹, G², G³ and G⁴ have any of the meanings defined hereinbefore except that any functional group is protected if necessary, with a cyclic amine compound of the Formula **XIII**: 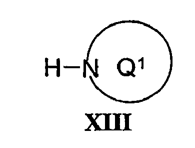 wherein the ring -NQ¹ has any of the meanings defined hereinbefore except that any functional group is protected if necessary;
and thereafter, if necessary:
(i) converting a quinazoline derivative of the Formula **I** into another quinazoline derivative of the Formula **I**;
(ii) removing any protecting group that is present (by conventional means);
(iii) forming a pharmaceutically acceptable salt.

Specific conditions for the above reactions are as follows:

### Process (a)

When L¹ is, for example; halogeno or a sulfonyloxy group, the reaction of process (a) is conveniently carried out in the presence of a suitable base. A suitable base is, for example, an alkali or alkaline earth metal carbonate, such as sodium carbonate, potassium carbonate, caesium carbonate or calcium carbonate. The reaction is, optionally, carried out in the presence of a source of iodide such as sodium iodide or potassium iodide or in the presence of a suitable alkali metal hydride such as sodium hydride or potassium hydride.

The reaction is conveniently carried out in the presence of a suitable inert solvent or diluent, for example an ester such as ethyl acetate, a halogenated solvent such as methylene chloride, chloroform or carbon tetrachloride, an ether such as tetrahydrofuran or 1,4-dioxan, an aromatic solvent such as toluene, an alcohol such as methanol or ethanol, or a dipolar aprotic solvent such as N,N-dimethylformamide, N,N-dimethylacetamide, N-methylpyrrolidin-2-one or dimethylsulfoxide. The reaction is conveniently carried out at a temperature in the range, for example, from 0 to 120°C, conveniently at or near ambient temperature and/or at about 50°C.

When L¹ is hydroxy, the reaction of process (a) is conveniently carried out under suitable Mitsunobu conditions. Suitable Mitsunobu conditions include, for example, reaction in the presence of a suitable tertiary phosphine and a di-alkylazodicarboxylate in an organic solvent such as THF, or suitably dichloromethane, and in the temperature range 0°C to 60°C, but conveniently at ambient temperature. A suitable tertiary phosphine includes for example tri-n-butylphosphine or suitably tri-phenylphosphine. A suitable di-alkylazodicarboxylate includes for example diethyl azodicarboxylate (DEAD) or suitably di-tert-butyl azodicarboxylate (DTAD). Details of Mitsunobu reactions are contained in Tet. Letts., 31, 699, (1990); The Mitsunobu Reaction, D.L.Hughes, Organic Reactions, 1992, Vol.42, 335-656 and Progress in the Mitsunobu Reaction, D.L.Hughes, Organic Preparations and Procedures International, 1996, Vol.28, 127-164.

### Process (b)

When L² is hydroxy, the reaction of process (b) is conveniently carried out in the presence of a suitable coupling agent. A suitable coupling agent is, for example, a suitable peptide coupling agent, such as O-(7-azabenzotriazol-1-yl)-*N,N,N',N'*-tetramethyluronium hexafluoro-phosphate (HATU) or a carbodiimide such as dicyclohexylcarbodiimide or 1-(3-dimethylaminopropyl)-3-ethylcarbodiimide hydrochloride (EDCI). The reaction of process (b) is optionally carried out in the presence of a suitable catalyst such as dimethylaminopyridine, 4-pyrrolidinopyridine, 2-hydroxypyridine *N-*oxide (HOPO) or 1-hydroxybenzotriazole (HOBT).

When L² is hydroxy, the reaction of process (b) may conveniently be carried out in the presence of a suitable base. A suitable base is, for example, an organic amine base such as pyridine, 2,6-lutidine, collidine, 4-dimethylaminopyridine, triethylamine, di-isopropylethylamine, N-methylmorpholine or diazabicyclo[5.4.0]undec-7-ene, or an alkali or alkaline earth metal carbonate, such as sodium carbonate, potassium carbonate, caesium carbonate or calcium carbonate.

The reaction of process (b) is conveniently carried out in the presence of a suitable inert solvent or diluent, for example an ester such as ethyl acetate, a halogenated solvent such as methylene chloride, chloroform or carbon tetrachloride, an ether such as tetrahydrofuran or 1,4-dioxan, an aromatic solvent such as toluene, an alcohol such as methanol or ethanol, or a dipolar aprotic solvent such as N,N-dimethylformamide, N,N-dimethylacetamide, N-methylpyrrolidin-2-one or dimethylsulfoxide. The reaction is conveniently carried out at a temperature in the range, for example, from 0 to 120°C. When L² is hydroxy, the reaction may conveniently be carried out at or near ambient temperature. When L² is (C1-C3)alkoxy, the reaction may conveniently be carried out at or near about 60°C.

Conveniently, this reaction may also be performed by heating the reactants in a sealed vessel using a suitable heating apparatus such as a microwave heater.

### Process (c)

The reaction of process (c) is conveniently carried out in the presence of a suitable inert solvent or diluent, for example an ester such as ethyl acetate, a halogenated solvent such as methylene chloride, chloroform or carbon tetrachloride, an ether such as tetrahydrofuran or 1,4-dioxan, an aromatic solvent such as toluene, an alcohol such as ethanol, or a dipolar aprotic solvent such as N,N-dimethylformamide, N,N-dimethylacetamide, N-methylpyrrolidin-2-one or dimethylsulfoxide. The reaction is conveniently carried out at a temperature in the range, for example, from 0 to 120°C, conveniently at or near ambient temperature.

### Process (d)

The reaction of process (d) is conveniently carried out in the presence of a suitable inert solvent or diluent, for example an ester such as ethyl acetate, a halogenated solvent such as methylene chloride, chloroform or carbon tetrachloride, an ether such as tetrahydrofuran or 1,4-dioxan, an aromatic solvent such as toluene, an alcohol such as methanol or ethanol, or a dipolar aprotic solvent such as N,N-dimethylformamide, N,N-dimethylacetamide, N-methylpyrrolidin-2-one or dimethylsulfoxide. The reaction is conveniently carried out at a temperature in the range, for example, from 0 to 120°C, conveniently at or near ambient temperature.

### Process (e)

In process (e), the quinazolin-4(3H)-one of the Formula **VIII** is conveniently reacted with a suitable activating agent, so as to replace the oxo group at the 4-position on the quinazolin-4(3H)-one ring by a suitable displaceable group, for example halogeno (such as chloro) and to form a quinazoline (hereinafter referred to as the "activated quinazoline") for reaction with the amine of the Formula **IX.** The activated quinazoline so formed may conveniently be used *in situ* without further purification.

The reaction of the quinazolin4(3H)-one of the Formula **VIII** with a suitable activating agent is conveniently carried out using conventional methods. For example, the quinazolin-4(3H)-one of the Formula **VIII** may be reacted with a suitable halogenating agent such as thionyl chloride, phosphoryl chloride or a mixture of carbon tetrachloride and triphenylphosphine.

The reaction of the activated quinazoline with the amine of the Formula **IX** is conveniently carried out in the presence of an acid, for example in the presence of a catalytic amount of an acid. Suitable acids include, for example hydrogen chloride gas (conveniently dissolved in a suitable inert solvent such as diethyl ether or dioxane) or hydrochloric acid.

Alternatively, the reaction of the activated quinazoline with the amine of the Formula **IX** may be carried out in the presence of a suitable base. A suitable base is, for example, N,N-diisopropyldiethylamine.

Alternatively, when the activated quinazoline contains a halogeno group (for example chloro) at the 4-position on the quinazoline ring, the reaction with the amine of the Formula **IX** may be carried out in the absence of an acid or a base. In this reaction displacement of the halogeno leaving group results in the formation of the acid (H-halogeno) *in-situ* and the autocatalysis of the reaction.

The above reactions are conveniently carried out in the presence of a suitable inert solvent or diluent, for example an alcohol or ester such as methanol, ethanol, isopropanol or ethyl acetate, a halogenated solvent such as dichloroethane, methylene chloride, chloroform or carbon tetrachloride, an ether such as tetrahydrofuran, diethyl ether or 1,4-dioxan, an aromatic solvent such as toluene, or a dipolar aprotic solvent such as N,N-dimethylformamide, N,N-dimethylacetamide, N-methylpyrrolidin-2-one or dimethylsulfoxide.

When conducted in the presence or absence of an acid, the above reactions are conveniently carried out at a temperature in the range, for example, 0 to 250°C, conveniently in the range 40 to 80°C or, preferably, at or near the reflux temperature of the solvent when used. When conducted in the presence of a base, the above reactions are conveniently carried out at a temperature in the range, for example, -78 to 30°C.

### Process (f)

Process (f) may conveniently be carried out in the presence of a suitable base. A suitable base is, for example, an alkali metal hydride, such as sodium hydride.

The reaction is conveniently carried out in the presence of a suitable inert solvent or diluent, for example an ether such as tetrahydrofuran or 1,4-dioxan, an aromatic solvent such as toluene, or a dipolar aprotic solvent such as N,N-dimethylformamide, N,N-dimethylacetamide, N-methylpyrrolidin-2-one or dimethylsulfoxide. The reaction is conveniently carried out at a temperature in the range, for example, from 0 to 120°C.

### Process (g)

The reaction of a quinazoline of the Formula **XII** with a compound of the Formula **XIII** may conveniently be carried out using analogous conditions to those used in process (b) when L² is hydroxy as discussed above.

### Starting Materials

### Starting Materials for Process (a)

The quinazoline of the Formula **II** may be obtained by conventional procedures, for example as illustrated in *Reaction Scheme 1:* 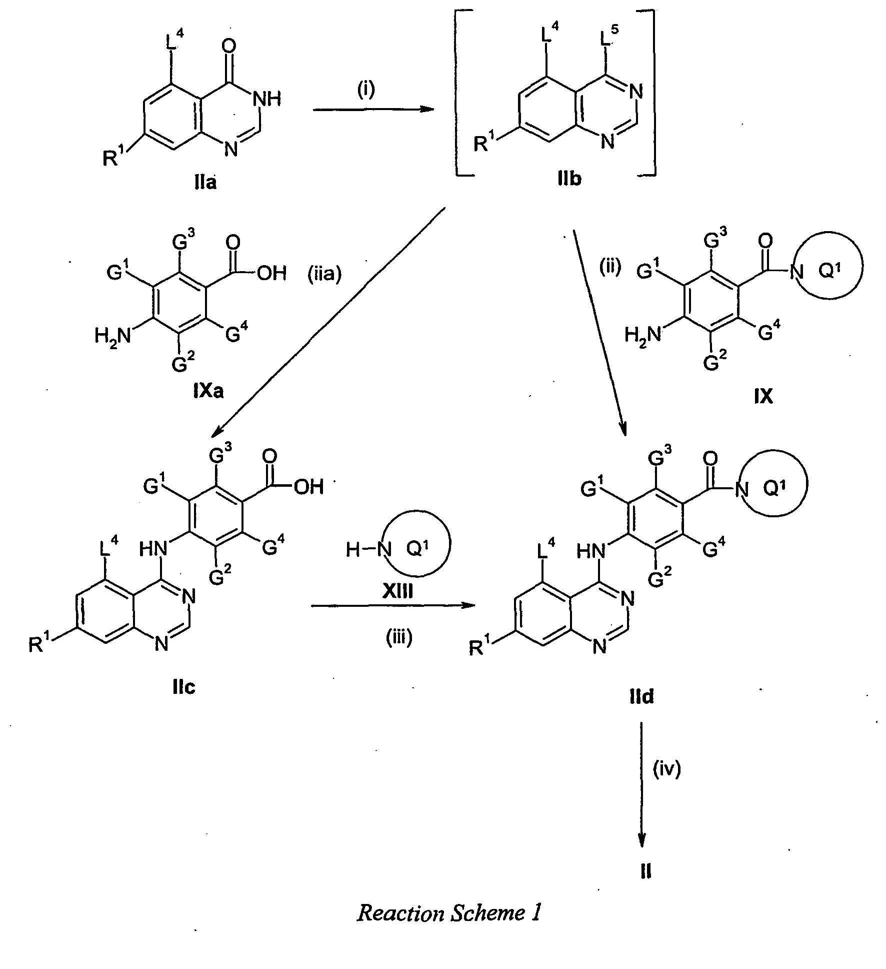 wherein L⁴ and L⁵ are suitable displaceable groups, provided that L⁵ is more labile than L⁴ and R¹, G¹, G², G³, G⁴ and the ring -NQ¹ have any of the meanings defined hereinbefore except that any functional group is protected if necessary.

A suitable displaceable group L⁴ is, for example, halogeno or a sulfonyloxy group, such as fluoro, chloro, methylsulfonyloxy or toluene-4-sulfonyloxy, particularly fluoro. A suitable displaceable group L⁵ is, for example, halogeno or an alkoxy, aryloxy, mercapto, alkylthio, arylthio, alkylsulfinyl, arylsulfinyl, alkylsulfonyl, arylsulfonyl, alkylsulfonyloxy or arylsulfonyloxy group, for example a chloro, bromo, methoxy, phenoxy, pentafluorophenoxy, methylthio, methanesulfonyl, methanesulfonyloxy or toluene-4-sulfonyloxy group. Preferably L⁴ and L⁵ are both halogeno, for example L⁴ is fluoro and L⁵ is chloro.

### Notes for Reaction Scheme 1:

### Step (i)

As the skilled person would appreciate, the conversion of a quinazolone of the Formula **IIa** to a quinazoline of the Formula **IIb** may be conducted using conventional methods, for example by reacting the compound of the Formula **IIa** with a suitable activating agent. For example, when L⁴ is fluoro and L⁵ is halogeno (for example chloro), 5-fluoro-quinazolin-4(3H)-one may be reacted with a suitable halogenating agent such as thionyl chloride, phosphoryl chloride or a mixture of carbon tetrachloride and triphenylphosphine.

### Steps (ii) and (iia)

The reaction of the quinazoline of the Formula **IIb** with the amine of the Formula **IX** or **IXa** may conveniently be carried out using analogous conditions to those used in process (e) as discussed above. The compound of the Formula **IIb** may be used *in situ* without purification.

### Step (iii)

The reaction of step (iii) may conveniently be carried out using analogous conditions to those used in process (g) as discussed above.

### Step (iv)

The conversion of a quinazoline of the Formula **IId** to a quinazoline of the Formula **II** may be carried out by reaction with a suitably protected oxygen nucleophile, followed by removal of the protecting group by conventional means. For example, the conversion may conveniently be carried out by reaction with *N-*acetylethanolamine in the presence of a suitable base. A suitable base is, for example, a strong non-nucleophilic base such as an alkali metal hydride (for example sodium hydride) or an alkali metal amide (for example lithium di-isopropylamide (LDA)). The reaction is conveniently carried out in the presence of a suitable inert solvent or diluent, for example an ether such as tetrahydrofuran or 1,4-dioxane, an aromatic solvent such as toluene, or a dipolar aprotic solvent such as N,N-dimethylformamide, N,N-dimethylacetamide, N-methylpyrrolidin-2-one or dimethylsulfoxide. The reaction is conveniently carried out at a temperature in the range, for example, from 10 to 250°C, preferably in the range from 100 to 150°C.

The conversion may alternatively be carried out by reaction with a suitable alkali metal alkoxide (for example sodium methoxide), followed by a conventional demethylation reaction. Any suitable demethylation reaction conditions may be used. For example, the demethylation step may be carried out by reaction with pyridinium hydrochloride at a temperature in the range from 50 to 180°C, by reaction with boron tribromide at a temperature in the range from -78 to 30°C or by reaction with a suitable thiolate, such as sodium thiophenolate at a temperature in the range from 50 to 200°C.

### Starting Materials for Reaction Scheme 1

The compounds of the Formula **IIa** are commercially available or may be prepared using conventional methods. For example, the 5-fluoro-quinazolin-4(3H)-one starting material is commercially available or can be prepared using conventional methods, for example as described in J. Org. Chem. 1952, 17, 164-176.

Compounds of the Formulae **IX** and **IXa** are commercially available compounds or they are known in the literature, or they can be prepared by standard processes known in the art. For example, compounds of the Formulae **IX** and **IXa** may be prepared in accordance with *Reaction Scheme 2:* 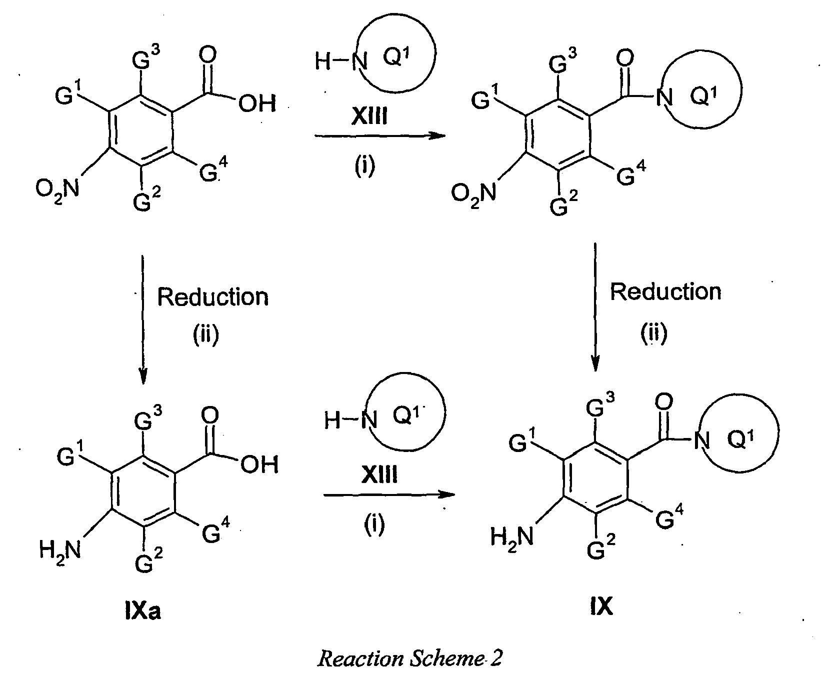 wherein G¹, G², G³, G⁴ and the ring -NQ¹ have any of the meanings defined hereinbefore except that any functional group is protected if necessary.

### Notes for Reaction Scheme 2:

### Step (i)

The reaction of step (i) may conveniently be carried out using analogous conditions to those used in process (g) as discussed above.

### Step (ii)

As the skilled person would appreciate, the reduction in step (ii) of *Reaction Scheme 2* may be conducted using conventional methods. For example, the reduction of the nitro group in step (ii) may be carried out under standard conditions, for example by catalytic hydrogenation over a platinum/carbon, palladium/carbon, platinum (IV) oxide or nickel catalyst, treatment with a metal such as iron, titanium (III) chloride, tin (II) chloride or indium, or treatment with another suitable reducing agent such as sodium dithionite.

The amides of the Formula **III** are commercially available, or they are known in the literature, or can be prepared using well-known processes in the art.

### Starting Materials for Process (b)

The quinazoline of the Formula **IV** may be obtained by conventional procedures. For example quinazoline compounds of the Formula **IV** wherein L² is (1-3C)alkoxy (such as methoxy) may be prepared by reaction of a compound of the Formula **II** as defined above or a compound of the Formula **IId** as defined above with a compound of the Formula **IVa:** 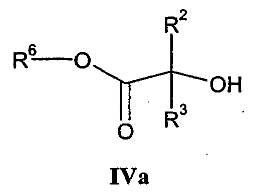 wherein R⁶ is a (1-3C)alkyl group and R² and R³ have any of the meanings defined hereinbefore except that any functional group is protected if necessary.

The reaction of a compound of the Formula **II** with a compound of the Formula **IVa** may conveniently be carried out under suitable Mitsunobu conditions as described above;

The reaction of a compound of the Formula **IId** with a compound of the Formula **IVa** is conveniently carried out in the presence of a suitable base. A suitable base is, for example, an alkali metal alkoxide, such as sodium methoxide or sodium ethoxide.

Quinazoline compounds of the Formula **IV** wherein L² is hydroxy (or a suitable salt thereof) may be prepared by reaction of a compound of the Formula **IV** wherein L² is (1-3C)alkoxy with a suitable alkali metal hydroxide, for example sodium hydroxide at room temperature. This reaction is conveniently carried out in the presence of a suitable inert solvent or diluent, for example an ether such as tetrahydrofuran or 1,4-dioxane or an alcohol such as methanol.

Quinazoline compounds of the Formula **IV** wherein L² is hydroxy (or a suitable salt thereof) may alternatively be prepared by reaction of a compound of the Formula **II** with a suitable halogenated (for example chlorinated) alcohol under suitable chlorotone reaction conditions, as appreciated by a person skilled in the art and, for example, described in Reference Example 27 of WO 03/077847.

The compounds of the Formulae **IVa** and **V** are commercially available, or they are known in the literature, or can be prepared using well-known processes in the art.

### Starting Materials for Process (c)

The compounds of the Formula **VI** can be prepared using well-known processes in the art. For example, the compounds of the Formula **VI** can be prepared by reaction of a compound of the Formula **II** as defined above with a compound of the Formula **VIa:** 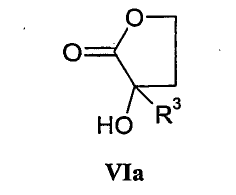 wherein R³ has any of the meanings defined hereinbefore except that any functional group is protected if necessary, for example under suitable Mitsunobu conditions, as discussed above.

The compounds of the Formula **V** and **VIa** are commercially available, or they are known in the literature, or can be prepared using well-known processes in the art.

### Starting Materials for Process (d)

The compounds of the Formula **V** are discussed above.

The compounds of the Formula **VII** may be prepared from compounds of the Formula **IV** wherein L² is hydroxy by an internal coupling reaction using a suitable coupling agent and a suitable base as described above (for example HATU and di-isopropylethylamine) under the reaction conditions discussed above for process (b).

### Starting Materials for Process (e)

The compounds of the Formula **VIII** may be prepared using well-known processes in the art. Compounds of the Formula **VIII** may, for example, be prepared by reaction of an appropriate quinazolin-4(3H)-one compound of the Formula **VIIIa:** 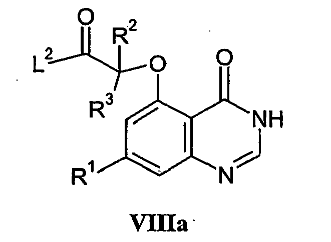 wherein L² is a suitable displaceable group as defined above or L² is hydroxy and R¹, R² and R³ have any of the meanings defined hereinbefore except that any functional group is protected if necessary, with a compound of the Formula **V** as defined above. This reaction may conveniently be carried out using analogous conditions to those used in process (b) as discussed above.

Alternatively, compounds of the Formula **VIII** may, for example, be prepared by the reaction of an appropriate quinazolin-4(3H)-one compound of the Formula **VIIIb:** 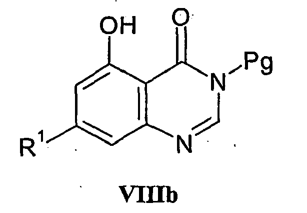 wherein Pg is a suitable appropriate protecting group (such as the pivaloyloxymethyl group) and R¹ has any of the meanings defined hereinbefore except that any functional group is protected if necessary, with an amide of the Formula **III** as defined above wherein L¹ in the amide of the Formula **III** is hydroxy. This reaction is typically conducted under suitable Mitsunobu conditions, as discussed above.

The compounds of the Formula **VIIIa** and **VIIIb** are commercially available, or they are known in the literature, or they can be prepared using well-known processes in the art.

The compounds of the Formula **IX** are commercially available, or they are known in the literature, or can be prepared using well-known processes in the art (for example as described in *Reaction Scheme* 2 above).

### Starting Materials for Process (f)

Quinazolines of the Formula **X** may be prepared using processes as discussed above, for example as discussed in *Reaction Scheme 1.*

The compounds of the Formula **XI** are commercially available, or they are known in the literature, or can be prepared using well-known processes in the art.

### Starting Materials for Process (g)

Quinazolines of the Formula **XII** may be prepared using processes as discussed above, for example as discussed in processes (a) to (f) above.

Compounds of the Formula **XIII** are commercially available compounds or they are known in the literature, or they can be can be prepared by standard processes known in the art.

The quinazoline derivative of the Formula **I** may be obtained from the above processes in the form of the free base or alternatively it may be obtained in the form of a salt, for example an acid addition salt. When it is desired to obtain the free base from a salt of the quinazoline derivative of the Formula **I,** the salt may be treated with a suitable base, for example, an alkali or alkaline earth metal carbonate or hydroxide, for example sodium carbonate, potassium carbonate, calcium carbonate, sodium hydroxide or potassium hydroxide, or by treatment with ammonia for example using a methanolic ammonia solution such as 7N ammonia in methanol.

The protecting groups used in the processes above may in general be chosen from any of the groups described in the literature or known to the skilled chemist as appropriate for the protection of the group in question and may be introduced by conventional methods. Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Specific examples of protecting groups are given below for the sake of convenience, in which "lower", as in, for example, lower alkyl, signifies that the group to which it is applied preferably has 1 to 4 carbon atoms. It will be understood that these examples are not exhaustive. Where specific examples of methods for the removal of protecting groups are given below these are similarly not exhaustive. The use of protecting groups and methods of deprotection not specifically mentioned are, of course, within the scope of the invention.

A carboxy protecting group may be the residue of an ester-forming aliphatic or arylaliphatic alcohol or of an ester-forming silanol (the said alcohol or silanol preferably containing 1 to 20 carbon atoms). Examples of carboxy protecting groups include straight or branched chain (1 to 12C)alkyl groups (for example isopropyl and tert-butyl); lower alkoxy- lower alkyl groups (for example methoxymethyl, ethoxymethyl and isobutoxymethyl); lower acyloxy-lower alkyl groups, (for example acetoxymethyl, propionyloxymethyl, butyryloxymethyl and pivaloyloxymethyl); lower alkoxycarbonyloxy-lower alkyl groups (for example 1-methoxycarbonyloxyethyl and 1-ethoxycarbonyloxyethyl); aryl-lower alkyl groups (for example benzyl, 4-methoxybenzyl, 2-nitrobenzyl, 4-nitrobenzyl, benzhydryl and phthalidyl); tri(lower alkyl)silyl groups (for example trimethylsilyl and tert-butyldimethylsilyl); tri(lower alkyl)silyl-lower alkyl groups (for example trimethylsilylethyl); and (2-6C)alkenyl groups (for example allyl). Methods particularly appropriate for the removal of carboxyl protecting groups include for example acid-, base-, metal- or enzymically-catalysed cleavage.

Examples of hydroxy protecting groups include lower alkyl groups (for example tert-butyl), lower alkenyl groups (for example allyl); lower alkanoyl groups (for example acetyl); lower alkoxycarbonyl groups (for example tert-butoxycarbonyl); lower alkenyloxycarbonyl groups (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitiobenzyloxycarbonyl); tri(lower alkyl)silyl (for example trimethylsilyl and tert-butyldimethylsilyl) and aryl-lower alkyl (for example benzyl) groups.

Examples of amino protecting groups include formyl, aryl-lower alkyl groups (for example benzyl and substituted benzyl, 4-methoxybenzyl, 2-nitrobenzyl and 2,4-dimethoxybenzyl, and triphenylmethyl); di-4-anisylmethyl and furylmethyl groups; lower alkoxycarbonyl (for example tert-butoxycarbonyl); lower alkenyloxycarbonyl (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitrobenzyloxycarbonyl); lower alkanoyloxyalkyl groups (for example pivaloyloxymethyl); trialkylsilyl (for example trimethylsilyl and tert-butyldimethylsilyl); alkylidene (for example methylidene) and benzylidene and substituted benzylidene groups.

Methods appropriate for removal of hydroxy and amino protecting groups include, for example, acid-, base-, metal- or enzymically-catalysed hydrolysis for groups such as 2-nitrobenzyloxycarbonyl, hydrogenation for groups such as benzyl and photolytically for groups such as 2-nitrobenzyloxycarbonyl. For example a tert butoxycarbonyl protecting group may be removed from an amino group by an acid catalysed hydrolysis using trifluoroacetic acid.

The reader is referred to Advanced Organic Chemistry, 4th Edition, by J. March, published by John Wiley & Sons 1992, for general guidance on reaction conditions and reagents and to Protective Groups in Organic Synthesis, 2nd Edition, by T. Green et al., also published by John Wiley & Son, for general guidance on protecting groups.

It will be appreciated that certain of the various ring substituents in the quinazoline derivatives of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogeno group.

When a pharmaceutically acceptable salt of a quinazoline derivative of the Formula **I** is required, for example an acid-addition salt, it may be obtained by, for example, reaction of said quinazoline derivative with a suitable acid using a conventional procedure.

As mentioned hereinbefore some of the compounds according to the present invention may contain one or more chiral centers and may therefore exist as stereoisomers. Stereoisomers may be separated using conventional techniques, e.g. chromatography or fractional crystallisation. The enantiomers may be isolated by separation of a racemate for example by fractional crystallisation, resolution or HPLC. The diastereoisomers may be isolated by separation by virtue of the different physical properties of the diastereoisomers, for example, by fractional crystallisation, HPLC or flash chromatography. Alternatively particular stereoisomers may be made by chiral synthesis from chiral starting materials under conditions which will not cause racemisation or epimerisation, or by derivatisation, with a chiral reagent. When a specific stereoisomer is isolated it is suitably isolated substantially free for other stereoisomers, for example containing less than 20%, particularly less than 10% and more particularly less than 5% by weight of other stereoisomers.

In the section above relating to the preparation of the quinazoline derivative of the Formula **I**, the expression "inert solvent" refers to a solvent which does not react with the starting materials, reagents, intermediates or products in a manner which adversely affects the yield of the desired product.

Persons skilled in the art will appreciate that, in order to obtain quinazoline derivatives of the invention in an alternative and in some occasions, more convenient manner, the individual process steps mentioned hereinbefore may be performed in different order, and/or the individual reactions may be performed at different stage in the overall route (i.e. chemical transformations may be performed upon different intermediates to those associated hereinbefore with a particular reaction).

Certain intermediates used in the processes described above are novel and form a further feature of the present invention. Accordingly there is provided a compound selected from a compound the Formulae **II, IV, VI, VII** and **XII** as hereinbefore defined, or a salt thereof. The intermediate may be in the form of a salt of the intermediate. Such salts need not be a pharmaceutically acceptable salt. For example it may be useful to prepare an intermediate in the form of a pharmaceutically non-acceptable salt if, for example, such salts are useful in the manufacture of a quinazoline derivative of the Formula **I.**

### Biological Assays

The inhibitory activities of compounds were assessed in non-cell based protein tyrosine kinase assays as well as in cell based proliferation assays before their *in vivo* activity was assessed in Xenograft studies.

### a) Protein Tyrosine Kinase phosphorylation Assays

This test measures the ability of a test compound to inhibit the phosphorylation of a tyrosine containing polypeptide substrate by EGFR , erbB2 and erbB4 tyrosine kinase enzyme.

Recombinant intracellular fragments of EGFR, erbB2 and erbB4 (accession numbers X00588, X03363 and L07868 respectively) were cloned and expressed in the baculovirus/Sf21 system. Lysates were prepared from these cells by treatment with ice-cold lysis buffer (20mM *N*-2-hydroxyethylpiperizine-N'-2-ethanesulfonic acid (HEPES) pH7.5, 150mM NaCl, 10% glycerol, 1% Triton X-100, 1.5mM MgCl₂, 1mM ethylene glycol-bis(β-aminoethyl ether) N',N',N',N'-tetraacetic acid (EGTA), plus protease inhibitors and then cleared by centrifugation.

Constitutive kinase activity of these recombinant proteins was determined by their ability to phosphorylate a synthetic peptide (made up of a random co-polymer of Glutamic Acid, Alanine and Tyrosine in the ratio of 6:3:1). Specifically, Maxisorb^{™} 96-well immunoplates were coated with synthetic peptide (0.2µg of peptide in a 100µl phosphate buffered saline (PBS) solution and incubated at 4°C overnight). Plates were washed in 50mM HEPES pH 7.4 at room temperature to remove any excess unbound synthetic peptide. EGFR or erbB2 activities were assessed by incubation in peptide coated plates for 20 minutes at room temperature in 50mM HEPES pH 7.4 at room temperature, adenosine trisphosphate (ATP) at Km concentration for the respective enzyme, 10mM MnCl₂, 0.05mM Na₃VO₄, 0.1mM DL-dithiothreitol (DTT), 0.05% Triton X-100 with test compound in DMSO (final concentration of 2.5%). Reactions were terminated by the removal of the liquid components of the assay followed by washing of the plates with PBS-T (phosphate buffered saline with 0.05% Tween 20).

The immobilised phospho-peptide product of the reaction was detected by immunological methods. Firstly, plates were incubated for 90 minutes at room temperature with anti-phosphotyrosine primary antibodies that were raised in the mouse (4G10 from Upstate Biotechnology). Following extensive washing, plates were treated with Horseradish Peroxidase (HRP) conjugated sheep anti-mouse secondary antibody (NXA931 from Amersham) for 60 minutes at room temperature. After further washing, HRP activity in each well of the plate was measured colorimetrically using 22'-Azino-di-[3-ethylbenzthiazoline sulfonate (6)] diammonium salt crystals (ABTS™ from Roche) as a substrate.

Quantification of colour development and thus enzyme activity was achieved by the measurement of absorbance at 405nm on a Molecular Devices ThermoMax microplate reader. Kinase inhibition for a given compound was expressed as an IC₅₀ value. This was determined by calculation of the concentration of compound that was required to give 50% inhibition of phosphorylation in this assay. The range of phosphorylation was calculated from the positive (vehicle plus ATP) and negative (vehicle minus ATP) control values.

### b) EGFR driven KB cell proliferation assay

This assay measures the ability of a test compound to inhibit the proliferation of human tumour cell line, KB (obtained from the American Type Culture Collection (ATCC)).

KB cells were cultured in Dulbecco's modified Eagle's medium (DMEM) containing 10% foetal calf serum, 2 mM glutamine and non-essential amino acids at 37°C in a 7.5% CO₂ air incubator. Cells were harvested from the stock flasks using Trypsin / ethylaminediaminetetraacetic acid (EDTA). Cell density was measured using a haemocytometer and viability was calculated using trypan blue solution before being seeded at a density of 1.25x10³ cells per well of a 96 well plate in DMEM containing 2.5% charcoal stripped serum, 1mM glutamine and non-essential amino acids at 37°C in 7.5% CO₂ and allowed to settle for 4 hours.

Following adhesion to the plate, the cells are treated with or without EGF (final concentration of 1ng/ml) and with or without compound at a range of concentrations in dimethylsulfoxide (DMSO) (0.1% final) before incubation for 4 days. Following the incubation period, cell numbers were determined by addition of 50µl of 3-(4,5-Dimethylthiazol-2-yl)-2,5-diphenyltetrazolium bromide (MTT) (stock 5mg/ml) for 2 hours. MTT solution was then tipped off, the plate gently tapped dry and the cells dissolved upon the addition of 100µl of DMSO.

Absorbance of the solubilised cells was read at 540nm using a Molecular Devices ThermoMax microplate reader. Inhibition of proliferation was expressed as an IC₅₀ value. This was determined by calculation of the concentration of compound that was required to give 50% inhibition of proliferation. The range of proliferation was calculated from the positive (vehicle plus EGF) and negative (vehicle minus EGF) control values.

### c) Clone 24 phospho-erbB2 cell assay

This immunofluorescence end point assay measures the ability of a test compound to inhibit the phosphorylation of erbB2 in a MCF7 (breast carcinoma) derived cell line which was generated by transfecting MCF7 cells with the full length erbB2 gene using standard methods to give a cell line that overexpresses full length wild type erbB2 protein (hereinafter 'Clone 24' cells).

Clone 24 cells were cultured in Growth Medium (phenol red free Dulbecco's modified Eagle's medium (DMEM) containing 10% foetal bovine serum, 2 mM glutamine and 1.2mg/ml G418) in a 7.5% CO₂ air incubator at 37°C. Cells were harvested from T75 stock flasks by washing once in PBS (phosphate buffered saline, pH7.4, Gibco No. 10010-015) and harvested using 2mls of Trypsin (1.25mg/ml) / ethylaminediaminetetraacetic acid (EDTA) (0.8mg/ml) solution. The cells were resuspended in Growth Medium. Cell density was measured using a haemocytometer and viability was calculated using Trypan Blue solution before being further diluted in Growth Medium and seeded at a density of 1x10⁴ cells per well (in 100µl) into clear bottomed 96 well plates (Packard, No. 6005182).

3 days later, Growth Medium was removed from the wells and replaced with 100µl Assay Medium (phenol red free DMEM, 2mM glutamine, 1.2mg/ml G418) either with or without erbB inhibitor compound. Plates were returned to the incubator for 4 hours and then 20µl of 20% formaldehyde solution in PBS was added to each well and the plate was left at room temperature for 30 minutes. This fixative solution was removed with a multichannel pipette, 100µl of PBS was added to each well and then removed with a multichannel pipette and then 50µl PBS was added to each well. Plates were then sealed and stored for up to 2 weeks at 4°C.

Immunostaining was performed at room temperature. Cells were washed once with 200µl PBS / Tween 20 (made by adding 1 sachet of PBS / Tween dry powder (Sigma, No. P3563) to 1L of double distilled H₂O) using a plate washer, then 100µl of 0.5% Triton X-100 / PBS was added to each well to permeabalise the cells. After 10 minutes, the plates were washed with 200µl PBS / Tween 20 and then 100µl Blocking Solution (5% Marvel dried skimmed milk (Nestle) in PBS) was added per well and the plates were incubated for 15 minutes. Following removal of the Blocking Solution with a plate washer, 30µl of rabbit polyclonal anti-phospho erbB2 IgG antibody (epitope phospho-Tyr 1248, SantaCruz, No. SC-12352-R), diluted 1:250 in Blocking Solution, was added to each well and incubated for 2 hours. Then this primary antibody solution was removed from the wells using a plate washer followed by two 200µl PBS / Tween 20 washes using a plate washer. 100µl of Blocking solution was added per well and the plates were incubated for 10 minutes. Then 30µl of Alexa-Fluor 488 goat anti-rabbit IgG secondary antibody (Molecular Probes, No. A-11008), diluted 1:750 in Blocking Solution, was added to each well. From now onwards, wherever possible, plates were protected from light exposure, at this stage by sealing with black backing tape. The plates were incubated for 45 minutes and then the secondary antibody solution was removed from the wells followed by three 200µl PBS / Tween 20 washes using a plate washer. Then 100µl PBS was added to each plate, incubated for 10 minutes and then removed using a plate washer. Then 50µl of PBS was added to each well and plates were resealed with black backing tape and stored at 4°C before analysis. Plates were analysed within six hours of completing the immunostaining.

The Fluorescence signal in each well was measured using an Acumen Explorer Instrument (Acumen Bioscience Ltd.), a plate reader that can be used to rapidly quantitate features of images generated by laser-scanning. The instrument was set to measure the number of fluorescent objects above a pre-set threshold value and this provided a measure of the phosphorylation status of erbB2 protein. Fluorescence dose response data obtained with each compound was exported into a suitable software package (such as Origin) to perform curve fitting analysis. Inhibition of erbB2 phosphorylation was expressed as an IC₅₀ value. This was determined by calculation of the concentration of compound that was required to give 50% inhibition of erbB2 phosphorylation signal.

### d) In vivo BT474C Xenograft assay

This assay measures the ability of a test compound to inhibit the growth of a specific variant of the BT-474 tumour cell line grown as a xenograft in Female Swiss athymic mice (Alderley Park, *nu*/*nu* genotype) (Baselga, J. et al. (1998) Cancer Research, 58, 2825-2831).

The BT-474 tumour cell line (human mammary carcinoma) was obtained from Dr Baselga (at Laboratorio Recerca Oncologica, Paseo Vall D'Hebron 119-129, Barcelona 08035, Spain). This cell line was subcloned and a certain population (hereinafter referred to as "BT474C") was obtained.

Female Swiss athymic (*nu*/*nu* genotype) mice were bred and maintained in Alderley Park in negative pressure Isolators (PFI Systems Ltd.). Mice were housed in a barrier facility with 12 hour light/dark cycles and provided with sterilised food and water *ad libitum.* All procedures were performed on mice of at least 8 weeks of age. BT474C tumour cell xenografts were established in the hind flank of donor mice by sub-cutaneous injections of 1 x 10⁷ freshly cultured cells in 100µl of serum free media with 50% Matrigel per animal. Animals were supplemented with oestradiol benzoate (Mesalin, Intravet UK 0.2 mg/ml), 100µg/animal injected subcutaneously on the day before cell implant, with subsequent weekly boosts of 50µg/animal; or by implantation of 0.5 mg 21 day release oestrogen pellets (Innovative Research of America) on the day before cell implant. As an example, selection on day 14 post-implant, mice were randomised into groups of 10 prior to the treatment with compound or vehicle control that was administered once daily at 0.1ml/10g body weight. Tumour volume was assessed twice weekly by bilateral Vernier calliper measurement, using the formula (length x width) x √(length x width) x (π/6), where length was the longest diameter across the tumour, and width was the corresponding perpendicular. Growth inhibition from start of treatment was calculated by comparison of the mean changes in tumour volume for the control and treated groups, and statistical significance between the two groups was evaluated using a Students t test.

### e) BT474C Cell Proliferation Assay

BT474C cells are a sub-cloned population of *in vivo* competent cells, as discussed above.

The BT474C assay is a MTS (3-(4,5-dimethylthiazol-2-yl)-5-(3-carboxymethoxyphenyl)-2-(4-sulfophenyl)-2H- tetrazolium, inner salt - Promega G1111) endpoint-based cell proliferation assay, which measures the ability of a test compound to inhibit the proliferation of cells over a four-day period. Cells are grown to logarithmic phase in growth media (phenol red free Dulbecco's modified Eagle's medium (DMEM) containing 10% foetal bovine serum, 10% M1 supplement (AstraZeneca internal supply), 1% oxaloacetic acid in a 7.5% CO₂ air incubator at 37°C. Cells are harvested from stock flasks by washing once in PBS (phosphate buffered saline, pH7.4, Gibco No. 10010-015) and removed using 2mls of Trypsin (1.25mg/ml) / ethylaminediaminetetraacetic acid (EDTA) (0.8mg/ml) solution. The cells are re-suspended in assay media (phenol red free Dulbecco's modified Eagle's medium (DMEM) containing 10% charcoal/Dextran stripped foetal bovine serum, 10% M1 supplement, 1% oxaloacetic acid. Cell density is measured using a haemocytometer and viability is calculated using Trypan Blue solution before being further diluted in Assay Medium and seeded at a density of 1x10⁴ cells per well (in 100µl) into clear bottomed 96 well plates (Costar 3598). One extra plate is set up to act as a Day 0 control plate.
4 hours later, assay medium containing test compound, serially diluted in 100% DMSO (Sigma D5879), in the form of a dose response is added across the plate in triplicate. The Day 0 plate is treated with MTS solution (Tetrazolium compound - made from MTS powder in a Phenazine ethosulfate (PES - Sigma P4544)/PBS) and incubated for 2 hours before the reaction is stopped by the addition of 10% SDS. The plate is read at 490nm on a spectrophotometer.

Assay plates are left at 37°C for 4 days and then treated with MTS solution (as above), which is converted to a soluble formazan product by active cells. After incubating the plates for 2 hours the reaction is stopped by the addition of 10% SDS (Sodium dodecyl sulphate) and the plates are read at 490nm on a spectrophotometer giving absorbance values relative to the concentration of converted dye.

Absorbance dose response data obtained with each compound is exported into a suitable software package (such as Origin) to perform curve-fitting analysis. Inhibition of BT474C cell proliferation is expressed as an IC₅₀ value (calculated as GI50 by use of a log/lin plot - analyzing data above the day 0 absorbance values). This is determined by calculation of the concentration of compound that is required to give 50% inhibition of cell proliferation.

### f) hERG-encoded Potassium Channel Inhibition Assay

### Cell culture for IonWorks™ HT:

The hERG-expressing Chinese hamster ovary K1 (CHO) cells described by Persson *et al*. (Persson, F., Carlsson, L., Duker, G., and Jacobson, I., Blocking characteristics of hERG, hNav1.5, and hKvLQT1/hminK after administration of the novel anti-arrhythmic compound AZD7009., J Cardiovasc.Electrophysiol.,16, 329-341.2005) were grown to semi-confluence at 37°C in a humidified environment (5% CO₂ in F-12 Ham medium containing L-glutamine, 10% foetal calf serum (FCS) and 0.6 mg/ml hygromycin (all Sigma). Prior to use, the monolayer was washed using a pre-warmed (37°C) 3ml aliquot of Versene 1:5,000 (Invitrogen). After aspiration of this solution the flask was incubated at 37°C in an incubator with a further 2 ml of Versene 1:5,000 for a period of 6 minutes. Cells were then detached from the bottom of the flask by gentle tapping and 10 ml of Dulbecco's-PBS containing calcium (0.9 mM) and magnesium (0.5 mM) (PBS; Invitrogen) was then added to the flask and aspirated into a 15 ml centrifuge tube prior to centrifugation (50 g, for 4 minutes). The resulting supernatant was discarded and the pellet gently re-suspended in 3 ml of PBS. A 0.5 ml aliquot of cell suspension was removed to determine viable cell number based on trypan blue exclusion (Cedex; Innovatis) and the cell re-suspension volume adjusted with PBS to give the desired final cell concentration. CHO-Kv1.5 cells, which were used to adjust the voltage offset on IonWorks™ HT, were maintained and prepared for use in the same way.

### IonWorks™ HT electrophysiology:

The principles and operation of this device have been described by Schroeder *et al.* (Schroeder, K., Neagle, B., Trezise, D. J., and Worley, J., Ionworks HT: a new high-throughput electrophysiology measurement platform, J Biomol Screen, 8, 50-64, 2003). Briefly, the technology is based on a 384-well plate (PatchPlate^{™}) in which a recording is attempted in each well by using suction to position and hold a cell on a small hole separating two isolated fluid chambers. Once sealing has taken place, the solution on the underside of the PatchPlate^{™} is changed to one containing amphotericin B. This permeablises the patch of cell membrane covering the hole in each well and in effect allows a perforated, whole-cell patch clamp recording to be made.

IonWorks™ HT (a beta-test machine from Essen Instruments) was operated at room temperature (~21°C) in the following way. The reservoir in the "Buffer" position was loaded with 4 ml of PBS and that in the "Cells" position with the CHO-hERG cell suspension described above. A 96-well plate (V-bottom, Greiner Bio-one) containing the compounds to be tested (at 3X their final test concentration) was placed in the "Plate 1" position and a PatchPlate™ was clamped into the PatchPlate™ station. Each compound plate was laid-out in 12 columns to enable ten, 8-point concentration-effect curves to be constructed; the remaining two columns on the plate were taken up with vehicle (final concentration 0.33% DMSO), to define the assay baseline, and a supra-maximal blocking concentration of cisapride (final concentration 10 µM), to define the 100% inhibition level. The fluidics-head (F-Head) of IonWorks™ HT then added 3.5 µl of PBS to each well of the PatchPlate™ and its underside was perfused with "internal" solution that had the following composition (in mM): K-Gluconate 100, KC140, MgCl₂ 3.2, EGTA 3 and HEPES 5 (all Sigma) (pH 7.25-7.30 using 10 M KOH). After priming and de-bubbling, the electronics-head (E-head) then moved round the PatchPlate™ performing a hole test (i.e. applying a voltage pulse to determine whether the hole in each well was open). The F-head then dispensed 3.5 µl of the cell suspension described above into each well of the PatchPlate™ and the cells were given 200 seconds to reach and seal to the hole in each well. Following this, the E-head moved round the PatchPlate™ to determine the seal resistance obtained in each well. Next, the solution on the underside of the PatchPlate™ was changed to "access" solution that had the following composition (in mM): KCl 140, EGTA 1, MgCl₂ 1 and HEPES 20 (pH 7.25-7.30 using 10 M KOH) plus 100 µg/ml of amphotericin B (all Sigma). After allowing 9 minutes for patch perforation to take place, the E-head moved round the PatchPlate™ 48 wells at a time to obtain pre-compound HERG current measurements. The F-head then added 3.5 µl of solution from each well of the compound plate to 4 wells on the PatchPlate™ (the final DMSO concentration was 0.33% in every well). This was achieved by moving from the most dilute to the most concentrated well of the compound plate to minimise the impact of any compound carry-over. After approximately three and a half minutes incubation, the E-head then moved around all 384-wells of the PatchPlate™ to obtain post-compound hERG current measurements. In this way, non-cumulative concentration-effect curves could be produced where, providing the acceptance criteria were achieved in a sufficient percentage of wells (see below), the effect of each concentration of test compound was based on recording from between 1 and 4 cells.

The pre- and post-compound hERG current was evoked by a single voltage pulse consisting of a 20 s period holding at -70 mV, a 160 ms step to -60 mV (to obtain an estimate of leak), a 100 ms step back to -70 mV, a 1 s step to +40 mV, a 2 s step to -30 mV and finally a 500 ms step to -70mV. In between the pre- and post-compound voltage pulses there was no clamping of the membrane potential. Currents were leak-subtracted based on the estimate of current evoked during the +10mV step at the start of the voltage pulse protocol. The current signal was sampled at 2.5k Hz.

Pre- and post-scan hERG current magnitude was measured automatically from the leak subtracted traces by the IonWorks™ HT software by taking a 40ms average of the current during the initial holding period at -70mV (baseline current) and subtracting this from the peak of the tail current response. The acceptance criteria for the currents evoked in each well were: pre-scan seal resistance >60 MΩ, pre-scan hERG tail current amplitude >150 pA; post-scan seal resistance >60 MΩ. The degree of inhibition of the hERG current was assessed by dividing the post-scan hERG current by the respective pre-scan hERG current for each well.

Although the pharmacological properties of the quinazoline derivatives of the Formula **I** vary with structural change as expected, in general activity possessed by the quinazoline derivatives of the Formula **I**, may be demonstrated at the following concentrations or doses in one or more of the above tests (a), (b), (c), (d) and (e):-
- Test (a):-: IC₅₀ in the range, for example, 0.001 - 1 µM;
- Test (b):-: IC₅₀ in the range, for example, 0.001 - 5 µM;
- Test (c):-: IC₅₀ in the range, for example, 0.001 - 5 µM;
- Test (d):-: activity in the range, for example, 1-200 mg/kg/day;
- Test (e):-: IC₅₀ in the range, for example, 0.001 - 5 µM;

No physiologically unacceptable toxicity was observed in Test (d) at the effective dose for quinazoline derivatives tested of the present invention. Test (f) shows a safe margin between target and hERG activity, suggesting the unlikelihood of arrhythmia caused by inhibition of the hERG channel. Accordingly no untoward toxicological effects are expected when a quinazoline derivative of the Formula **I**, or a pharmaceutically acceptable salt thereof, as defined hereinbefore is administered at the dosage ranges defined hereinafter.

By way of example, Table A illustrates the activity of representative compounds according to the invention. Column 2 of Table A shows IC₅₀ data from Test (a) for the inhibition of EGFR tyrosine kinase protein phosphorylation; column 3 shows IC₅₀ data from Test (a) for the inhibition of erbB2 tyrosine kinase protein phosphorylation; and column 4 shows IC₅₀ data for inhibition of proliferation of BT474C cells in Test (e) described above:

**Table A**

| **Example Number** | **IC₅₀ (µM) Test (a): Inhibition of EGFR tyrosine kinase protein phosphorylation** | **IC₅₀ (µM) Test (a): Inhibition of erbB2 tyrosine kinase protein phosphorylation** | **IC₅₀ (µM) Test (e): Inhibition of proliferation of BT474C cells** |
|---|---|---|---|
| 1 | 0.333 | 0.015 | 0.179 |
| 2 | 0.869 | 0.022 | 0.857 |

According to a further aspect of the invention there is provided a pharmaceutical composition which comprises a quinazoline derivative of the Formula **I**, or a pharmaceutically acceptable thereof, as defined hereinbefore in association with a pharmaceutically acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a quinazoline derivative of the Formula **I** will naturally vary according to the nature and severity of the conditions, the age and sex of the animal or patient and the route of administration, according to well known principles of medicine.

In using a quinazoline derivative of the Formula **I** for therapeutic or prophylactic purposes it will generally be administered so that a daily dose in the range, for example, 0.1 mg/kg to 75 mg/kg body weight is received, given if required in divided doses. In general lower doses will be administered when a parenteral route is employed. Thus, for example, for intravenous administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration is however preferred, particularly in tablet form. Typically, unit dosage forms will contain about 0.5 mg to 0.5 g of a quinazoline derivative of this invention.

We have found that the quinazoline derivatives of the present invention possess anti-proliferative properties such as anti-cancer properties that are believed to arise from their erbB, particularly EGF and more particularly erbB2 receptor tyrosine kinase inhibitory activity. Furthermore, certain of the quinazoline derivatives according to the present invention possess substantially better potency against the erbB2 receptor tyrosine kinase, than against other tyrosine kinases enzymes, such as EGFR tyrosine kinase. Such quinazoline derivatives possess sufficient potency against the erbB2 receptor tyrosine kinase that they may be used in an amount sufficient to inhibit erbB2 receptor tyrosine kinase whilst demonstrating little, or significantly lower, activity against other tyrosine kinases such as EGFR. Such quinazoline derivatives are likely to be useful for the selective inhibition of erbB2 receptor tyrosine kinase and are likely to be useful for the effective treatment of, for example, erbB2 driven tumours.

Accordingly, the quinazoline derivatives of the present invention are expected to be useful in the treatment of diseases or medical conditions mediated alone or in part by and erbB, particularly erbB2 receptor tyrosine kinases, i.e. the quinazoline derivatives may be used to produce an erbB, particularly an erbB2, receptor tyrosine kinase inhibitory effect in a warm-blooded animal in need of such treatment. Thus the quinazoline derivatives of the present invention provide a method for the treatment of malignant cells characterised by inhibition of the erbB, particularly the erbB2, receptor tyrosine kinase. Particularly the quinazoline derivatives of the invention may be used to produce an anti-proliferative and/or pro-apoptotic and/or anti-invasive effect mediated alone or in part by the inhibition of erbB, particularly erbB2, receptor tyrosine kinases. Particularly, the quinazoline derivatives of the present invention are expected to be useful in the prevention or treatment of those tumours that are sensitive to inhibition of an erbB, particularly the erbB2, receptor tyrosine kinase that are involved in the signal transduction steps which drive proliferation and survival of these tumour cells. Accordingly the quinazoline derivatives of the present invention are expected to be useful in the treatment and/or prevention of a number of hyperproliferative disorders by providing an anti-proliferative effect. These disorders include, for example psoriasis, benign prostatic hyperplasia (BPH), atherosclerosis and restenosis and, in particular, erbB, more particularly erbB2, receptor tyrosine kinase driven tumours. Such benign or malignant tumours may affect any tissue and include non-solid tumours such as leukaemia, multiple myeloma or lymphoma, and also solid tumours, for example bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval tumours.

According to this aspect of the invention there is provided a quinazoline derivative of the Formula **I**, or a pharmaceutically acceptable salt thereof, for use as a medicament.

Thus according to this aspect of the invention there is provided the use of a quinazoline derivative of the Formula **I**, or a pharmaceutically acceptable salt thereof, as defined hereinbefore in the manufacture of a medicament for use in the production of an anti-proliferative effect in a warm-blooded animal such as man.

A method for producing an anti-proliferative effect in a warm-blooded animal, such as man, in need of such treatment may comprise administering to said animal an effective amount of a quinazoline derivative of the Formula **I**, or a pharmaceutically acceptable salt thereof, as hereinbefore defined.

According to a further aspect of the invention there is provided a quinazoline derivative of the Formula **I**, or a pharmaceutically acceptable salt thereof, for use in the production of an anti-proliferative effect in a warm-blooded animal such as man.

According to a further aspect of the invention there is provided the use of a quinazoline derivative of the Formula **I**, or a pharmaceutically acceptable salt thereof, as defined hereinbefore in the manufacture of a medicament for use in the production of an anti-proliferative effect which effect is produced alone or in part by inhibiting erbB2 receptor tyrosine kinase in a warm-blooded animal such as may.

A method for producing an anti-proliferative effect which effect is produced alone or in part by inhibiting erbB2 receptor tyrosine kinase in a warm-blooded animal, such as man, in need of such treatment may comprise administering to said animal an effective amount of a quinazoline derivative of the Formula **I**, or a pharmaceutically acceptable salt thereof, as hereinbefore defined.

According to a further aspect of the invention there is provided a quinazoline derivative of the Formula **I**, or a pharmaceutically acceptable salt thereof, for use in the production of an anti-proliferative effect which effect is produced alone or in part by inhibiting erbB2 receptor tyrosine kinase in a warm-blooded animal such as man.

According to a further aspect of the present invention there is provided the use of a quinazoline derivative of the Formula **I**, or a pharmaceutically acceptable salt thereof, as defined hereinbefore in the manufacture of a medicament for use in the treatments of a disease or medical condition (for example a cancer as mentioned herein) mediated alone or in part by erbB, particularly erbB2, receptor tyrosine kinase.

A method for treating a disease or medical condition (for example a cancer as mentioned herein) mediated alone or in part by erbB, particularly erbB2, receptor tyrosine kinase in a warm-blooded animal, such as man, in need of such treatment, may comprise administering to said animal an effective amount of a quinazoline derivative of the Formula I**,** or a pharmaceutically acceptable salt thereof, as defined hereinbefore.

According to a further aspect of the invention there is provided a quinazoline derivative of the Formula **I**, or a pharmaceutically acceptable salt thereof, for use in the treatment of a disease or medical condition (for example a cancer as mentioned herein) mediated alone or in part by erbB, particularly erbB2, receptor tyrosine kinase.

According to a further aspect of the invention there is provided the use of a quinazoline derivative of the Formula **I**, or a pharmaceutically acceptable salt thereof, as defined hereinbefore in the manufacture of a medicament for use in the prevention or treatment of those tumours which are sensitive to inhibition of one or more erbB receptor tyrosine kinases, such as BGF and/or erbB2 and/or erbB4 (especially erbB2) receptor tyrosine kinase that are involved in the signal transduction steps which lead to the proliferation of tumour cells.

A method for the prevention or treatment of those tumours which are sensitive to inhibition of one or more erbB receptor tyrosine kinases, such as EGF and/or erbB2 and/or erbB4 (especially erbB2) receptor tyrosine kinases, that are involved in the signal transduction steps which lead to the proliferation and/or survival of tumour cells in a warm-blooded animal, such as man, in need of such treatment, may comprise administering to said animal an effective amount of a quinazoline derivative of the Formula **I**, or a pharmaceutically acceptable salt thereof, as defined hereinbefore.

According to a further aspect of the invention there is provided a quinazoline derivative of the Formula **I**, or a pharmaceutically acceptable salt thereof, for use in the prevention or treatment of those tumours which are sensitive to inhibition of one or more erbB receptor tyrosine kinases, such as EGF and/or erbB2 and/or erbB4 (especially erbB2) receptor tyrosine kinase, that are involved in the signal transduction steps which lead to the proliferation and/or survival of tumour cells.

According to a further aspect of the invention there is provided the use of a quinazoline derivative of the Formula **I**, or a pharmaceutically acceptable salt thereof, as defined hereinbefore in the manufacture of a medicament for use in providing an EGF and/or erbB2 and/or erbB4 (especially erbB2) receptor tyrosine kinase inhibitory effect.

A method for providing an EGF and/or erbB2 and/or erbB4 (especially erbB2) receptor tyrosine kinase inhibitory effect in a warm-blooded animal, such as man, in need of such treatment, may comprise administering to said animal an effective amount of a quinazoline derivative of the Formula **I**, or a pharmaceutically acceptable salt thereof, as defined hereinbefore.

According to a further aspect of the invention there is provided a quinazoline derivative of the Formula **I**, or a pharmaceutically acceptable salt thereof, for use in providing an EGF and/or erbB2 and/or erbB4 (especially erbB2) receptor tyrosine kinase inhibitory effect.

According to a further aspect of the invention there is provided the use of a quinazoline derivative of the Formula **I**, or a pharmaceutically acceptable salt thereof, as defined hereinbefore in the manufacture of a medicament for use in providing a selective erbB2 kinase inhibitory effect.

A method for providing a selective erbB2 kinase inhibitory effect in a warm-blooded animal, such as man, in need of such treatment, may comprise administering to said animal an effective amount of a quinazoline derivative of the Formula **I**, or a pharmaceutically acceptable salt thereof, as defined hereinbefore.

According to a further aspect of the invention there is provided a quinazoline derivative of the Formula **I**, or a pharmaceutically acceptable salt thereof, for use in providing a selective erbB2 kinase inhibitory effect.

By "a selective erbB2 kinase inhibitory effect" is meant that the quinazoline derivative of the Formula **I** is more potent against erbB2 receptor tyrosine kinase than it is against other kinases. In particular some of the compounds according to the invention are more potent against erbB2 receptor kinase than it is against other tyrosine kinases such as other erbB receptor tyrosine kinases, particularly EGFR tyrosine kinase. For example a selective erbB2 kinase inhibitor according to the invention is at least 5 times, preferably at least 10 times more portent against erbB2 receptor tyrosine kinase than it is against EGFR tyrosine kinase, as determined from the relative IC₅₀ values in suitable assays (for example the by comparing the IC₅₀ value from the Clone 24 phospho-erbB2 cell assay (a measure of the erbB2 tyrosine kinase inhibitory activity in cells) with the IC₅₀ from the KB cell assay (a measure of the EGFR tyrosine kinases inhibitory activity in cells) for a given test compound as described above).

According to a further aspect of the present invention there is provided the use of a quinazoline derivative of the Formula **I**, or a pharmaceutically acceptable salt thereof, as defined hereinbefore in the manufacture of a medicament for use in the treatments of a cancer, for example a cancer selected from leukaemia, multiple myeloma, lymphoma, bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval cancer.

A method for treating a cancer, for example a cancer selected from selected from leukaemia, multiple myeloma, lymphoma, bile duct, bone, bladder, brain/CNS, breast, colorectal, cervical, endometrial, gastric, head and neck, hepatic, lung, muscle, neuronal, oesophageal, ovarian, pancreatic, pleural/peritoneal membrane, prostate, renal, skin, testicular, thyroid, uterine and vulval cancer in a warm-blooded animal, such as man, in need of such treatment, may comprise administering to said animal an effective amount of a quinazoline derivative of the Formula **I**, or a pharmaceutically acceptable salt thereof, as defined hereinbefore.

According to a further aspect of the invention there is provided a quinazoline derivative of the Formula **I,** or a pharmaceutically acceptable salt thereof, for use in the treatment of a cancer, for example a cancer selected from leukaemia, multiple myeloma, lymphoma, bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval cancer.

As mentioned above the size of the dose required for the therapeutic or prophlyactic treatment of a particular disease will necessarily be varied depending upon, amongst other things, the host treated, the route of administration and the severity of the illness being treated.

The quinazoline derivatives of the invention may be administered in the form of a pro-drug, by which we mean a compound that is broken down in a warm-blooded animal, such as man, to release a quinazoline derivative of the invention. A pro-drug may be used to alter the physical properties and/or the pharmacokinetic properties of a quinazoline derivative of the invention. A pro-drug can be formed when the quinazoline derivative of the invention contains a suitable group or substituent to which a property-modifying group can be attached. Examples of pro-drugs include *in vivo* cleavable ester derivatives that may be formed at a hydroxy group in a quinazoline derivative of the Formula **I** and *in vivo* cleavable amide derivatives that may be formed at an amino group in a quinazoline derivative of the Formula **I**.

Accordingly, the present invention includes those quinazoline derivatives of the Formula **I** as defined hereinbefore when made available by organic synthesis and when made available within the human or animal body by way of cleavage of a pro-drug thereof. Accordingly, the present invention includes those quinazoline derivatives of the Formula **I** that are produced by organic synthetic means and also such quinazoline derivatives that are produced in the human or animal body by way of metabolism of a precursor compound, that is a quinazoline derivative of the Formula **I** may be a synthetically-produced quinazoline derivative or a metabolically-produced quinazoline derivative.

A suitable pharmaceutically acceptable pro-drug of a quinazoline derivative of the Formula **I** is one that is based on reasonable medical judgement as being suitable for administration to the human or animal body without undesirable pharmacological activities and without undue toxicity.

Various forms of pro-drug have been described, for example in the following documents :-
a) Methods in Enzymology, Vol. 42, p. 309 to 396, edited by K. Widder, et al. (Academic Press, 1985);
b) Design of Pro-drugs, edited by H. Bundgaard, (Elsevier, 1985);
c) A Textbook of Drug Design and Development, edited by Krogsgaard-Larsen and H. Bundgaard, Chapter 5 "Design and Application of Pro-drugs", edited by H. Bundgaard, p. 113 to 191 (1991);
d) H. Bundgaard, Advanced Drug Delivery Reviews, 8, 1 to 38 (1992); and
e) H. Bundgaard, et al., Journal of Pharmaceutical Sciences, 77, 285 (1988).

The anti-proliferative treatment defined hereinbefore may be applied as a sole therapy or may involve, in addition to the quinazoline derivative of the invention, conventional surgery or radiotherapy or chemotherapy. Such chemotherapy may include one or more of the following categories of anti-tumour agents:-
(i) other antiproliferative/antineoplastic drugs and combinations thereof, as used in medical oncology, such as alkylating agents (for example cis-platin, oxaliplatin, carboplatin, cyclophosphamide, nitrogen mustard, melphalan, chlorambucil, busulphan, temozolamide and nitrosoureas); antimetabolites (for example gemcitabine and antifolates such as fluoropyrimidines like 5-fluorouracil and tegafur, raltitrexed, methotrexate, cytosine arabinoside, and hydroxyurea); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere and polokinase inhibitors); and topoisomerase inhibitors (for example epipodophyllotoxins like etoposide and teniposide, amsacrine, topotecan and camptothecin);
(ii) cytostatic agents such as antioestrogens (for example tamoxifen, fulvestrant, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) anti-invasion agents (for example c-Src kinase family inhibitors like 4-(6-chloro-2,3-methylenedioxyanilino)-7-[2-(4-methylpiperazin-1-yl)ethoxy]-5-tetrahydropyran-4-yloxyquinazoline (AZD0530; International Patent Application WO 01/94341) and *N*-(2-chloro-6-methylphenyl)-2-{6-[4-(2-hydroxyethyl)piperazin-1-yl]-2-methylpyrimidin-4-ylamino}thiazole-5-carboxamide (dasatinib, BMS-354825; J. Med. Chem., 2004, 47, 6658-6661), and metalloproteinase inhibitors like marimastat, inhibitors of urokinase plasminogen activator receptor function or antibodies to Heparanase);
(iv) inhibitors of growth factor function: for example such inhibitors include growth factor antibodies and growth factor receptor antibodies (for example the anti-erbB2 antibody trastuzumab [Herceptin™] and the anti-erbB1 antibody cetuximab [Erbitux, C225]); such inhibitors also include tyrosine kinase inhibitors, for example inhibitors of the epidermal growth factor family (for example EGFR family tyrosine kinase inhibitors such as *N*-(3-chloro-4-fluorophenyl)-7-methoxy-6-(3-morpholinopropoxy)quinazolin-4-amine (gefitinib, ZD1839), *N*-(3-ethynylphenyl)-6,7-bis(2-methoxyethoxy)quinazolin-4-amine (erlotinib, OSI-774) and 6-acrylamido-*N*-(3-chloro-4-fluorophenyl)-7-(3-morpholinopropoxy)-quinazolin-4-amine (Cl 1033), erbB2 tyrosine kinase inhibitors such as lapatinib, inhibitors of the hepatocyte growth factor family, inhibitors of the platelet-derived growth factor family such as imatinib, inhibitors of serine/threonine kinases (for example Ras/Raf signalling inhibitors such as farnesyl transferase inhibitors, for example sorafenib (BAY 43-9006)), inhibitors of cell signalling through MEK and/or AKT kinases; inhibitors of the hepatocyte growth factor family, c-kit inhibitors, abl kinase inhibitors, IGF receptor (insulin-like growth factor) kinase inhibitors; aurora kinase inhibitors (for example AZD1152, PH739358, VX-680, MLN8054, R763, MP235, MP529, VX-528 AND AX39459) and cyclin dependent kinase inhibitors such as CDK2 and/or CDK4 inhibitors;
(v) antiangiogenic agents such as those which inhibit the effects of vascular endothelial growth factor, [for example the anti-vascular endothelial cell growth factor antibody bevacizumab (Avastin™) and VEGF receptor tyrosine kinase inhibitors such as 4-(4-bromo-2-fluoroanilino)-6-methoxy-7-(1-methylpiperidin-4-ylmethoxy)quinazoline (ZD6474; Example 2 within WO 01/32651), 4-(4-fluoro-2-methylindol-5-yloxy)-6-methoxy-7-(3-pyrrolidin-1-ylpropoxy)quinazoline (AZD2171; Example 240 within WO 00/47212), vatalanib (PTK787; WO 98/35985) and SU11248 (sunitinib; WO 01/60814), compounds such as those disclosed in International Patent Applications WO97/22596, WO 97/30035, WO 97/32856 and WO 98/13354 and compounds that work by other mechanisms (for example linomide, inhibitors of integrin αvβ3 function and angiostatin)];
(vi) vascular damaging agents such as Combretastatin A4 and compounds disclosed in International Patent Applications WO 99/02166, WO 00/40529, WO 00/41669, WO 01/92224, WO 02/04434 and WO 02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Such conjoint treatment may be achieved by way of the simultaneous, sequential or separate dosing of the individual components of the treatment. Such combination products employ the quinazoline derivatives of this invention within the dosage range described hereinbefore and the other pharmaceutically-active agent within its approved dosage range.

According to this aspect of the invention there is provided a pharmaceutical product comprising a quinazoline derivative of the Formula **I** as defined hereinbefore and an additional anti-tumour agent as defined hereinbefore for the conjoint treatment of cancer.

Although the quinazoline derivatives of the Formula **I** are primarily of value as therapeutic agents for use in warm-blooded animals (including man), they are also useful whenever it is required to inhibit the effects of the erbB receptor tyrosine protein kinases. Thus, they are useful as pharmacological standards for use in the development of new biological tests and in the search for new pharmacological agents.

The invention will now be illustrated by the following examples in which, unless stated otherwise:
(i) temperatures are given in degrees Celsius (°C); operations were carried out at room or ambient temperature, that is, at a temperature in the range of 18 to 25°C;
(ii) organic solutions were dried over anhydrous magnesium sulfate or anhydrous sodium sulfate; evaporation of solvent was carried out using a rotary evaporator under reduced pressure (600 to 4000 Pascals; 4.5 to 30mmHg) with a bath temperature of up to 60°C;
(iii) chromatography means flash chromatography on silica gel; thin layer chromatography (TLC) was carried out on silica gel plates;
(iv) in general, the course of reactions was followed by TLC and / or analytical LC-MS, and reaction times are given for illustration only;
(v) final products had satisfactory proton nuclear magnetic resonance (NMR) spectra and/or mass spectral data;
(vi) yields are given for illustration only and are not necessarily those which can be obtained by diligent process development; preparations were repeated if more material was required;
(vii) when given, NMR data is in the form of delta values for major diagnostic protons, given in parts per million (ppm) relative to tetramethylsilane (TMS) as an internal standard, determined at 400 MHz using perdeuterio dimethyl sulfoxide (DMSO-d₆) as solvent unless otherwise indicated; the following abbreviations have been used: s, singlet; d, doublet; t, triplet; q, quartet; m, multiplet; b, broad;
(viii) chemical symbols have their usual meanings; SI units and symbols are used;
(ix) solvent ratios are given in volume:volume (v/v) terms; and
(x) mass spectra were run with an electron energy of 70 electron volts in the chemical ionization (CI) mode using a direct exposure probe; where indicated ionization was effected by electron impact (EI), fast atom bombardment (FAB) or electrospray (ESP); values for m/z are given; generally, only ions which indicate the parent mass are reported; and unless otherwise stated, the mass ion quoted is (MH)⁺ which refers to the protonated mass ion; reference to M⁺ is to the mass ion generated by loss of an electron; and reference to M-H⁺ is to the mass ion generated by loss of a proton;
(xi) unless stated otherwise compounds containing an asymmetrically substituted carbon and/or sulfur atom have not been resolved;
(xii) where a synthesis is described as being analogous to that described in a previous example the amounts used are the millimolar ratio equivalents to those used in the previous example;
(xiii) all microwave reactions were carried out in a CEM Discover™ microwave synthesiser;
(xiv) preparative high performance liquid chromatography (HPLC) was performed on a Gilson instrument using the following conditions:

| | |
|---|---|
| Column: | 21 mm x 10 cm Hichrom RPB |
| Solvent A: | Water + 0.1% trifluoroacetic acid, |
| Solvent B: | Acetonitrile + 0.1 % trifluoroacetic acid |
| Flow rate: | 18 ml / min |
| Run time: | 15 minutes with a 10 minute gradient from 5-95% B |
| Wavelength: | 254 nm, bandwidth 10 nm |
| Injection volume | 2.0-4.0 ml; |

xv) analytical HPLC was performed on a LC/MS Waters 2790 / ZMD Micromass system using the following conditions (so as to measure retention times (t_{R}):

| | |
|---|---|
| Waters Symmetry column: | C18, 3.5mM, 4.6 x 50 mm |
| Detection: | UV 254 nM and MS |
| Elution: | flow rate 2.5 ml/min, linear gradient from 95% water and 5% methanol containing 5% formic acid to 40% water, 55% acetonitrile and 5% methanol containing 5% formic acid over 3 minutes, then linear gradiant to 95% acetonitrile and 5% methanol containing 5% formic acid over 1 minute; |

(xvi) the following abbreviations have been used:
- HATU: O-(7-Azabenzotriazol-1-yl)-*N,N,N,N-*Tetramethyluronium Hexafluoro-Phosphate;
- DEAD: diethyl azodicarboxylate;
- DTAD: di-tert-butyl azodicarboxylate;
- EDCI: 1-(3-dimethylaminopropyl)-3-ethylcarbodiimide hydrochloride;
- THF: tetrahydrofuran;
- DMF: *N,N-*dimethylformamide;
- DMA: *N,N*-dimethylacetamide;
- DCM: dichloromethane;
- DMSO: dimethylsulfoxide;
- IPA: Isopropyl alcohol;
- Ether: diethyl ether; and
- TFA: trifluoroacetic acid.

### Example 1

### (2R)-2-[(4-{[4-(azepan-1-ylcarbonyl)-3-chlorophenyl]amino}quinazolin-5-yl)oxy]-N-(2-hydroxyethyl)-N-methylpropanamide

A mixture of 2-(methyl{(2*R*)-2-[(4-oxo-3,4-dihydroquinazolin-5-yl)oxy]propanoyl}amino)ethyl acetate (250 mg, 0.75 mmol), triphenylphosphine (590 mg, 2.25 mmol) and carbon tetrachloride (2.17 ml, 22.5 mmol) in 1,2-dichloroethane (5 ml) was stirred at 45°C for 2 hours. 4-(Azepan-1-ylcarbonyl)-3-chloroaniline (706 mg, 3.15 mmol) was added. The mixture was cooled and the solvents were evaporated under vacuum. Acetonitrile (15 ml) was added. The mixture was stirred at 75°C for 1 hour. After cooling, a solution of 7N methanolic ammonia was added. The mixture was evaporated under vacuum and the residue was purified by chromatography on silica gel (eluant: 1% to 2.5% methanol in DCM) to give 2-[{(2*R*)-2-[(4-{[4-(azepan-1-ylcarbonyl)-3-chlorophenyl]amino}quinazolin-5-yl)oxy]propanoyl}(methyl)amino]ethyl acetate as a white solid (296 mg). This compound was dissolved in pyrrolidine (1.56 ml) and the mixture was heated at 45°C for 1 hour. After evaporation of the solvents, the residue was injected on an HPLC column (C18, 5 micron, 19 mm diameter, 100 mm length) of a preparative HPLC-MS system with a mixture of water and acetonitrile containing 2g/l of ammonium carbonate (gradient). After evaporation of the solvents, the mixture was dissolved in dichloromethane and evaporated under vacuum to give the title compound as a white foam (185 mg, 47%); NMR Spectrum (CDCl₃) (2 rotamers) 1.9-1.5 (m, 11H), 3.04 and 3.23 (s, 3H), 3.33 (m, 2H), 4.0-3.5 (m, 6H), 5.71 and 5.37 (m, 1H), 6.87 and 6.75 (m, 1H), 7.6-7.2 (m, 3H), 7.95 (m, 1H), 8.35 (m, 1H), 8.65 and 8.56 (m, 1H); Mass spectrum MH⁺ 526.

The 2-(methyl{(2*R*)-2-[(4-oxo-3,4-dihydroquinazolin-5-yl)oxy]propanoyl}amino)ethyl acetate used as starting material was made as follows:

Sodium hydride (10 g, 60% dispersion in oil, 244 mmol) was added portion-wise to a solution of (R)-lactamide (8.14 g, 91.5 mmol) and 5-fluoroquinazolin-4(3H)-one (10 g, 61 mmol) in DMA (100 ml) at room temperature. The mixture was stirred at 80°C for 3 hours. After cooling, additional sodium hydride (2 g, 60% dispersion in oil, 61 mmol) was added and the mixture was heated at 80°C for 3 hours. After cooling, acetic acid (18.3 ml) was added slowly. After evaporation of the solvents, the residue was triturated in ether to give a solid. Some of this solid (10 g) was suspended in methanol (200 ml) and concentrated sulfuric acid (14 ml) was added. The mixture was stirred at reflux for 6 hours. After cooling, the minerals were filtered off. The filtrate was evaporated. The residue was purified by chromatography on silica gel (eluant: 3% methanol in DCM) to give methyl (2*R*)-2-[(4-oxo-3,4-dihydroquinazolin-5-yl)oxy]propanoate (6.8 g); NMR Spectrum 1.56 (d, 3H), 3.67 (s, 3H), 4.99 (q, 1H), 6.84 (d, 1H), 7.23 (d, 1H), 7.64 (t, 1H), 7.98 (s, 1H); Mass spectrum: MH⁺ 249.

A solution of methyl (2*R*)-2-[(4-oxo-3,4-dihydroquinazolin-5-yl)oxy]propanoate (6.8 g, 27.4 mmol) and 2-(methylamino)ethanol (11 ml, 137 mmol) in methanol ( 20 ml) was heated at reflux for 6 hours. After cooling, the solvents were evaporated under vacuum and the residue was purified by chromatography on silica gel (eluant: 5% methanol in DCM). Trituration in ether and crystallisation in acetonitrile afforded (2*R*)-*N*-(2-hydroxyethyl)-*N-*methyl-2-[(4-oxo-3,4-dihydroquinazolin-5-yl)oxy]propanamide as a white solid (5 g, 63%); Mass spectrum: MH⁺ 292.

A suspension of (2*R*)-*N*-(2-hydroxyethyl)-*N*-methyl-2-[(4-oxo-3,4-dihydroquinazolin-5-yl)oxy]propanamide (4.9 g, 16.8 mmol), acetic anhydride (15.8 ml, 168 mmol) and pyridine (5 ml) was heated at 100°C for 45 minutes. After cooling, the solvents were evaporated under vacuum. The residue was taken in a mixture of methanol (4 ml) and water (4 ml). The mixture was stirred at room temperature for 30 minutes. The solvents were evaporated under vacuum and the residue was purified by chromatography on silica gel (eluant: 3% methanol in DCM) to give 2-(methyl{(2*R*)-2-[(4-oxo-3,4-dihydroquinazolin-5-yl)oxy]propanoyl}amino)ethyl acetate as a white solid (4.8 g, 86%); NMR Spectrum (2 rotamers) 1.50 (m, 3H), 1.91 (s, 3H), 3.11 and 2.84 (s, 3H), 3.52 and 3.74-3.67 (m, 2H), 4.10 (m, 2H), 5.21 (m, 1H), 6.83 and 6.70 (d, 1H), 7.18 (m, 1H), 7.60 (m, 1H), 7.69 (s, 1H); Mass spectrum MH⁺ 334.

The 4-(azepan-1-ylcarbonyl)-3-chloroaniline used as starting material was made as follows:

EDCI (2.45 g, 12.8 mmol) was added to a mixture of 4-amino-2-chlorobenzoic acid (2 g, 11.7 mmol) and azepane (1.32 ml, 11.7 mmol) in DCM (50 ml). The mixture was stirred at room temperature for 3 hours. The mixture was washed with saturated sodium bicarbonate and dried over MgSO₄. After evaporation of the solvents, the residue was purified by chromatography on silica gel (eluant: 2% methanol in DCM) to give 4-(azepan-1-ylcarbonyl)-3-chloroaniline as a pale solid (1.68 g, 57%); NMR Spectrum (CDCl₃) 1.58 (m, 6H), 1.82 (m, 2H), 3.28 (t, 2H), 3.9-3.6 (m, 4H), 6.55 (dd, 1H), 6.67 (d, 1H), 7.02 (d, 1H); Mass spectrum MH⁺ 253.

### Example 2

### (2R)-2-[(4-{[3-chloro-4-(piperidin-1-ylcarbonyl)phenyl]amino}quinazolin-5-yl)oxy]-N-(2-hydroxyethyl)-N-methylpropanamide

Using an analogous procedure to Example 1, 2-(methyl{(2*R*)-2-[(4-oxo-3,4-dihydroquinazolin-5-yl)oxy]propanoyl}amino)ethyl acetate (250 mg, 0.75 mmol) was reacted with 3-chloro-4-(piperidin-1-ylcarbonyl)aniline (189 mg, 0.79 mmol) to give the title compound as a white solid (237 mg, 62%); NMR Spectrum (CDCl₃) (2 rotamers) 1.9-1.4 (m, 9H), 3.04 and 3.23 (s, 3H), 3.27 (m, 2H), 3.9-3.5 (m, 6H), 5.71 and 5.33 (m, 1H), 6.86 and 6.73 (m, 1H), 7.6-7.2 (m, 3H), 8.05-7.85 (m, 1H), 8.40-8.25 (m, 1H), 8.64 and 8.54 (m, 1H); Mass spectrum: MH⁺ 512.

The 3-chloro-4-(piperidin-1-ylcarbonyl)aniline was made from 4-amino-2-chlorobenzoic acid and piperidine according to the procedure in Example 1, starting material 3-chloro-4-(piperidin-1-ylcarbonyl)aniline: Yield: 1.55 g, 56 % (pale solid); NMR Spectrum: (CDCl₃) 1.44 (m, 2H), 1.65 (m, 4H), 3.22 (m, 2H), 4.1-3.5 (m, 4H), 6.55 (dd, 1H), 6.66 (d, 1H), 7.02 (d, 1H); Mass spectrum: MH⁺ 239.

### Example 3

### (2R)-2-[(4-{[3-chloro-4-(pyrrolidin-1-ylcarbonyl)phenyl]amino}quinazolin-5-yl)oxy]-N-(2-hydroxyethyl)-N-methylpropanamide

Using an analogous procedure to Example 1, 2-(methyl{(2*R*)-2-[(4-oxo-3,4-dihydroquinazolin-5-yl)oxy]propanoyl}amino)ethyl acetate (250 mg, 0.75 mmol) was reacted with 3-chloro-4-(pyrrolidin-1-ylcarbonyl)aniline (177 mg, 0.79 mmol) to give the title compound as a white solid (212 mg, 57%); NMR Spectrum (CDCl₃) (2 rotamers) 1.66 (m, 3H), 1.88 (m, 2H), 1.96 (m, 2H), 3.04 and 3.23 (s, 3H), 3.28 (m, 2H), 3.65 (t, 2H), 3.95-3.7 and 3.50 (m, 4H), 5.71 and 5.33 (m, 1H), 6.86 and 6.73 (m, 1H), 7.6-7.2 (m, 3H), 7.97 and 7.91 (m, 1H), 8.37 and 8.32 (m, 1H), 8.63 and 8.52 (m, 1H); Mass spectrum: MH⁺ 498.

The 3-chloro-4-(pyrrolidin-1-ylcarbonyl)aniline was made from 4-amino-2-chlorobenzoic acid and pyrrolidine according an analogous procedure to Example 1, starting material:

3-chloro-4-(pyrrolidin-1-ylcarbonyl)aniline: Yield: 833 mg, 64 %; NMR Spectrum (CDCl₃) 1.87 (m, 2H), 1.95 (m, 2H), 3.24 (m, 2H), 3.62 (m, 2H), 3.85 (m, 2H), 6.56 (dd, 1H), 6.66 (d, 1H), 7.08 (d, 1H); Mass spectrum MH⁺ 225.

### Example 4

### (2R)-2-[(4-{[4-(azepan-1-ylcarbonyl)-3-chlorophenyl]amino}quinazolin-5-yl)oxy]-N,N-dimethylpropanamide

A mixture of (2*R*)-*N,N*-dimethyl-2-[(4-oxo-3,4-dihydroquinazolin-5-yl)oxy]propanamide (200 mg, 0.77 mmol), triphenylphosphine (603 mg, 2.3 mmol) and carbon tetrachloride (2.2 ml, 23 mmol) in 1,2-dichloroethane (5 ml) was stirred at 45°C for 2 hours. The mixture was cooled. 4-(Azepan-1-ylcarbonyl)-3-chloroaniline (202 mg, 0.8 mmol) was added and the solvents were evaporated under vacuum. Acetonitrile (10 ml) was added and the mixture was stirred at 75°C for 3 hours. After cooling, a solution of 7N methanolic ammonia was added and the solvents were evaporated under vacuum. The residue was purified by chromatography on silica gel (eluant: 2% to 4% methanol in DCM) and triturated in a mixture of DCM and pentane to give the title compound as a white solid (241 mg, 65%); NMR Spectrum 1.55 (m, 6H), 1.58 (d, 3H), 1.73 (m, 2H), 2.95 (s, 3H), 3.14 (s, 3H), 3.24 (m, 2H), 3.60 (m, 2H), 5.87 (q, 1H), 7.38 (m, 3H), 7.78 (t, 1H), 8.09 (dd, 1H), 8.45 (d, 1H), 8.63 (s, 1H), 11.31 (s, 1H); Mass spectrum 496

The (2*R*)-*N,N*-dimethyl-2-[(4-oxo-3,4-dihydroquinazolin-5-yl)oxy]propanmide used as starting material was made as follows:

Sodium hydride (1.24 g, 60% in oil, 31 mmol) was added portion-wise to a solution of 5-methoxyquinazolin-4(3*H*)-one (5 g, 28.4 mmol, prepared as described in WO-96/09294, pages 28 and 29) in anhydrous DMF (50 ml) while maintaining the temperature at 25°C. The mixture was stirred at room temperature for 30 minutes. Chloromethyl pivalate (4.45 ml, 31 mmol) was added at room temperature and the reaction mixture stirred for 3 hours. Additional sodium hydride (0.12 g, 3 mmol) and chloromethyl pivalate (0.67 ml, 4.5 mmol) were added and the mixture was stirred another hour. After evaporation of the solvents under high vacuum, the mixture was diluted with water and extracted with DCM. After drying with magnesium sulfate and evaporation of the solvents, the residue was purified by chromatography on silica gel (eluant: ethyl acetate- petroleum ether, 6:4 to 8:2) to give (5-methoxy-4-oxoquinazolin-3(4*H*)-yl)methyl pivalate as a white solid (7.4 g, 90%); HPLC t_{R} 2.69 min; Mass spectrum MH⁺ 291.

Magnesium bromide (7 g, 38 mmol) was added to a solution of (5-methoxy-4-oxoquinazolin-3(4*H*)-yl)methyl pivalate (7.4 g, 25.5 mmol) in pyridine (25 ml). The mixture was stirred at 120°C for one hour. After cooling, the solvents were evaporated under high vacuum. Diluted acetic acid (15 ml in 100 ml water) was added. The precipitated solid was filtered, washed with water and dried under high vacuum in the presence of P₂O₅ to give (5-hydroxy-4-oxoqliinazolin-3(4*H*)-yl)methyl pivalate as a white solid (6.33 g, 90%); NMR Spectrum (CDCl₃) 1.23 (s, 9H), 5.93 (s, 2H), 6.99 (d, 1H), 7.22 (d, 1H), 7.68 (t, 1H), 8.21 (s, 1H); Mass spectrum MH⁺ 277.

DTAD (13.34 g, 58 mmol) was added portion-wise to an ice-cooled solution of (5-hydroxy-4-oxoquinazolin-3(4*H*)-yl)methyl pivalate (8 g, 29 mmol), triphenylphosphine (15.2 g, 58 mmol) and (*S*)-*N,N*-dimethyl lactamide (5.1 g, 43.5 mmol; prepared as described in Larcheveque M., Synthesis 1986, 1, 60) in DCM (300 ml). The mixture was stirred at room temperature for one hour. After evaporation of the solvents under vacuum, the residue was diluted with 6N methanolic ammonia (100 ml). The mixture was stirred at room temperature for 18 hours. After evaporation of the solvents, the residue was triturated in ether. The resulting solid was filtered and purified further by chromatography on silica gel (eluant: 3 to 5% methanol in DCM) to give (2*R*)-*N,N*-dimethyl-2-[(4-oxo-3,4-dihydroquinazolin-5-yl)oxy]propanamide as a white solid (5.4 g, 71%); NMR Spectrum (CDCl₃) 1.77 (d, 3H), 2.94 (s, 3H), 3.19 (s, 3H), 5.10 (q, 1H), 6.92 (d, 1H), 7.35 (d, 1H), 7.63 (t, 1H), 8.00 (s, 1H); Mass spectrum MH⁺ 262.

### Example 5

### (2R)-2-[(4-{[3-chloro-4-(piperidin-1-ylcarbonyl)phenyl]amino}quinazolin-5-yl)oxy]-N,N-dimethylpropanamide

Using the same procedure as in Example 4, (2*R*)-*N,N*-dimethyl-2-[(4-oxo-3,4-dihydroquinazolin-5-yl)oxy]propanamide (200 mg, 0.77 mmol) was reacted with 3-chloro-4-(piperidin-1-ylcarbonyl)aniline (190 mg, 0.8 mmol) to give the title compound as a white solid (210 mg, 57%); NMR Spectrum 1.7-1.4 (m, 6H), 1.58 (d, 3H), 2.95 (s, 3H), 3.14 (s, 3H), 3.35 (m, 2H), 3.63 (m, 2H), 5.87 (q, 1H), 7.39 (m, 3H), 7.78 (t, 1H), 8.09 (dd, 1H), 8.45 (d, 1H), 8.63 (s, 1H), 11.31 (s, 1H); Mass spectrum 482.

### Example 6

### (2R)-2-[(4-{[3-chloro-4-(pyrrolidin-1-ylcarbonyl)phenyl] amino}quinazolin-5-yl)oxy]-N,N-dimethylpropanamide

Using the same procedure as in Example 4, (2*R*)-*N,N*-dimethyl-2-[(4-oxo-3,4-dihydroquinazolin-5-yl)oxy]propanamide (200 mg, 0.77 mmol) was reacted with 3-chloro-4-(pyrrolidin-1-ylcarbonyl)aniline (179 mg, 0.8 mmol) to give the title compound as a white solid (202 mg, 56%); NMR Spectrum 1.58 (d, 3H), 1.90-1.83 (m, 4H), 2.95 (s, 3H), 3.14 (s, 3H), 3.16 (t, 2H), 3.48 (t, 2H), 5.87 (q, 1H), 7.39 (m, 3H), 7.78 (t, 1H), 8.09 (dd, 1H), 8.45 (d, 1H), 8.63 (s, 1H), 11.31 (s, 1H); Mass spectrum 468.

## Claims

1. A quinazoline derivative of the Formula **I**: 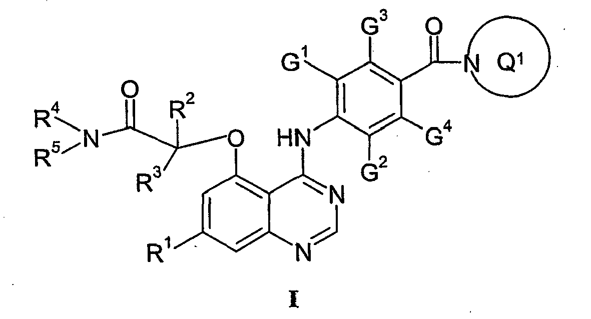 wherein:
**R¹** is selected from hydrogen, hydroxy, (1-4C)alkoxy and (1-4C)alkoxy(1-4C)alkoxy;
**R² and R³,** which may be the same or different, are selected from hydrogen, (1-4C)alkyl, (2-4C)alkenyl and (2-4C)alkynyl, which (1-4C)alkyl optionally bears one or more hydroxy substituents;
**R⁴ and R⁵,** which may be the same or different, are selected from hydrogen, (1-4C)alkyl, (3-4C)alkenyl and (3-4C)alkynyl, which (1-4C)alkyl optionally bears one or more substituents independently selected from halogeno, cyano, hydroxy, amino, (1-4C)alkylamino, di-[(1-4C)alkyl]amino and (1-4C)alkoxy, or
R⁴ and R⁵ together with the nitrogen atom to which they are attached form a saturated 4, 5, 6 or 7 membered heterocyclic ring containing one nitrogen heteroatom and optionally containing one or more additional heteroatoms independently selected from oxygen, nitrogen and sulfur,
and wherein any heterocyclic ring formed by R⁴, R⁵ and the nitrogen atom to which they are attached optionally bears one or more substituents independently selected from halogeno, cyano, hydroxy, (1-4C)alkyl and (1-4C)alkoxy;
**G¹ and G²,** which may be the same or different, are selected from hydrogen and halogeno;
**G³ and G⁴,** which may be the same or different, are selected from hydrogen, halogeno, cyano, (1-4C)alkyl, (1-4C)alkoxy, (2-4C)alkenyl and (2-4C)alkynyl;
**the ring -NQ¹** is a nitrogen-linked, saturated or partially unsaturated, 4, 5, 6, 7 or 8 membered heterocyclic ring containing one nitrogen heteroatom and optionally containing one or more additional heteroatoms independently selected from oxygen, nitrogen and sulfur, and which heterocyclic ring NQ¹ optionally bears one or more substituents independently selected from halogeno, cyano, hydroxy, (1-4C)alkyl, (1-4C)allcoxy and hydroxy-(1-4C)allcyl;
and wherein any heterocyclic ring formed by R⁴, R⁵ and the nitrogen atom to which they are attached and/or any heterocyclic ring NQ¹ optionally bears 1 or 2 oxo or thioxo substituents;
or a pharmaceutically acceptable salt thereof.

2. A quinazoline derivative according to claim 1, wherein R¹ is selected from hydrogen, hydroxy, methoxy, ethoxy and methoxyethoxy.

3. A quinazoline derivative according to claim 1 or 2, wherein R¹ is hydrogen.

4. A quinazoline derivative according to any one or more of the preceding claims, wherein G¹ and G² are both hydrogen.

5. A quinazoline derivative according to any one or more of the preceding claims, wherein one of G³ or G⁴ is halogeno and the other of G³ and G⁴ is hydrogen.

6. A quinazoline derivative according to any one or more of the preceding claims, wherein R² and R³, which are the same or different, are selected from hydrogen and (1-2C)alkyl.

7. A quinazoline derivative according to any one or more of the preceding claims, wherein R² is hydrogen and R³ is (1-2C)alkyl.

8. A quinazoline derivative according to any one or more of the preceding claims, wherein R⁴ and R⁵, which may be the same or different, are selected from hydrogen, (1-4C)alkyl, (3-4C)alkenyl and (3-4C)alkynyl, which (1-4C)alkyl optionally bears one or more substituents independently selected from halogeno, cyano, hydroxy, amino, (1-4C)alkylamino, di-[(1-4C)alkyl]amino and (1-4C)alkoxy.

9. A quinazoline derivative according to any one or more of claims 1 to 7, wherein R⁴ and R⁵, which may be the same or different, are selected from hydrogen and (1-4C)alkyl, which (1-4C)alkyl optionally bears one or more hydroxy substituents, or
R⁴ and R⁵ together with the nitrogen atom to which they are attached form a saturated 4, 5, 6 or 7 membered heterocyclic ring which optionally contains one or more additional heteroatoms independently selected from oxygen, nitrogen and sulfur,
and wherein any heterocyclic ring formed by R⁴, R⁵ and the nitrogen atom to which they are attached optionally bears one or more substituents independently selected from halogeno, cyano, hydroxy, (1-4C)allcyl and (1-4C)alkoxy,
and wherein any heterocyclic ring formed by R⁴, R⁵ and the nitrogen atom to which they are attached optionally bears 1 or 2 oxo or thioxo substituents.

10. A quinazoline derivative according to any one or more of the preceding claims, wherein R⁴ and R⁵ are both (1-4C)alkyl, which (1-4C)alkyl optionally bears one or more hydroxy substituents.

11. A quinazoline derivative according to any one or more of the preceding claims, wherein R⁴ is methyl and R⁵ is (1-4C)alkyl, which (1-4C)alkyl optionally bears one or more hydroxy substituents.

12. A quinazoline derivative according to any one or more of the preceding claims, wherein R⁴ and R⁵ are both methyl.

13. A quinazoline derivative according to any one or more of claims 1 to 11, wherein R⁴ is methyl and R⁵ is 2-hydroxyethyl.

14. A quinazoline derivative according to any one or more of the preceding claims, wherein the ring -NQ¹ is a nitrogen-linked, saturated or partially unsaturated, 5, 6 or 7 membered heterocyclic ring containing one nitrogen heteroatom and optionally containing one or two additional heteroatoms independently selected from oxygen, nitrogen and sulfur, and which heterocyclic ring -NQ¹ optionally bears one or more substituents independently selected from halogeno, cyano, hydroxy, (1-4C)alkyl, (1-4C)alkoxy and hydroxy-(1-4C)alkyl, and which heterocyclic ring -NQ¹ optionally bears 1 or 2 oxo or thioxo substituents.

15. A quinazoline derivative according to any one or more of the preceding claims, wherein the ring -NQ¹ is a nitrogen-linked, saturated or partially unsaturated, 5, 6 or 7 membered heterocyclic ring containing one nitrogen heteroatom, and which heterocyclic ring -NQ¹ optionally bears one or more substituents independently selected from halogeno, cyano, hydroxy, (1-4C)alkyl, (1-4C)alkoxy and hydroxy-(1-4C)alkyl, and which heterocyclic ring -NQ¹ optionally bears 1 or 2 oxo or thioxo substituents.

16. A quinazoline derivative according to any one or more of the preceding claims, wherein the ring -NQ¹ is selected from azepan-1-yl, piperidin-1-yl and pyrrolidin-1-yl.

17. A quinazoline derivative of the Formula **I** selected from one or more of the following:
(2*R*)-2-[(4-{[4-(azepan-1-ylcarbonyl)-3-chlorophenyl]amino}quinazolin-5-yl)oxy]-*N*-(2-hydroxyethyl)-*N-*methylpropanamide;
(2*R*)-2-[(4-{[3-chloro-4-(piperidin-1-ylcarbonyl)phenyl]amino}quinazolin-5-yl)oxy]-*N-*(2-hydroxyethyl)-*N-*methylpropanamide;
(2*R*)-2-[(4-{[3-chloro-4-(pyrrolidin-1-ylcarbonyl)phenyl]amino}quinazolin-5-yl)oxy]-*N-*(2-hydroxyethyl)-*N-*methylpropanamide;
(2*R*)-2-[(4-{[4-(azepan-1-ylcarbonyl)-3-chlorophenyl]amino}quinazolin-5-yl)oxy]-*N,N-*dimethylpropanamide;
(2*R*)-2-[(4-{[3-chloro-4-(piperidin-1-ylcarbonyl)phenyl]amino} quinazolin-5-yl)oxy]-*N,N-*dimethylpropanamide; and
(2*R*)-2-[(4-{[3-chloro-4-(pyrrolidin-1-ylcarbonyl)phenyl]amino}quinazolin-5-yl)oxy]-*N,N-*dimethylpropanamide;
or a pharmaceutically acceptable salt thereof.

18. A pharmaceutical composition which comprises a quinazoline derivative of the Formula **I**, or a pharmaceutically acceptable salt thereof, according to any one or more of claims 1 to 17 in association with a pharmaceutically acceptable diluent or carrier.

19. A pharmaceutical product which comprises a quinazoline derivative of the Formula **I**, or a pharmaceutically acceptable salt thereof, according to any one or more of claims 1 to 17 and an additional anti-tumour agent for the conjoint treatment of cancer.

20. A quinazoline derivative of the Formula **I**, or a pharmaceutically acceptable salt thereof, according to any one or more of claim 1 to 17 for use as a medicament.

21. Use of a quinazoline derivative of the Formula **I,** or a pharmaceutically acceptable salt thereof, according to any one or more of claims 1 to 17 in the manufacture of a medicament for use in the production of an anti-proliferative effects in a warm-blooded animal.

22. A quinazoline derivative of the Formula **I**, or a pharmaceutically acceptable salt thereof, according to any one or more of claims 1 to 17 for the production of an anti-proliferative effect in a warm-blooded animal.

23. Use of a quinazoline derivative of the Formula **I**, or a pharmaceutically acceptable salt thereof, according to any one or more of claims 1 to 17 in the manufacture of a medicament for use in the treatment of a disease or medical condition mediated alone or in part by erbB receptor tyrosine kinase.

24. A quinazoline derivative of the Formula **I**, or a pharmaceutically acceptable salt thereof, according to any one or more of claims 1 to 17 for the treatment of a disease or medical condition mediated alone or in part by erbB receptor tyrosine kinase in a warm-blooded animal.

25. Use of a quinazoline derivative of the Formula **I**, or a pharmaceutically acceptable salt thereof, according to any one or more of claims 1 to 17 in the manufacture of a medicament for use in the prevention or treatment of those tumours which are sensitive to inhibition of one or more erbB receptor tyrosine kinase that are involved in the signal transduction steps which lead to the proliferation and/or survival of tumour cells in a warm-blooded animal.

26. A quinazoline derivative of the Formula **I**, or a pharmaceutically acceptable salt thereof, according to any one or more of claims 1 to 17 for the prevention or treatment of those tumours which are sensitive to inhibition of one or more erbB receptor tyrosine kinase that are involved in the signal transduction steps which lead to the proliferation and/or survival of tumour cells in a warm-blooded animal.

27. Use of a quinazoline derivative of the Formula **I**, or a pharmaceutically acceptable salt thereof, according to any one or more of claims 1 to 17 in the manufacture of a medicament for use in the treatment of cancer.

28. A quinazoline derivative of the Formula **I**, or a pharmaceutically acceptable salt thereof, according to any one or more of claims 1. to 17 for the treatment of cancer in a warm-blooded animal.

29. A process for the preparation of a quinazoline derivative of the Formula **I,** or a pharmaceutically acceptable salt thereof, according to claim 1 which comprises:
(a) the reaction of a quinazoline of the Formula **II**: 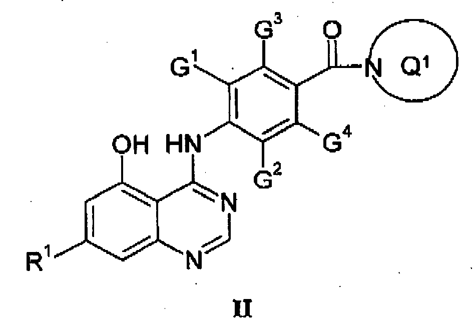 wherein R¹, G¹, G², G³, G⁴ and the ring -NQ¹ have any of the meanings defined in claim 1 except that any functional group is protected if necessary, with an amide of the Formula **III:** 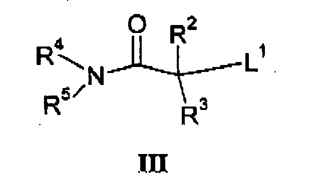 wherein R², R³, R⁴ and R⁵ have any of the meanings defined in claim 1 except that any functional group is protected if necessary and L¹ is a suitable displaceable group; or
(b) the coupling, conveniently in the presence of a suitable base, of a quinazoline of the Formula **IV** (or a suitable salt thereof): 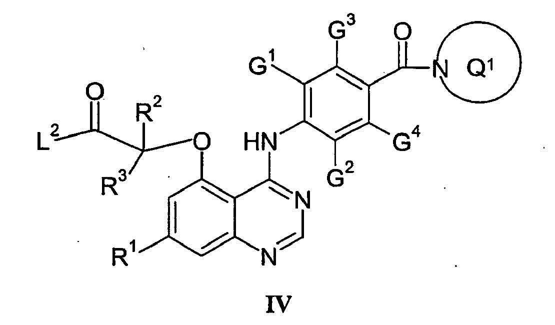 wherein R¹, R², R³, G¹, G², G³, G⁴ and the ring -NQ¹ have any of the meanings defined in claim 1 except that any functional group is protected if necessary, and L² is a suitable displaceable group or L² is hydroxy, which hydroxy group is conveniently combined with a suitable coupling agent to produce a displaceable group, with an amine of the Formula **V**: 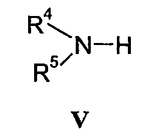 wherein R⁴ and R⁵ have any of the meanings defined in claim 1 except that any functional group is protected if necessary; or
(c) for quinazoline derivatives of the Formula **I** wherein R² is 2-hydroxyethyl, the reaction of a quinazoline of the Formula **VI**: 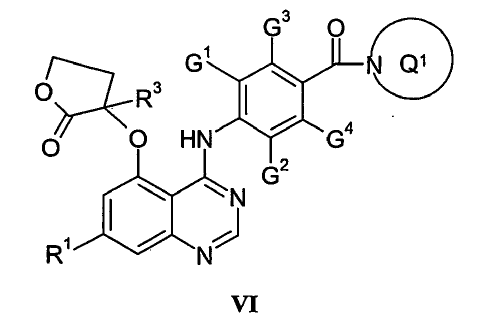 wherein R¹, R³, G¹, G², G³, G⁴ and the ring -NQ¹ have any of the meanings defined in claim 1 except that any functional group is protected if necessary, with an amine of the Formula **V**: 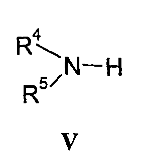 wherein R⁴ and R⁵ have any of the meanings defined in claim 1 except that any functional group is protected if necessary; or
(d) the reaction of a quinazoline of the Formula **VII**: 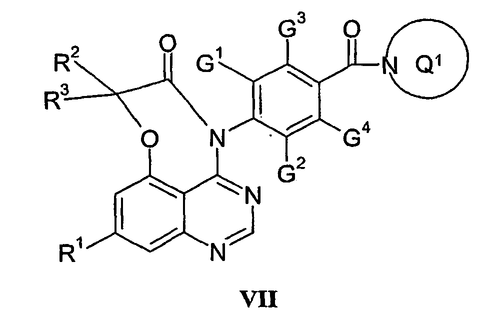 wherein R¹, R², R³, G¹, G², G³, G⁴ and the ring -NQ¹ have any of the meanings defined in claim 1 except that any functional group is protected if necessary, with an amine of the Formula **V**: 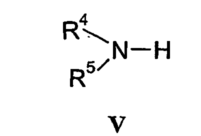 wherein R⁴ and R⁵ have any of the meanings defined in claim 1 except that any functional group is protected if necessary; or
(e) the reaction of a quinazolin-4(3H)-one of the Formula **VIII:** 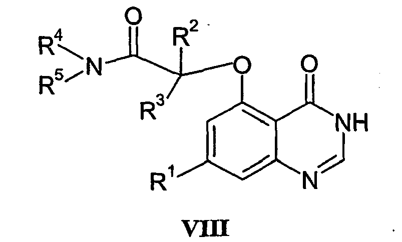 wherein R¹, R², R³, R⁴ and R⁵ have any of the meanings defined in claim 1 except that any functional group is protected if necessary, with a suitable activating group and an amine of the Formula **IX:** 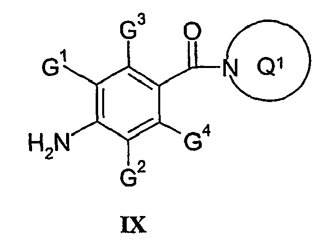 wherein G¹, G², G³, G⁴ and the ring -NQ¹ have any of the meanings defined in claim 1 except that any functional group is protected if necessary; or
(f) the reaction of a quinazoline of the Formula **X**: 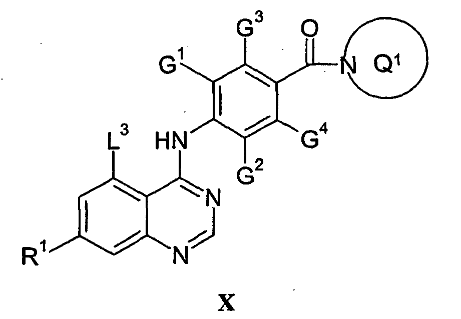 wherein R¹, G¹, G², G³, G⁴ and the ring -NQ¹ have any of the meanings defined in claim 1 except that any functional group is protected if necessary and L³ is a suitable displaceable group with a compound of the Formula **XI:** 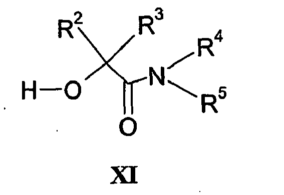 wherein R², R³, R⁴ and R⁵ have any of the meanings defined in claim 1 except that any functional group is protected if necessary; or
(g) the coupling of a quinazoline of the Formula **XII:** 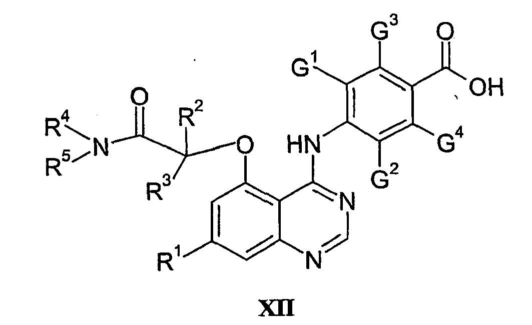 wherein R¹, R², R³, R⁴, R⁵, G¹, G², G³ and G⁴ have any of the meanings defined in claim 1 except that any functional group is protected if necessary, with a cyclic amine compound of the Formula **XIII**: 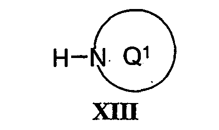 wherein the ring -NQ¹ has any of the meanings defined in claim 1 except that any functional group is protected if necessary;
and thereafter, if necessary:
(i) converting a quinazoline derivative of the Formula **I** into another quinazoline derivative of the Formula **I**;
(ii) removing any protecting group that is present;
(iii) forming a pharmaceutically acceptable salt.

30. A compound of the Formula **II, IV, VI, VII** and/or **XII** as defined in claim 29, or a salt thereof.

## Patentansprüche

1. Chinazolinderivat der Formel I: 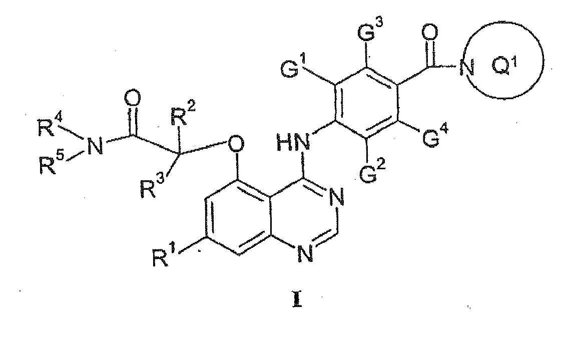 worin:
R¹ unter Wasserstoff, Hydroxy, C₁₋₄-Alkoxy und C₁₋₄-Alkoxy-C₁₋₄-alkoxy ausgewählt ist;
R² und R³ gleich oder verschieden sein können und unter Wasserstoff, C₁-₄-Alkyl, C₂₋₄-Alkenyl und C₂-₄-Alkinyl ausgewählt sind, wobei das C₁₋₄-Alkyl gegebenenfalls einen oder mehrere Hydroxysubstituenten trägt;
R⁴ und R⁵ gleich oder verschieden sein können und unter Wasserstoff, C₁₋₄-Alkyl, C₃₋₄-Alkenyl und C₃₋₄-Alkinyl ausgewählt sind, wobei das C₁₋₄-Alkyl gegebenenfalls einen oder mehrere unabhängig voneinander unter Halogen, Cyano, Hydroxy, Amino, C₁₋₄-Alkylamino, Di(C₁₋₄-alkyl)amino und C₁₋₄-Alkoxy ausgewählte Substituenten trägt, oder
R⁴ und R⁵ zusammen mit dem Stickstoffatom, an das sie gebunden sind, einen gesättigten 4-, 5-, 6- oder 7-gliedrigen heterocyclischen Ring bilden, der ein Stickstoff-Heteroatom und gegebenenfalls ein oder mehrere zusätzliche Heteroatome, die unabhängig voneinander unter Sauerstoff, Stickstoff und Schwefel ausgewählt sind, enthält, und worin jeder durch R⁴, R⁵ und das Stickstoffatom, an das sie gebunden sind, gebildete heterocyclische Ring gegebenenfalls einen oder mehrere unabhängig voneinander unter Halogen, Cyano, Hydroxy, C₁₋₄-Alkyl und C₁₋₄-Alkoxy ausgewählte Substituenten trägt;
G¹ und G² gleich oder verschieden sein können und unter Wasserstoff und Halogen ausgewählt sind;
G³ und G⁴ gleich oder verschieden sein können und unter Wasserstoff, Halogen, Cyano, C₁₋₄-Alkyl, C₁₋₄-Alkoxy, C₂₋₄-Alkenyl und C₂₋₄-Alkinyl ausgewählt sind;
der Ring -NQ¹ für einen über Stickstoff gebundenen gesättigten oder teilweise ungesättigten 4-, 5-, 6-, 7- oder 8-gliedrigen heterocyclischen Ring steht, der ein Stickstoff-Heteroatom und gegebenenfalls ein oder mehrere zusätzliche Heteroatome, die unabhängig voneinander unter Sauerstoff, Stickstoff und Schwefel ausgewählt sind, enthält und gegebenenfalls einen oder mehrere unabhängig voneinander unter Halogen, Cyano, Hydroxy, C₁₋₄-Alkyl, C₁₋₄-Alkoxy und Hydroxy-C₁₋₄-alkyl ausgewählte Substituenten trägt;
und worin jeder durch R⁴, R⁵ und das Stickstoffatom, an das sie gebunden sind, gebildete heterocyclische Ring und/oder jeder heterocyclische Ring -NQ¹ gegebenenfalls 1 oder 2 Oxo- oder Thioxosubstituenten trägt;
oder ein pharmazeutisch annehmbares Salz davon.

2. Chinazolinderivat nach Anspruch 1, worin R¹ unter Wasserstoff, Hydroxy, Methoxy, Ethoxy und Methoxyethoxy ausgewählt ist.

3. Chinazolinderivat nach Anspruch 1 oder 2, worin R¹ für Wasserstoff steht.

4. Chinazolinderivat nach einem oder mehreren der vorhergehenden Ansprüche, worin G¹ und G² beide für Wasserstoff stehen.

5. Chinazolinderivat nach einem oder mehreren der vorhergehenden Ansprüche, worin eine der Gruppen G³ und G⁴ für Halogen steht und die andere für Wasserstoff steht.

6. Chinazolinderivat nach einem oder mehreren der vorhergehenden Ansprüche, worin R² und R³ gleich oder verschieden sein können und unter Wasserstoff und C₁₋₂-Alkyl ausgewählt sind.

7. Chinazolinderivat nach einem oder mehreren der vorhergehenden Ansprüche, worin R² für Wasserstoff steht und R³ für C₁₋₂-Alkyl steht.

8. Chinazolinderivat nach einem oder mehreren der vorhergehenden Ansprüche, worin R⁴ und R⁵ gleich oder verschieden sein können und unter Wasserstoff, C₁₋₄-Alkyl, C₃₋₄-Alkenyl und C₃₋₄-Alkinyl ausgewählt sind, wobei das C₁₋₄-Alkyl gegebenenfalls einen oder mehrere unabhängig voneinander unter Halogen, Cyano, Hydroxy, Amino, C₁₋₄-Alkylamino, Di(C₁₋₄-alkyl)amino und C₁₋₄-Alkoxy ausgewählte Substituenten trägt.

9. Chinazolinderivat nach einem oder mehreren der Ansprüche 1 bis 7, worin R⁴ und R⁵ gleich oder verschieden sein können und unter Wasserstoff und C₁₋₄-Alkyl ausgewählt sind, wobei das C₁₋₄-Alkyl gegebenenfalls einen oder mehrere Hydroxysubstituenten trägt, oder
R⁴ und R⁵ zusammen mit dem Stickstoffatom, an das sie gebunden sind, einen gesättigten 4-, 5-, 6- oder 7-gliedrigen heterocyclischen Ring bilden, der gegebenenfalls ein oder mehrere zusätzliche Heteroatome, die unabhängig voneinander unter Sauerstoff, Stickstoff und Schwefel ausgewählt sind, enthält,
und worin jeder durch R⁴, R⁵ und das Stickstoffatom, an das sie gebunden sind, gebildete heterocyclische Ring gegebenenfalls einen oder mehrere unabhängig voneinander unter Halogen, Cyano, Hydroxy, C₁₋₄-Alkyl und C₁₋₄-Alkoxy ausgewählte Substituenten trägt,
und worin jeder durch R⁴, R⁵ und das Stickstoffatom, an das sie gebunden sind, gebildete heterocyclische Ring gegebenenfalls 1 oder 2 Oxo- oder Thioxosubstituenten trägt.

10. Chinazolinderivat nach einem oder mehreren der vorhergehenden Ansprüche, worin R⁴ und R⁵ beide für C₁-₄-Alkyl stehen, wobei das C₁₋₄-Alkyl gegebenenfalls einen oder mehrere Hydroxysubstituenten trägt.

11. Chinazolinderivat nach einem oder mehreren der vorhergehenden Ansprüche, worin R⁴ für Methyl steht und R⁵ für C₁₋₄-Alkyl steht, wobei das C₁₋₄-Alkyl gegebenenfalls einen oder mehrere Hydroxysubstituenten trägt.

12. Chinazolinderivat nach einem oder mehreren der vorhergehenden Ansprüche, worin R⁴ und R⁵ beide für Methyl stehen.

13. Chinazolinderivat nach einem oder mehreren der Ansprüche 1 bis 11, worin R⁴ für Methyl steht und R⁵ für 2-Hydroxyethyl steht.

14. Chinazolinderivat nach einem oder mehreren der vorhergehenden Ansprüche, worin der Ring -NQ¹ für einen über Stickstoff gebundenen gesättigten oder teilweise ungesättigten 5-, 6- oder 7-gliedrigen heterocyclischen Ring steht, der ein Stickstoff-Heteroatom und gegebenenfalls ein oder zwei zusätzliche Heteroatome, die unabhängig voneinander unter Sauerstoff, Stickstoff und Schwefel ausgewählt sind, enthält, gegebenenfalls einen oder mehrere unabhängig voneinander unter Halogen, Cyano, Hydroxy, C₁₋₄-Alkyl, C₁₋₄-Alkoxy und Hydroxy-C₁₋₄-alkyl ausgewählte Substituenten trägt und gegebenenfalls 1 oder 2 Oxo- oder Thioxosubstituenten trägt.

15. Chinazolinderivat nach einem oder mehreren der vorhergehenden Ansprüche, worin der Ring -NQ¹ für einen über Stickstoff gebundenen gesättigten oder teilweise ungesättigten 5-, 6- oder 7-gliedrigen heterocyclischen Ring steht, der ein Stickstoff-Heteroatom enthält, gegebenenfalls einen oder mehrere unabhängig voneinander unter Halogen, Cyano, Hydroxy, C₁₋₄-Alkyl, C₁₋₄-Alkoxy und Hydroxy-C₁₋₄-alkyl ausgewählte Substituenten trägt und gegebenenfalls 1 oder 2 Oxo- oder Thioxosubstituenten trägt.

16. Chinazolinderivat nach einem oder mehreren der vorhergehenden Ansprüche, worin der Ring -NQ¹ unter Azepan-1-yl, Piperidin-1-yl und Pyrrolidin-1-yl ausgewählt ist.

17. Chinazolinderivat der Formel I, ausgewählt unter einer oder mehreren der folgenden Verbindungen:
(2*R*)-2-[(4-{[4-(Azepan-1-ylcarbonyl)-3-chlorphenyl]amino}chinazolin-5-yl)oxy]-*N-*(2-hydroxyethyl)-*N-*methylpropanamid;
(2*R*)-2-[(4-{[3-Chlor-4-(piperidin-1-ylcarbonyl)-phenyl]amino}chinazolin-5-yl)oxy]-*N-*(2-hydroxyethyl)-*N-*methylpropanamid;
(2*R*)-2-[(4-{[3-Chlor-4-(pyrrolidin-1-ylcarbonyl)-phenyl]amino}chinazolin-5-yl)oxy]-*N-*(2-hydroxyethyl)-*N-*methylpropanamid;
(2*R*)-2-[(4-{[4-(Azepan-1-ylcarbonyl)-3-chlorphenyl]amino}chinazolin-5-yl)oxy]-*N,N*-dimethyl-propanamid;
(2*R*)-2-[(4-{[3-Chlor-4-(piperidin-1-ylcarbonyl)-phenyl]amino}chinazolin-5-yl)oxy]-*N,N*-dimethyl-propanamid und
(2*R*)-2-[(4-{[3-Chlor-4-(pyrrolidin-1-ylcarbonyl)-phenyl]amino}chinazolin-5-yl)oxy]-*N,N*-dimethyl-propanamid;
oder ein pharmazeutisch annehmbares Salz davon.

18. Pharmazeutische Zusammensetzung, die ein Chinazolinderivat der Formel I oder ein pharmazeutisch annehmbares Salz davon nach einem oder mehreren der Ansprüche 1 bis 17 zusammen mit einem pharmazeutisch annehmbaren Verdünnungsmittel oder Träger enthält.

19. Pharmazeutisches Produkt, das ein Chinazolinderivat der Formel I oder ein pharmazeutisch annehmbares Salz davon nach einem oder mehreren der Ansprüche 1 bis 17 und ein zusätzliches Antitumormittel enthält, für die Kombinationsbehandlung von Krebs.

20. Chinazolinderivat der Formel I oder ein pharmazeutisch annehmbares Salz davon nach einem oder mehreren der Ansprüche 1 bis 17 zur Verwendung als Arzneimittel.

21. Verwendung eines Chinazolinderivats der Formel I oder eines pharmazeutisch annehmbaren Salzes davon nach einem oder mehreren der Ansprüche 1 bis 17 bei der Herstellung eines Arzneimittels zur Verwendung bei der Erzielung einer antiproliferativen Wirkung bei einem Warmblüter.

22. Chinazolinderivat der Formel I oder ein pharmazeutisch annehmbares Salz davon nach einem oder mehreren der Ansprüche 1 bis 17 zur Erzielung einer antiproliferativen Wirkung bei einem Warmblüter.

23. Verwendung eines Chinazolinderivats der Formel I oder eines pharmazeutisch annehmbaren Salzes davon nach einem oder mehreren der Ansprüche 1 bis 17 bei der Herstellung eines Arzneimittels zur Verwendung bei der Behandlung einer Erkrankung oder eines medizinischen Zustands, die bzw. der allein oder teilweise durch erbB-Rezeptor-Tyrosinkinase vermittelt wird.

24. Chinazolinderivat der Formel I oder ein pharmazeutisch annehmbares Salz davon nach einem oder mehreren der Ansprüche 1 bis 17 zur Behandlung einer Erkrankung oder eines medizinischen Zustands, die bzw. der allein oder teilweise durch erbB-Rezeptor-Tyrosinkinase vermittelt wird, bei einem Warmblüter.

25. Verwendung eines Chinazolinderivats der Formel I oder eines pharmazeutisch annehmbaren Salzes davon nach einem oder mehreren der Ansprüche 1 bis 17 bei der Herstellung eines Arzneimittels zur Verwendung bei der Prävention oder Behandlung derjenigen Tumore, die gegenüber der Inhibierung einer oder mehrerer der erbB-Rezeptor-Tyrosinkinasen, die an den zur Proliferation und/oder zum Überleben von Tumorzellen führenden Signaltransduktionsschritten beteiligt sind, empfindlich sind, bei einem Warmblüter.

26. Chinazolinderivat der Formel I oder ein pharmazeutisch annehmbares Salz davon nach einem oder mehreren der Ansprüche 1 bis 17 zur Prävention oder Behandlung derjenigen Tumore, die gegenüber der Inhibierung einer oder mehrerer der erbB-Rezeptor-Tyrosinkinasen, die an den zur Proliferation und/oder zum Überleben von Tumorzellen führenden Signaltransduktionsschritten beteiligt sind, empfindlich sind, bei einem Warmblüter.

27. Verwendung eines Chinazolinderivats der Formel I oder eines pharmazeutisch annehmbaren Salzes davon nach einem oder mehreren der Ansprüche 1 bis 17 bei der Herstellung eines Arzneimittels zur Behandlung von Krebs.

28. Chinazolinderivat der Formel I oder ein pharmazeutisch annehmbares Salz davon nach einem oder mehreren der Ansprüche 1 bis 17 zur Behandlung von Krebs bei einem Warmblüter.

29. Verfahren zur Herstellung eines Chinazolinderivats der Formel I oder eines pharmazeutisch annehmbaren Salzes davon nach Anspruch 1, bei dem man:
(a) ein Chinazolin der Formel II: 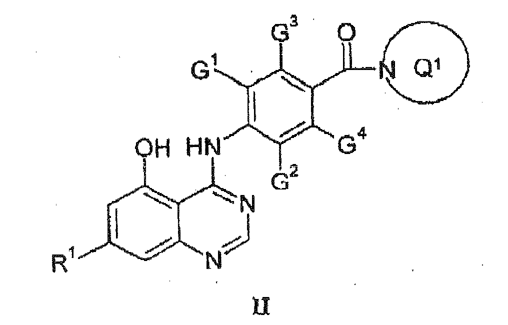 worin R¹, G¹, G², G³, G⁴ und der Ring -NQ¹ eine der in Anspruch 1 angegebenen Bedeutungen besitzen, wobei jedoch jede funktionelle Gruppe gegebenenfalls geschützt ist, mit einem Amid der Formel III: 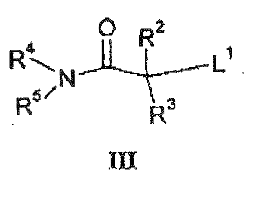 worin R², R³, R⁴ und R⁵ eine der in Anspruch 1 angegebenen Bedeutungen besitzen, wobei jedoch jede funktionelle Gruppe gegebenenfalls geschützt ist, und L¹ für eine geeignete verdrängbare Gruppe steht, umsetzt; oder
(b) ein Chinazolin der Formel IV (oder ein geeignetes Salz davon): 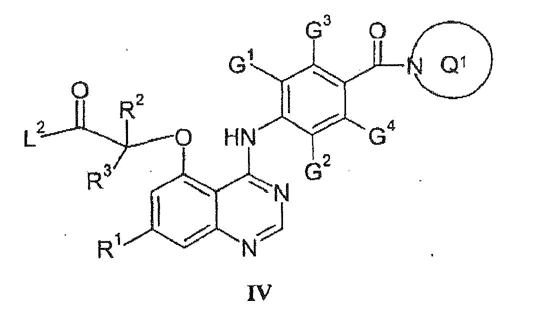 worin R¹, R², R³, G¹, G², G³, G⁴ und der Ring -NQ¹ eine der in Anspruch 1 angegebenen Bedeutungen besitzen, wobei jedoch jede funktionelle Gruppe gegebenenfalls geschützt ist, und L² für eine geeignete verdrängbare Gruppe steht oder L² für Hydroxy steht, wobei die Hydroxygruppe zweckmäßigerweise mit einem geeigneten Kupplungsmittel zur Bildung einer verdrängbaren Gruppe kombiniert wird, mit einem Amin der Formel V: 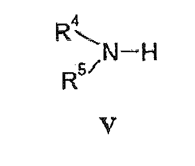 worin R⁴ und R⁵ eine der in Anspruch 1 angegebenen Bedeutungen besitzen, wobei jedoch jede funktionelle Gruppe gegebenenfalls geschützt ist, kuppelt, zweckmäßigerweise in Gegenwart einer geeigneten Base; oder
(c) für Chinazolinderivate der Formel I, worin R² für 2-Hydroxyethyl steht, ein Chinazolin der Formel VI: 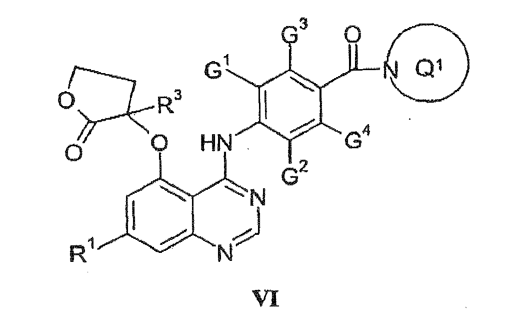 worin R¹, R³, G¹, G², G³, G⁴ und der Ring -NQ¹ eine der in Anspruch 1 angegebenen Bedeutungen besitzen, wobei jedoch jede funktionelle Gruppe gegebenenfalls geschützt ist, mit einem Amin der Formel V: 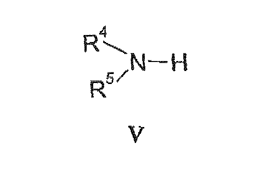 worin R⁴ und R⁵ eine der in Anspruch 1 angegebenen Bedeutungen besitzen, wobei jedoch jede funktionelle Gruppe gegebenenfalls geschützt ist, umsetzt; oder
(d) ein Chinazolin der Formel VII: 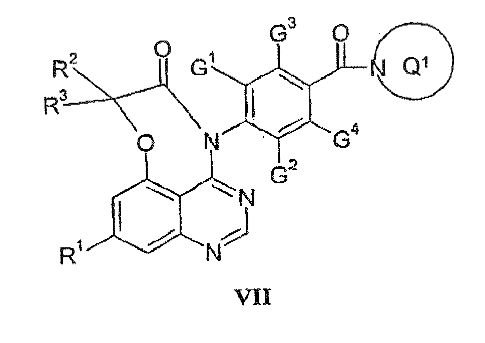 worin R¹, R², R³, G¹, G², G³, G⁴ und der Ring -NQ¹ eine der in Anspruch 1 angegebenen Bedeutungen besitzen, wobei jedoch jede funktionelle Gruppe gegebenenfalls geschützt ist, mit einem Amin der Formel V: 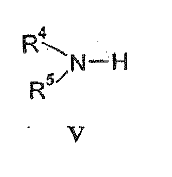 worin R⁴ und R⁵ eine der in Anspruch 1 angegebenen Bedeutungen besitzen, wobei jedoch jede funktionelle Gruppe gegebenenfalls geschützt ist, umsetzt; oder
(e) ein Chinazolin-4(3H)-on der Formel VIII: 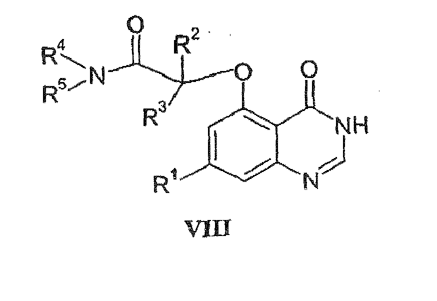 worin R¹, R², R³, R⁴ und R⁵ eine der in Anspruch 1 angegebenen Bedeutungen besitzen, wobei jedoch jede funktionelle Gruppe gegebenenfalls geschützt ist, mit einer geeigneten Aktivierungsgruppe und einem Amin der Formel IX: 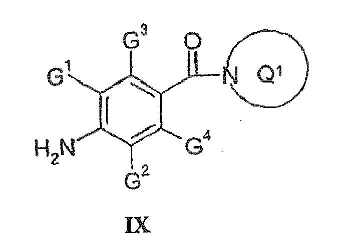 worin G¹, G², G³, G⁴ und der Ring -NQ¹ eine der in Anspruch 1 angegebenen Bedeutungen besitzen, wobei jedoch jede funktionelle Gruppe gegebenenfalls geschützt ist, umsetzt; oder
(f) ein Chinazolin der Formel X: 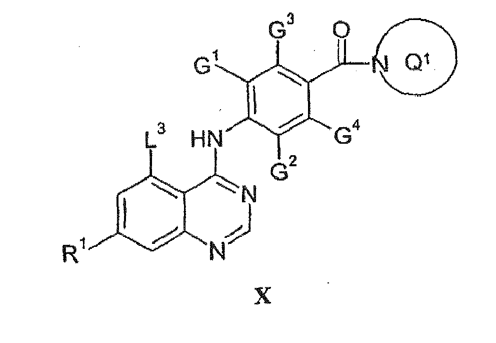 worin R¹, G¹, G², G³, G⁴ und der Ring -NQ¹ eine der in Anspruch 1 angegebenen Bedeutungen besitzen, wobei jedoch jede funktionelle Gruppe gegebenenfalls geschützt ist, und L³ für eine geeignete verdrängbare Gruppe steht, mit einer Verbindung der Formel XI: 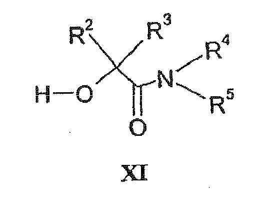 worin R², R³, R⁴ und R⁵ eine der in Anspruch 1 angegebenen Bedeutungen besitzen, wobei jedoch jede funktionelle Gruppe gegebenenfalls geschützt ist, umsetzt; oder
(g) ein Chinazolin der Formel XII: 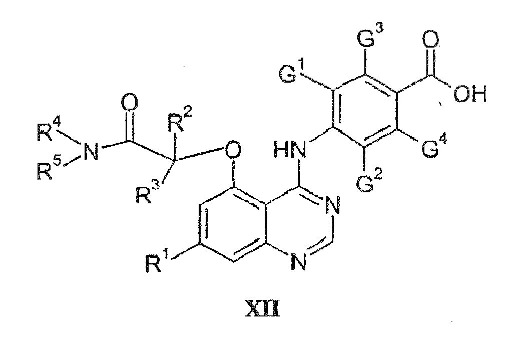 worin R¹, R², R³, R⁴, R⁵, G¹, G², G³ und G⁴ eine der in Anspruch 1 angegebenen Bedeutungen besitzen, wobei jedoch jede funktionelle Gruppe gegebenenfalls geschützt ist, mit einer cyclischen Aminverbindung der Formel XIII: 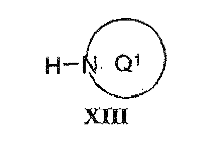 worin der Ring -NQ¹ eine der in Anspruch 1 angegebenen Bedeutungen besitzen, wobei jedoch jede funktionelle Gruppe gegebenenfalls geschützt ist, kuppelt;
und danach gegebenenfalls:
(i) ein Chinazolinderivat der Formel I in ein anderes Chinazolinderivat der Formel I umwandelt;
(ii) jede vorhandene Schutzgruppe abspaltet;
(iii) ein pharmazeutisch annehmbares Salz bildet.

30. Verbindung der Formel II, IV, VI, VII und/oder XII gemäß Anspruch 29 oder ein Salz davon.

## Revendications

1. Dérivé de la quinazoline de formule **I** : 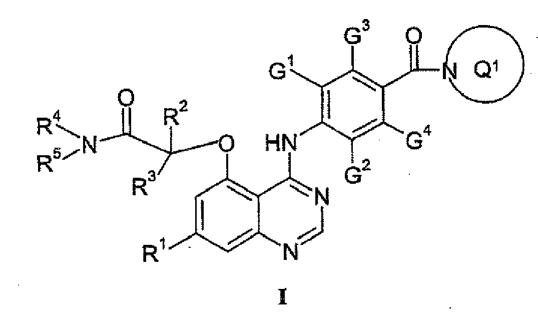 dans laquelle :
**R¹** est choisi parmi hydrogène, hydroxy, (1-4C)alcoxy et (1-4C) alcoxy (1-4C) alcoxy ;
**R² et R³,** qui peuvent être identiques ou différents, sont choisis parmi hydrogène, (1-4C)alkyle, (2-4C)alcényle et (2-4C)alcynyle, lequel (1-4C)alkyle porte éventuellement un ou plusieurs substituants hydroxy ;
**R⁴ et R⁵,** qui peuvent être identiques ou différents, sont choisis parmi hydrogène, (1-4C)alkyle, (3-4C)alcényle et (3-4C)alcynyle, lequel (1-4C)alkyle porte éventuellement un ou plusieurs substituants choisis indépendamment parmi halogéno, cyano, hydroxy, amino, (1-4C)alkylamino, di-[(1-4C)alkyl]amino et (1-4C)alcoxy, ou
R⁴ et R⁵ ensemble avec l'atome d'azote auquel ils sont liés forment un cycle hétérocyclique saturé à 4, 5, 6 ou 7 chaînons contenant un hétéroatome d'azote et contenant éventuellement un ou plusieurs hétéroatomes supplémentaires, choisis indépendamment parmi oxygène, azote et soufre, et où tout cycle hétérocyclique formé par R⁴, R⁵ et l'atome d'azote auquel ils sont liés porte éventuellement un ou plusieurs substituants choisis indépendamment parmi halogéno, cyano, hydroxy, (1-4C) alkyle et (1-4C) alcoxy ;
**G¹ et G²,** qui peuvent être identiques ou différents, sont choisis parmi hydrogène et halogéno ;
**G³ et G⁴,** qui peuvent être identiques ou différents, sont choisis parmi hydrogène, halogéno, cyano, (1-4C)alkyle, (1-4C)alcoxy, (2-4C)alcényle et (2-4C)alcynyle ;
**le cycle -NQ¹** est un cycle hétérocyclique saturé ou partiellement insaturé à 4, 5, 6, 7 ou 8 chaînons lié à l'azote, contenant un hétéroatome d'azote et contenant éventuellement un ou plusieurs hétéroatomes supplémentaires choisis indépendamment parmi oxygène, azote et soufre, et lequel cycle hétérocyclique -NQ¹ porte éventuellement un ou plusieurs substituants choisis indépendamment parmi halogéno, cyano, hydroxy, (1-4C)alkyle, (1-4C)alcoxy et hydroxy-(1-4C) alkyle ;
et où tout cycle hétérocyclique formé par R⁴, R⁵ et l'atome d'azote auquel ils sont liés et/ou tout cycle hétérocyclique -NQ¹ portent éventuellement 1 ou 2 substituants oxo ou thioxo ;
ou un sel pharmaceutiquement acceptable de celui-ci .

2. Dérivé de la quinazoline selon la revendication 1, **caractérisé en ce que** R¹ est choisi parmi hydrogène, hydroxy, méthoxy, éthoxy et méthoxyéthoxy.

3. Dérivé de la quinazoline selon la revendication 1 ou 2, **caractérisé en ce que** R¹ est hydrogène.

4. Dérivé de la quinazoline selon l'une quelconque ou plusieurs des revendications précédentes, **caractérisé en ce que** G¹ et G² sont tous deux hydrogène.

5. Dérivé de la quinazoline selon l'une quelconque ou plusieurs des revendications précédentes, **caractérisé en ce que** l'un de G³ ou G⁴ est halogéno et l'autre de G³ et G⁴ est hydrogène.

6. Dérivé de la quinazoline selon l'une quelconque ou plusieurs des revendications précédentes, **caractérisé en ce que** R² et R³, qui peuvent être identiques ou différents, sont choisis parmi hydrogène et (1-2C)alkyle.

7. Dérivé de la quinazoline selon l'une quelconque ou plusieurs des revendications précédentes, **caractérisé en ce que** R² est hydrogène et R³ est (1-2C)alkyle.

8. Dérivé de la quinazoline selon l'une quelconque ou plusieurs des revendications précédentes, **caractérisé en ce que** R⁴ et R⁵, qui peuvent être identiques ou différents, sont choisis parmi hydrogène, (1-4C)alkyle, (3-4C)alcényle et (3-4C)alcynyle, lequel (1-4C)alkyle porte éventuellement un ou plusieurs substituants choisis indépendamment parmi halogéno, cyano, hydroxy, amino, (1-4C)alkylamino, di-[(1-4C)alkyl]amino et (1-4C)alcoxy.

9. Dérivé de la quinazoline selon l'une quelconque ou plusieurs des revendications 1 à 7, **caractérisé en ce que** R⁴ et R⁵, qui peuvent être identiques ou différents, sont choisis parmi hydrogène et (1-4C)alkyle, lequel (1-4C)alkyle porte éventuellement un ou plusieurs substituants hydroxy, ou
R⁴ et R⁵ ensemble avec l'atome d'azote auquel ils sont liés forment un cycle hétérocyclique saturé à 4, 5, 6 ou 7 chaînons qui contient éventuellement un ou plusieurs hétéroatomes supplémentaires choisis indépendamment parmi oxygène, azote et soufre,
et où tout cycle hétérocyclique formé par R⁴, R⁵ et l'atome d'azote auquel ils sont liés porte éventuellement un ou plusieurs substituants choisis indépendamment parmi halogéno, cyano, hydroxy, (1-4C)alkyle et (1-4C)alcoxy ,
et où tout cycle hétérocyclique formé par R⁴, R⁵ et l'atome d'azote auquel ils sont liés porte éventuellement 1 ou 2 substituants oxo ou thioxo.

10. Dérivé de la quinazoline selon l'une quelconque ou plusieurs des revendications précédentes, **caractérisé en ce que** R⁴ et R⁵ sont tous deux (1-4C)alkyle, lequel (1-4C)alkyle porte éventuellement un ou plusieurs substituants hydroxy.

11. Dérivé de la quinazoline selon l'une quelconque ou plusieurs des revendications précédentes, **caractérisé en ce que** R⁴ est méthyle et R⁵ est (1-4C)alkyle, lequel (1-4C)alkyle porte éventuellement un ou plusieurs substituants hydroxy.

12. Dérivé de la quinazoline selon l'une quelconque ou plusieurs des revendications précédentes, **caractérisé en ce que** R⁴ et R⁵ sont tous deux méthyle.

13. Dérivé de la quinazoline selon l'une quelconque ou plusieurs des revendications 1 à 11, **caractérisé en ce que** R⁴ est méthyle et R⁵ est 2-hydroxyéthyle.

14. Dérivé de la quinazoline selon l'une quelconque ou plusieurs des revendications précédentes, **caractérisé en ce que** le cycle -NQ¹ est un cycle hétérocyclique saturé ou partiellement insaturé à 5, 6 ou 7 chaînons lié à l'azote, contenant un hétéroatome d'azote et contenant éventuellement un ou deux hétéroatomes supplémentaires choisis indépendamment parmi oxygène, azote et soufre, et lequel cycle hétérocyclique -NQ¹ porte éventuellement un ou plusieurs substituants choisis indépendamment parmi halogéno, cyano, hydroxy, (1-4C)alkyle, (1-4C)alcoxy et hydroxy-(1-4C)alkyle, et lequel cycle hétérocyclique -NQ¹ porte éventuellement 1 ou 2 substituants oxo ou thioxo.

15. Dérivé de la quinazoline selon l'une quelconque ou plusieurs des revendications précédentes, **caractérisé en ce que** le cycle -NQ¹ est un cycle hétérocyclique saturé ou partiellement insaturé à 5, 6 ou 7 chaînons lié à l'azote, contenant un hétéroatome d'azote, et lequel cycle hétérocylique -NQ¹ porte éventuellement un ou plusieurs substituants choisis indépendamment parmi halogéno, cyano, hydroxy, (1-4C)alkyle, (1-4C)alcoxy et hydroxy-(1-4C)alkyle, et lequel cycle hétérocyclique -NQ¹ porte éventuellement 1 ou 2 substituants oxo ou thioxo.

16. Dérivé de la quinazoline selon l'une quelconque ou plusieurs des revendications précédentes, **caractérisé en ce que** le cycle -NQ¹ est choisi parmi azépan-1-yle, pipéridin-1-yle et pyrrolidin-1-yle.

17. Dérivé de la quinzoline de formule I, choisi parmi un ou plusieurs de ce qui suit :
le (2*R*)-2-[(4-{[4-(azépan-1-ylcarbonyl)-3-chlorophényl]amino}quinazolin-5-yl)oxy]-*N-*(2-hydroxyéthyl)-*N-*méthylpropanamide ;
le (2*R*)-2-[(4-{[3-chloro-4-{pipéridin-1-ylcarbonyl)phényl]amino}quinazolin-5-yl)oxy]-*N-*(2-hydroxyéthyl)-*N-*méthylpropanamide ;
le (2*R*)-2-[(4-{[3-chloro-4-(pyrrolidin-1-ylcarbonyl)phényl]amino}quinazolin-5-yl)oxy]-*N-*(2-hydroxyéthyl)-*N-*méthylpropanamide ;
le (2*R*)-2-[(4-{[4-(azépan-1-ylcarbonyl)-3-chlorophényl]amino}quinazolin-5-yl)oxy]-*N,N-*diméthylpropanamide ;
le (2R)-2-[(4-{[3-chloro-4-(pipéridin-1-ylcarbonyl)phényl]amino}quinazolin-5-yl)oxy]-*N,N-*diméthylpropanamide ; et
le (2R)-2-[(4-{[3-chloro-4-(pyrrolidin-1-ylcarbonyl)phényl]amino}quinazolin-5-yl)oxy]-*N,N-*diméthylpropanamide ;
ou un sel pharmaceutiquement acceptable de celui-ci .

18. Composition pharmaceutique, **caractérisée en ce qu'**elle comprend un dérivé de la quinazoline de formule **I,** ou un sel pharmaceutiquement acceptable de celui-ci, selon l'une quelconque ou plusieurs des revendications 1 à 17, en association avec un diluant ou un support pharmaceutiquement acceptable.

19. Produit pharmaceutique, **caractérisé en ce qu'**il comprend un dérivé de la quinazoline de formule **I,** ou un sel pharmaceutiquement acceptable de celui-ci, selon l'une quelconque ou plusieurs des revendications 1 à 17, et un agent anti-tumoral supplémentaire pour le traitement conjoint du cancer.

20. Dérivé de la quinazoline de formule **I,** ou un sel pharmaceutiquement acceptable de celui-ci, selon l'une quelconque ou plusieurs des revendications 1 à 17, destiné à être utilisé comme médicament.

21. Utilisation d'un dérivé de la quinazoline de formule **I,** ou d'un sel pharmaceutiquement acceptable de celui-ci, selon l'une quelconque ou plusieurs des revendications 1 à 17, dans la fabrication d'un médicament destiné à être utilisé dans la production d'un effet anti-prolifératif chez un animal à sang chaud.

22. Dérivé de la quinazoline de formule **I,** ou un sel pharmaceutiquement acceptable de celui-ci, selon l'une quelconque ou plusieurs des revendications 1 à 17, pour la production d'un effet anti-prolifératif chez un animal à sang chaud.

23. Utilisation d'un dérivé de la quinazoline de formule **I,** ou d'un sel pharmaceutiquement acceptable de celui-ci, selon l'une quelconque ou plusieurs des revendications 1 à 17, dans la fabrication d'un médicament destiné à être utilisé dans le traitement d'une maladie ou d'une affection médicale médiée totalement ou en partie par le récepteur tyrosine kinase erbB.

24. Dérivé de la quinazoline de formule **I,** ou un sel pharmaceutiquement acceptable de celui-ci, selon l'une quelconque ou plusieurs des revendications 1 à 17, pour le traitement d'une maladie ou d'une affection médicale médiée totalement ou en partie par le récepteur tyrosine kinase erbB chez un animal à sang chaud.

25. Utilisation d'un dérivé de la quinazoline de formule **I,** ou d'un sel pharmaceutiquement acceptable de celui-ci, selon l'une quelconque ou plusieurs des revendications 1 à 17, dans la fabrication d'un médicament destiné à être utilisé dans la prévention ou le traitement des tumeurs qui sont sensibles à l'inhibition d'un ou plusieurs récepteurs tyrosine kinases erbB qui sont impliqués dans les étapes de transduction des signaux qui conduisent à la prolifération et/ou à la survie de cellules tumorales chez un animal à sang chaud.

26. Dérivé de la quinazoline de formule **I,** ou un sel pharmaceutiquement acceptable de celui-ci, selon l'une quelconque ou plusieurs des revendications 1 à 17, pour la prévention ou le traitement des tumeurs qui sont sensibles à l'inhibition d'un ou plusieurs récepteurs tyrosine kinases erbB qui sont impliqués dans les étapes de transduction des signaux qui conduisent à la prolifération et/ou à la survie de cellules tumorales chez un animal à sang chaud.

27. Utilisation d'un dérivé de la quinazoline de formule **I,** ou d'un sel pharmaceutiquement acceptable de celui-ci, selon l'une quelconque ou plusieurs des revendications 1 à 17, dans la fabrication d'un médicament destiné à être utilisé dans le traitement du cancer.

28. Dérivé de la quinazoline de formule **I,** ou un sel pharmaceutiquement acceptable de celui-ci, selon l'une quelconque ou plusieurs des revendications 1 à 17, pour le traitement du cancer chez un animal à sang chaud.

29. Procédé de préparation d'un dérivé de la quinazoline de formule **I,** ou d'un sel pharmaceutiquement acceptable de celui-ci, selon la revendication 1, **caractérisé en ce qu'**il comprend :
(a) la réaction d'une quinazoline de formule **II :** 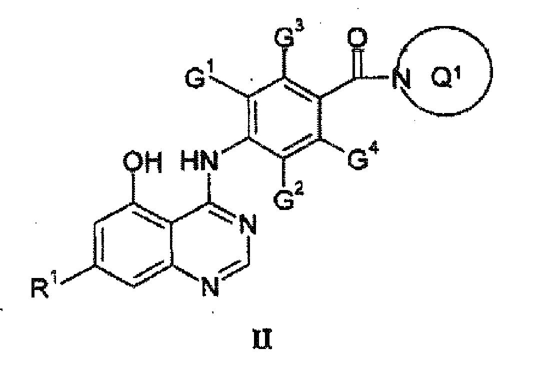 dans laquelle R¹, G¹, G², G³, G⁴ et le cycle -NQ¹ ont l'une quelconque des significations définies dans la revendication 1 sauf que toute fonction est protégée le cas échéant, avec un amide de formule **III :** 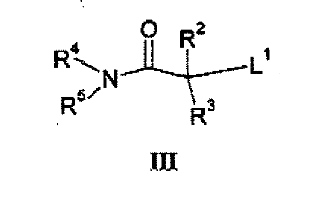 dans laquelle R², R³, R⁴ et R⁵ ont l'une quelconque des significations définies dans la revendication 1 sauf que toute fonction est protégée le cas échéant et L¹ est un groupement déplaçable convenable ; ou
(b) le couplage, commodément en présence d'une base convenable, d'une quinazoline de formule **IV** (ou d'un sel convenable de celle-ci) : 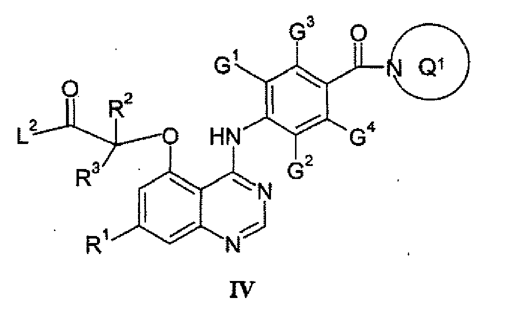 dans laquelle R¹, R², R³, G¹, G², G³, G⁴ et le cycle -NQ¹ ont l'une quelconque des significations définies dans la revendication 1 sauf que toute fonction est protégée le cas échéant, et L² est un groupement déplaçable convenable ou L² est hydroxy, lequel groupement hydroxy est combiné commodément avec un agent de couplage convenable pour produire un groupement déplaçable, avec une amine de formule **V :** 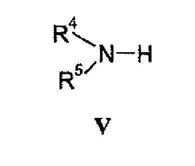 dans laquelle R⁴ et R⁵ ont l'une quelconque des significations définies dans la revendication 1 sauf que toute fonction est protégée le cas échant ; ou
(c) pour des dérivés de la quinazoline de formule **I** dans laquelle R² est 2-hydroxyéthyle, la réaction d'une quinazoline de formule **VI :** 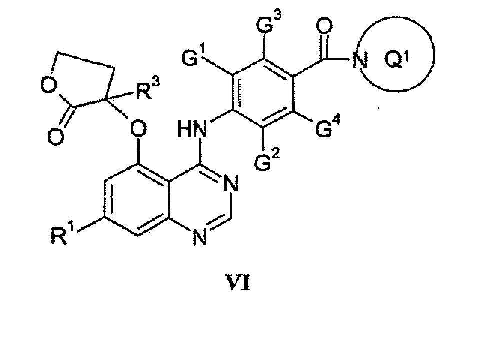 dans laquelle R¹, R³, G¹, G², G³, G⁴ et le cycle -NQ¹ ont l'une quelconque des significations définies dans la revendication 1 sauf que toute fonction est protégée le cas échéant, avec une amine de formule **V :** 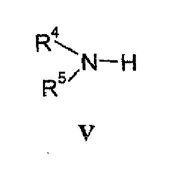 dans laquelle R⁴ et R⁵ ont l'une quelconque des significations définies dans la revendication 1 sauf que toute fonction est protégée le cas échant ; ou
(d) la réaction d'une quinazoline de formule **VII :** 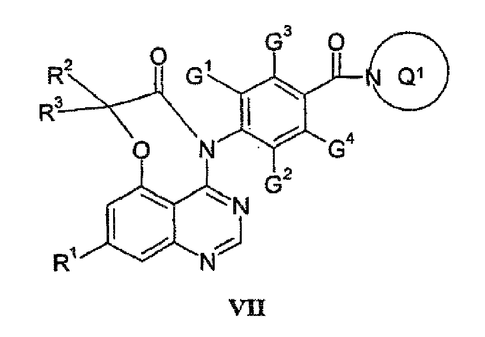 dans laquelle R¹, R², R³, G¹, G², G³, G⁴ et le cycle -NQ¹ ont l'une quelconque des significations définies dans la revendication 1 sauf que toute fonction est protégée le cas échéant, avec une amine de formule **V :** 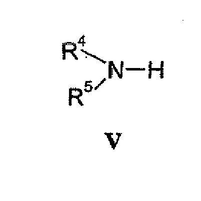 dans laquelle R⁴ et R⁵ ont l'une quelconque des significations définies dans la revendication 1 sauf que toute fonction est protégée le cas échant ; ou
(e) la réaction d'une quinazolin-4(3H)-one de formule **VIII :** 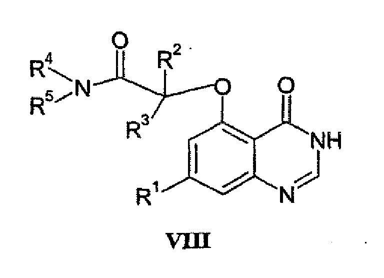 dans laquelle R¹, R², R³, R⁴ et R⁵ ont l'une quelconque des significations définies dans la revendication 1 sauf que toute fonction est protégée le cas échéant, avec un groupement activateur convenable et une amine de formule **IX :** 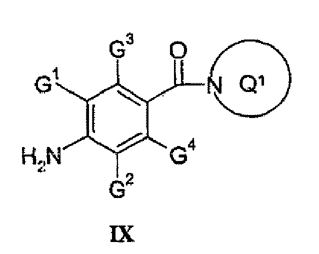 dans laquelle G¹, G², G³, G⁴ et le cycle -NQ¹ ont l'une quelconque des significations définies dans la revendication 1 sauf que toute fonction est protégée le cas échéant ; ou
(f) la réaction d'une quinazoline de formule **X :** 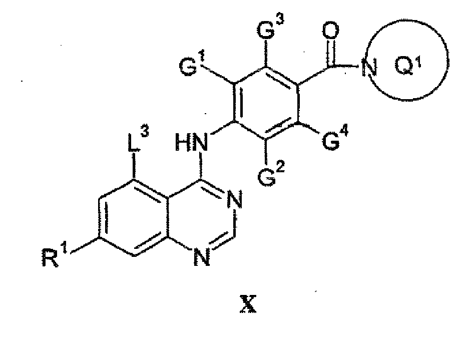 dans laquelle R¹, G¹, G², G³, G⁴ et le cycle -NQ¹ ont l'une quelconque des significations définies dans la revendication 1 sauf que toute fonction est protégée le cas échéant et L³ est un groupement déplaçable convenable, avec un composé de formule **XI :** 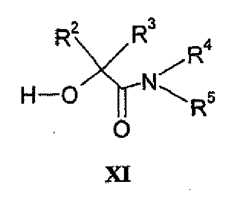 dans laquelle R², R³, R⁴ et R⁵ ont l'une quelconque des significations définies dans la revendication 1 sauf que toute fonction est protégée le cas échéant ; ou
(g) le couplage d'une quinazoline de formule **XII :** 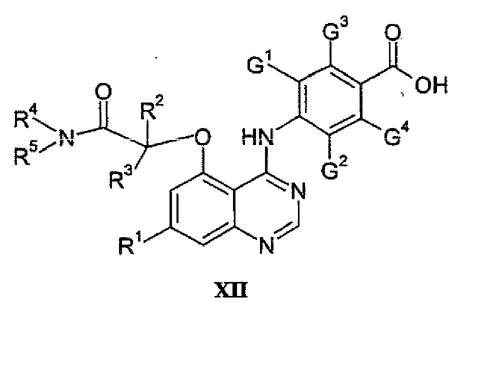 dans laquelle R¹, R², R³, R⁴, R⁵, G¹, G², G³ et G⁴ ont l'une quelconque des significations définies dans la revendication 1 sauf que toute fonction est protégée le cas échéant, avec un composé aminé cyclique de formule **XIII :** 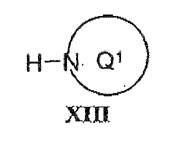 dans laquelle le cycle -NQ¹ a l'une quelconque des significations définies dans la revendication 1 sauf que toute fonction est protégée le cas échéant ;
et ensuite, le cas échéant :
(i) la transformation d'un dérivé de la quinazoline de formule **I** en un autre dérivé de la quinazoline de formule **I ;**
(ii) l'élimination de tout groupement protecteur présent ;
(iii) la formation d'un sel pharmaceutiquement acceptable.

30. Composé de formule **II, IV, VI, VII** et/ou **XII** tel que défini dans la revendication 29, ou un sel de celui-ci.
